# FASCICULE DE BREVET EUROPEEN

(11) **EP 2 315 767 B1**
(45) Date de publication et mention de la délivrance du brevet: **04.09.2013**
(21) Numéro de dépôt: 09784467.4
(22) Date de dépôt: 06.07.2009
(51) Int. Cl.: C07D 471/04, A61K 31/4375, A61P 35/00

(54) **DERIVES DE PYRIDINO-PYRIDINONES, LEUR PREPARATION ET LEUR APPLICATION EN THERAPEUTIQUE**
PYRIDINO-PYRIDINONDERIVATE, DEREN HERSTELLUNG UND DEREN THERAPEUTISCHE VERWENDUNG
PYRIDINO-PYRIDINONE DERIVATIVES, PREPARATION THEREOF, AND THERAPEUTIC USE THEREOF

(30) Priorité: 08.07.2008 FR 0803862
(43) Date de publication de la demande: 04.05.2011
(73) Titulaire: SANOFI, 75008 Paris (FR)
(72) Inventeur: BELLEVERGUE, Patrice, F-75013 Paris (FR); LASSALLE, Gilbert, F-75013 Paris (FR); MCCORT, Gary, F-75013 Paris (FR); MARTIN, Valérie, F-75013 Paris (FR); SAVI, Pierre, F-75013 Paris (FR); VOLLE-CHALLIER, Cécile, F-75013 Paris (FR)
(74) Mandataire: Bouron, Estelle
(86) Numéro de dépôt international: PCT/FR2009/051321
(87) Numéro de publication internationale: WO 2010/004197

(56) Documents cités:
- SUPURAN A ET AL: "Protein kinase inhibitors as anticancer agents" EXPERT OPINION ON THERAPEUTIC PATENTS, INFORMA HEALTHCARE, GB, vol. 14, no. 1, 1 janvier 2004 (2004-01-01), pages 35-53, XP002336074 ISSN: 1354-3776

## Description

La présente invention se rapporte à des dérivés de pyridino-pyridinones substitués en position 7 par un aryle ou hétéroaryle lui-même substitué de manière optimum par un motif de type -[C(R3)(R4)]ₘ-U-N(R5)(R6), à leur préparation et à leur application en thérapeutique en tant qu'inhibiteurs de l'activité kinase des récepteurs aux ligands PDGFs (Platelet derived growth factors) et éventuellement des récepteurs au ligand FLT3 (fms-like tyrosine kinase receptor).

Les récepteurs FLT3 et PDGF-R sont des membres de classe III de la famille des récepteurs à tyrosine kinases (RTK), dont font également partie le récepteur du Stem cell factor (c-kit) et du M-CSF (c-fms). Ils sont caractérisés pas un domaine extracellulaire composé de 5 domaines immunoglobuline-like contenant la région de liaison au ligand, un domaine transmembranaire, et une partie intracellulaire composée d'un domaine juxtamembranaire, d'un domaine kinase séparé en deux par un domaine insert (split domain) (Ullrich & Schlessinger, 1990).
La fixation des ligands sur les RTK induit la dimérisation des récepteurs, l'activation de leur partie tyrosine kinase qui conduit à la transphosphorylation des résidus tyrosine (Weiss & Schlessinger, 1998). Ces résidus phosphorylés servent ainsi de point d'ancrage aux protéines intracellulaires de la signalisation qui *in fine* provoquent différentes réponses cellulaires : la maintenance, la division, la prolifération, la différentiation, ou bien encore la migration cellulaire. (Claesson-Welsh, 1994).

Le gène codant pour FLT3 est situé sur le chromosome 13q12 (Rosnet et al, 1992) et code pour la protéine FLT3 (antigène CD135) exprimée spécifiquement par les cellules hématopoïétiques et plus particulièrement les cellules immatures comme les cellules souches hématopoïétiques et les progéniteurs multipotents myéloïdes et lymphoïdes et son expression disparaît au cours de la différentiation hématopoïétique. Son ligand, le FLT3 Ligand induit la dimérisation du récepteur, suivi de l'autophophosphorylation de la partie intracellulaire du récepteur qui conduit à l'activation de la cascade de signalisation. Les effets de l'activation du récepteur par son ligand sont la survie et l'expansion des progéniteurs multipotents.
Deux isoformes de récepteurs aux PDGFs ont été mis en évidence, la chaine PDGF-Ralpha et la chaine PDGF-Rbeta, qui suite à la fixation de leurs ligands s'homo- ou s'hétérodimérisent et induisent la signalisation intracellulaire. Les récepteurs aux PDGF sont essentiellement exprimés par les cellules d'origine mésenchymateuse et sont notamment retrouvés sur les fibroblastes, les cellules musculaires lisses, les péricytes et les cellules gliales (Ross et coll.1986, Heldin, 1992).
Le « Platelet Derived Growth Factor », PDGF, protéine de poids moléculaire d'environ 30.000 daltons, est sécrété essentiellement par les plaquettes, accessoirement par l'endothélium, les muscles lisses vasculaires et les monocytes. Il est formé de deux chaînes polypeptidiques reliées entre elles par des ponts disulfures formant soit des homodimères, soit des hétérodimères. Quatre gènes (7p22, 22q13, 4q31 et 11q22) ont été décrits comme codant pour 4 différentes chaines polypeptiques (A, B C et D), qui une fois dimérisées donnent cinq ligands biologiquement actifs PDGF-AA, BB, CC, DD et AB (pour revue, Yu et al, 2003). Une spécificité de liaison existe, dont notamment le PDGF-AA pour l'isoforme alpha du récepteur, le PDGF-D pour la forme BB, et le PDGF-C pour la forme alpha et alpha/beta. Les PDGFs ligands sont de puissants mitogènes, mais sont également impliqués dans les phénomènes de migration, survie, apoptose et transformation cellulaire.

Les inhibiteurs de la fonction de PDGF-R alpha, beta et FLT3 interviennent dans différents domaines thérapeutiques. Parmi les phénomènes physiopathologiques où ces récepteurs peuvent être impliqués on retrouve, les cancers liquides ou leucémies, les cancers solides avec ou sans métastases ciblant les cellules tumorales et/ou les cellules de l'environnement tumoral (vasculaires, fibroblastiques), les fibroses et les maladies vasculaires :

### A. Cancer liquides

Les leucémies sont de différents types et touchent soit le compartiment myéloide soit le compartiment lymphoïde.
L'expression de FLT3 dans les cellules leucémiques issues de leucémies aigues myéloïdes (LAM) est de l'ordre de 100% des cas, et FLT3 contribue ainsi à stimuler la survie et la prolifération des cellules leucémiques (Carow et al, 1996 ; Drexler et al, 1996, Stacchini et al, 1996).
De plus, FLT3 est le siège de mutations activatrices dans 22 à 30% des LAM adultes et 11 % de l'enfant. Il s'agit le plus fréquemment de duplications en tandem (ITD) dans la région transmembranaire du récepteur (plus particulièrement les exons 14 et 15). Ces mutations conservent le cadre de lecture et leur taille peuvent varier entre 18 et 111 paires de bases. Plus rarement dans environ 7% des LAM, une mutation ponctuelle sur le résidu D835 situé dans le domaine kinase est retrouvée. Dans la majorité des cas, les formes FLT3 ITD ont un plus grand risque de rechute et sont des marqueurs de pronostic de survie faible. Ces deux types de mutations conduisent à une activité constitutive du domaine kinase indépendante de la stimulation par le ligand et ont été montrées comme transformant les cellules hématopoïétiques *in vitro* et *in vivo* (Mizuki et al, 2000; Tse et al ; 2000). De façon élégante, Kelly et coll. (2002), ont montré dans un modèle de reconstitution de moelle osseuse chez la souris que FLT3 ITD provoquait un syndrome myéloprolifératif.

L'intérêt d'utiliser des inhibiteurs de l'activité tyrosine kinase a été reporté à la fois *in vitro* et *in vivo* par plusieurs équipes, et récemment dans le modèle de reconstitution de moelle osseuse FLT3 ITD, un tel inhibiteur a été montré comme capable d'induire la régression de la tumeur et d'augmenter la survie des animaux (Ofarrel, 2003).
De plus, des données récentes mettent en évidence l'intérêt de combiner de tels inhibiteurs avec des cytotoxiques tel que la daunorubicine (Levis et al, 2004).

De façon intéressante, les cellules blastiques de type LAM peuvent également surexprimer d'autres récepteurs à activité kinase comme c-kit ou bien encore PDGF-R.

### Les syndromes myéloprolifératifs/dysplasiques

De façon assez fréquente, des anormalités cytogénétiques suite à des translocations chromosomiques ont été reportées dans les syndromes myéloprolifératifs. Ces réarrangements génèrent des protéines de fusion à activité tyrosine kinase dérégulées impliquées dans la prolifération des cellules myéloides blastiques.

### -protéines de fusion à activité kinase PDGF-R beta

Les protéines de fusion à activité kinase PDGF-R beta sont constituées de la partie intracellulaire de PDGF-R-beta et d'autre part d'un domaine N-ter d'une autre protéine (en général un facteur de transcription). Ont été reportés notamment dans les leucémies myélomonocytaire chronique (LMMC) : Rab5/PDGF-Rbeta, H4-PDGF-Rbeta, HIP1-PDGF-RB ou bien encore Tel/PDGF-R beta. Cette dernière est la plus représentée. Elle est issue de la translocation t(5;12)(q31;p12) et code pour une protéine de fusion constituée de la partie N-terminale du facteur de transcription Tel et de la partie C-terminal de PDGF-Rbeta. Un domaine d'oligomérisation présent dans la partie Tel, conduit à une forme dimérisée de la protéine de fusion et à l'activité constitutive du domaine kinase. Cette protéine a été montrée *in vitro* comme capable de transformer des cellules hématopoïétiques à plusieurs reprises et notamment de façon détaillée dans l'article de M. Carrol et coll. (PNAS, 1996, 93, 14845-14850). *In vivo,* cette protéine de fusion conduit à un syndrome d'hyperprolifération des cellules myéloïdes (Ritchie et coll., 1999).
De plus, chez l'animal comme en clinique chez l'homme, il a été montré que des inhibiteurs de l'activité tyrosine kinase inhibent la prolifération des cellules blastiques et permettent d'enrayer le processus de leucémogénèse.

### -protéines de fusion à activité kinase PDGF-R alpha

Deux protéines de fusion impliquant PDGF-R alpha ont été reportées: bcr-PDGF-Ralpha présente dans une leucémie myéloide chronique (CML) atypique et FIP1L1-PDGF-Ralpha retrouvée dans une sous-population de leucémies, les LEC « leucémies à éosinophiles », provenant d'un syndrome d'hyper-éosinophilie (Griffin et coll. 2003). Cette protéine de fusion porte une activité constitutive du domaine kinase de PDGF-R alpha et est responsable de la prolifération anarchique de ces cellules.

Des inhibiteurs de l'activité kinase de PDGF-R alpha ont montré l'efficacité sur la prolifération de cellules FIP1L1-PDGF-R alpha positives et récemment un composé inhibiteur a reçu l'indication pour les HES/CEL.

Ainsi, inhiber l'activité kinase de PDGF-Ralpha et beta et l'activité FLT3wt et FLT3ITD comme le font les composés de l'invention, s'avèrent d'intérêt thérapeutique pour les LAM.

Outre les LAM et les syndromes myéloprolifératifs, d'autres leucémies peuvent être intéressantes à cibler avec de tels inhibiteurs dont les B-LAL et les T-LAL (leucémies aigues lymphoïdes-B ou lymphoïdes-T), où FLT3 est également exprimé. De plus, de par l'expression normale de FLT3 sur les cellules souches hématopoïétiques et la mise en évidence de son expression sur les cellules souches leucémiques, des inhibiteurs de l'activité kinase de FLT3 pourraient s'avérer d'intérêt dans toutes les leucémies (dont les CML) où le rôle des cellules souches leucémiques dans la récidive où la résistance est impliquée.

### B. Cancers solides

Des inhibiteurs de l'activité tyrosine kinase des récepteurs PDGF-R alpha et beta peuvent être d'intérêt pour les cancers solides soit en ciblant directement la cellule tumorale qui par autocrinie ou paracrinie est sensible à l'activité d'inhibiteur TK de PDGF-R, soit en ciblant les cellules de l'environnement en déstabilisant le réseau pour favoriser l'association avec d'autres agents thérapeutiques.

### -Exemples de cancers solides dont la cible est la cellule tumorale

### * Cancer des tissus mous : le sarcome d'Ewing

Le sarcome d'Ewing est une forme de cancer des os qui touche principalement les enfants et les jeunes adultes (la moyenne d'âge est de 13 ans). Il concerne 10% des tumeurs osseuses primaires et le risque de métastases est important. C'est une tumeur rare touchant 2 à 3 personnes par million d'habitants par an. Les cellules tumorales sont caractérisées par une translocation chromosomique t(11;22) codant pour la protéine de fusion EWS/FLI1.
Les cellules responsables sont celles du mésenchyme, qui expriment le récepteur PDGF-R-beta qui induit la motilité et la croissance des cellules de sarcome d'Ewing sous stimulation par le PDGF-BB (Üren et al, 2003). De plus, Zwerner and May (2001) ont montré l'expression de PDGF-C par les cellules de sarcome d'Ewing.
Ces mêmes cellules expriment également le récepteur RTK c-kit et il a été montré qu'un inhibiteur de l'activité kinase de PDGF-R et c-kit est capable d'inhiber la croissance tumorale de lignées de sarcome d'Ewing dans un modèle de xénogreffe chez la souris (Merchant et coll., 2002).

### * Tumeur du tissu conjonctif (GIST, dermatofibrosarcome)

### - les GISTs (tumeurs stromales gastro-intestinales)

Le groupe de Fletcher (2004) s'est intéressé aux 15 % des GIST chez qui KIT n'est ni muté ni surexprimé (KIT-wt). Ces auteurs ont observé une forte surexpression du récepteur PDGF-R alpha. Cette situation est retrouvée dans environ un tiers de ces GIST KIT-wt. Quant aux mutations de PDGFRA, les auteurs en observent (35 %) dans les cas où KIT est normal. Les PDGFRA mutées ont une activité tyrosine kinase élevée et constitutives et touchent l'acide aspartique en position 842. De la même façon que pour les Sarcomes d'Ewing, deux inhibiteurs de l'activité kinase de c-kit et PDGF-R ont montré leur efficacité *in vitro et in vivo* sur la proliferation des cellules PDGF-Ralpha muté (Le Tourneau et coll. 2007; Corless et coll., 2005).

### - les dermatofibrosarcomes (de Darier et Ferrand ou protuberans ou DFSP)

Le dermatofibrosarcome de Darier et Ferrand (ou DFSP) est une tumeur dermique à cellules fusiformes de malignité intermédiaire caractérisée par une évolution lente avec un risque majeur de récidive en cas d'exérèse insuffisante. Une anomalie génétique présente dans 95% des cas a été découverte en 1990, avec la mise en évidence notamment de la translocation des chromosomes 17 et 22 t(17-22)(q22; q13) qui aboutit à la fusion des gènes COL1A1 et PDGF B et la grande quantité de PDGFB surexprime son récepteur tyrosine kinase, le PDGFR. Inhiber l'activité kinase de PDGF-R est une thérapie prometteuse puisque *in vitro,* cela conduit à l'inhibition de la prolifération et l'apoptose des cellules tumorales et *in vivo* cela permet la réduction de la croissance tumorale dans des modèles de greffe de tumeurs chez la souris immunodéficientes (Sjöblom T et coll., 2001). De plus, des études cliniques ont montré l'efficacité (rémission complète ou totale) d'une telle molécule dans les DFSP (pour revue voir Mc Arthur, 2007).

### * Gliomes et Glioblastomes :

Le glioblastome est la tumeur de cerveau la plus répandue et la plus agressive avec une survie médiane autour de 1 an. PDGFs et ses récepteurs (alpha et beta) sont fréquemment exprimés dans les gliomes. La possibilité existe qu'une boucle autocrine/paracrine puisse contribuer à la pathogénicité de ces tumeurs. Le recepteur PDGF-R-alpha est exprimé préférentiellement dans les cellules de la tumeur, alors que le récepteur PDGF-beta est exprimé préférentiellement dans les cellules endothéliales vasculaires de la tumeur. Bloquer l'activité kinase du PDGF-R a montré son efficacité 1) *in vitro* par la diminution du nombre de colonies en soft agar et l'inhibition de la prolifération de lignées cellulaires 2) sur la réduction de la croissance tumorale dans des modèles de greffe chez la souris nude 3) en association avec l'irradiation dans des modèles de greffe intracraniale de cellules de lignées de glioblastomes (Oerbel et al, 2006; Geng et coll., 2005, Strawn et coll., 1994 Chin et al, 1997).

Ainsi, les composés de l'invention sont d'intérêt pour les sarcomes d'Ewing, les GIST, les dermatofibrosarcomes mais aussi les tumeurs desmoides, les hémangiomes et autres fibrosarcomes pour lesquelles des données d'expression du PDGF-R existent.

### C. Cibler PDGF-R dans l'environnement tumoral

### Angiogénèse

Les cellules de l'environnement de la tumeur font partie intégrante du développement du cancer que ce soit dans le cas d'une tumeur primaire ou secondaire (métastases). Parmi les cellules de l'environnement qui expriment PDGF-R et pour lesquelles le rôle de ce récepteur a été mis en évidence, on retrouve les cellules murales des vaisseaux c'est à dire les péricytes et les cellules musculaires lisses mais aussi les fibroblastes activés.
L'angiogénèse est un processus de génération de nouveaux vaisseaux capillaires à partir de vaisseaux préexistants ou par mobilisation et différentiation de cellules de la moelle osseuse. Ainsi, à la fois une prolifération incontrôlée des cellules endothéliales et une mobilisation d'angioblastes à partir de la moelle osseuse sont observées dans les processus de néovascularisation des tumeurs. Il a été montré *in vitro* et *in vivo* que plusieurs facteurs de croissance stimulent la prolifération endothéliale comme le VEGF et les FGF. Outre ces mécanismes, il a été également mis en évidence que les cellules murales comme les péricytes et les cellules musculaires lisses participent à la stabilisation des vaisseaux néoformés. L'invalidation de PDGF-R beta cause un déficit des péricytes chez la souris et conduit à la mort des animaux en fin de gestation due à des micro-hémorragies et des oedèmes (Hellström et al, 1999, Hellström et al, 2001). Dans une élégante étude de transplantation, l'expression de PDGF-R-beta par les péricytes a été montrée nécessaire pour leur recrutement au niveau des vaisseaux tumoraux via la rétention de PDGF-B par les cellules endothéliales mais aussi par le PDGF-B sécrété par les cellules tumorales (Abramsson et al, 2003). Dans le modèle transgénique Rip1Tag2 de tumeur pancréatique, Song et coll. ont également montré l'expression de PDGF-R beta sur les progéniteurs périvasculaires dans la moelle issus de la moelle osseuse, progéniteurs qui se différencient en péricytes matures autour de la tumeur.
L'intérêt de bloquer l'activité de PDGF-R sur les péricytes tumoraux a été démontré par l'utilisation d'inhibiteur de l'activité tyrosine kinase de PDGF-R dans des modèles animaux (modèle transgénique de tumeur du pancréas et implantation de tumeur de glioma),et l'effet sur la croissance tumorale s'avère profond en association avec un inhibiteur de l'activité kinase de VEGF-R (Bergers et coll., 2003). Des données de la littérature (Cao et al, 2002, Fons et coll., 2004) ont mis en évidence l'intervention de PDGF-R alpha et du PDGF-C en angiogénèse et dans la différenciation des progéniteurs endothéliaux vers les cellules de type péricytes et cellules musculaires lisses.

### Fibroblastes activés

PDGF-R est abondant dans le stroma tumoral et est retrouvé sur les fibroblastes activés (myofibroblastes). Il a été montré dans deux études que l'association d'inhibiteurs ou antagonistes de PDGF-R avec des agents cytotoxiques conduit à une diminution de la microdensité des vaisseaux des cancers de l'ovaire (Apte et coll.2004) et des cancers du pancréas (Hwang et coll., 2003). PDGF-R beta régule la pression du tissu interstitiel de la tumeur (Heuchel et coll., 1999) et la co-administration des inhibiteurs de PDGF-R et des agents de chimiothérapie améliore leur délivrance dans les cellules tumorales en diminuant la pression intra-tumorale (Griffon-Etienne, 1999). Enfin dans un modèle murin, l'administration d'un inhibiteur de l'activité kinase de PDGF-R améliore la consommation d'agents de chimiothérapie par la tumeur et ainsi augmente leur efficacité (Griffon-Etienne, 1999; Pietras et coll., 2002 ; Pietras et coll., 2003). Ces effets sont certainement l'effet des TAF (tumour associated fibroblast), encore appelés CAF (carcinoma associated fibroblastes), fibroblastes activés présents autour de la tumeur qui expriment PDGF-R, comme l'illustrent les travaux récents de Hwang et coll. (2008), Kain et al (2008), Pietras et al (2008) dans des modèles vivo de cancer pancréatique et de cancérogénèse cervical. La stimulation par le PDGF ligand produit par les cellules tumorales stimule les fibroblastes qui produisent la matrice extracellulaire et ainsi augmentent la tension interstitielle. Aussi, diminuer cette tension peut faciliter la délivrance des drogues au sein de la tumeur et ainsi augmenter leur efficacité. Les fibroblastes activés présents dans le stroma tumoral représentent donc une nouvelle cible thérapeutique en oncologie (pour revue voir Bouzin & Feron, 2007).

### Métastases

Plusieurs travaux indiquent que le couple PDGF-R et PDGF-ligand est impliqué dans le développement des métastases, certainement par leur action sur l'angiogénèse et la metastatisation par la circulation sanguine, mais aussi par un effet direct sur la lymphangiogénèse et donc les métastases disséminées par les vaisseaux lymphatiques. Une revue documente notamment le rôle direct du PDGF-BB dans la lymphangiogénèse et les métastases lymphatiques (Cao et coll., 2005). Mais la majorité des travaux impliquent l'expression de PDGF-R dans l'environnement des métastases qui favorisent la prise et le développement des tumeurs secondaires. L'exemple le plus fréquemment rapporté est le développement des métastases osseuses.

### * Exemple du cancer de la prostate :

L'os est fréquemment le siège de métastases. 85 à 100% des patients qui meurent de cancer de prostate ont des métastases osseuses. La chimiothérapie améliore la survie sans progression et la survie globale cependant en raison de l'extrême hétérogénéité des métastases osseuses chez un même patient, la chimiothérapie n'est pas curatrice. Il a été montré en utilisant un modèle de souris immunodéficientes que PDGF-BB joue un rôle important dans le développement des métastases osseuses ostéoblastiques *in vivo* (Yu et coll., 2003). Le PDGF-DD, quant à lui, accélère la croissance des cellules tumorales prostatiques et augmente leur interaction avec les cellules du stroma. L'expression du récepteur PDGF alpha et beta a été mis en évidence respectivement dans 62 et 75% des cancers de prostate. De plus, une étude immunohistochimique a montré que la tumeur prostatique et ses métastases exprimaient PDGF-R (Hwang et coll., 2003). Kim et coll. (2003) ont montré que PDGF-R est exprimé sur les métastases osseuses et les cellules endothéliales vasculaires dépendantes des métastases. Un inhibiteur de tyrosine kinase de PDGF R associé à un agent cytotoxique, réduit substantiellement les métastases osseuses du cancer de la prostate dans un modèle murin (Uehara et coll., 2003). De plus, cette même association conduit à l'apoptose des cellules tumorales, des cellules vasculaires endothéliales et l'inhibition de la croissance des cellules tumorales dans l'os. Le blocage de ces récepteurs et de leurs voies de signalisation dans l'os constitue une approche thérapeutique nouvelle (Hwang et coll. 2003 ; Uehara et coll., 2003). Chez l'homme, des essais cliniques ont montré le gain que présente l'association d'inhibiteur de PDGF-R et de cytotoxique chez des patients atteints de cancers de prostate hormonorésistants avec des métastases osseuses. Une diminution du marqueur (antigène prostatique spécifique) PSA > 50% a été en effet observée chez 38% des patients. La durée moyenne de la réponse du PSA était de 8 mois et la durée de la survie sans progression était de 11 mois.
Au vu des ces différents travaux, il apparaît que les composés de l'invention sont d'intérêt pour le traitement des cancers solides par leur effet sur les cellules de l'environnement et ce en association avec d'autres agents thérapeutiques comme des cytotoxiques ou des inhibiteurs de l'angiogénèse.

### D. Fibroses

Les fibroses sont souvent la cause d'un événement primaire comme un cancer, le traitement par la radiothérapie, les hépatites, l'alcoolémie. L'implication de PDGF est clairement démontrée dans la fibrose pulmonaire (incluant asbestose), rénale (glomérulonéphrite), médullaire (souvent associé aux leucémies à mégacaryocytes), induite par la radiothérapie ainsi que la fibrose hépatique et pancréatique (liée à l'alcoolémie ou à des hépatites) (pour revue voir JC Bonner, 2004). Une surexpression de PDGF a été notamment clairement montrée et des résultats dans des modèles *vivo* avec des inhibiteurs de l'activité TK de PDGF-R ont été également rapportés. Parmi ces études, celle de Einter et coll. (2002) a montré que le PDGF-CC est un puissant inducteur de fibrose rénale. Les auteurs ont testé l'efficacité d'un anticorps neutralisant dans un modèle de ligature urétrale unilatérale, où la fibrose se développe particulièrement rapidement. Ils ont observé un effet antifibrosant très marqué avec une réduction de l'accumulation de myofibroblastes, une réduction de l'accumulation de matrice extracellulaire et une réduction des dépôts de collagène IV. Une autre étude conduite dans un modèle de fibrose pulmonaire induite par la Bléomycine chez la souris a montré l'efficacité d'un inhibiteur de l'activité TK de PDGF-R sur la prévention de la fibrose par inhibition de la prolifération des cellules mésenchymales (Aono et coll., 2005). Dans un modèle de fibrose induite par l'amiante, un inhibiteur de PDGF-R TK a réduit la progression de la fibrose dans le parenchyme pulmonaire et le dépot de collagène (Vuorinen K, Gao F, Oury TD, Kinnula VL, Myllärniemi M. Imatinib mesylate inhibits fibrogenesis in asbestos-induced interstitial pneumonia. Exp Lung Res. 2007 Sep;33(7):357-73). Plusieurs équipes ont montré l'implication de PDGF-R dans la fibrose hépatique. Il est clairement montré que PDGFBB et DD possèdent des caractéristiques pro-fibrogéniques sur les cellules stellates hépatiques (Rovida et coll., 2008; Borkham-Kamphorst et coll., 2007). In vivo, un inhibiteur de PDGF-R TK est capable de réduire la fibrogénèse précoce dans un modèle de ligature du canal biliaire chez le rat (Neef et col.,2006).

Aussi, au vue des données de la littérature, les composés de l'invention semblent d'intérêt thérapeutique pour les différents types de fibrose.

### E. Maladies vasculaires : athérosclérose & resténose, artériosclérose

La prolifération et la migration de cellules musculaires lisses vasculaires contribuent à l'hypertrophie intimale des artères et joue ainsi un rôle prépondérant dans l'athérosclérose et dans la resténose après angioplastie et endoarterectomie. Il a été clairement démontré *in vitro* et *in vivo* dans des modèles animaux, que PDGF est impliqué dans ces phénomènes. In vivo, il a été montré notamment une augmentation de l'expression de PDGF dans un modèle « Vein graft » chez le cochon. De plus, il a été également montré qu'un inhibiteur de l'activité TK de PDGF-R diminuait de façon conséquente la taille des lésions de l'artère thoracique et abdominale de souris diabetiques ApoE-KO (animaux traités à la streptozotocin), Une autre étude a montré que l'inhibition de la signalisation induite par PDGF (TK or PDGF A antisens) conduit à une diminution de la formation de la neointima formation dans les modèles « balloon injury » et de « coronary artery restenosis ». (Deguchi J, 1999, Ferns et coll. 1991, Sirois et al, 1997, Lindner et coll. 1995)

Ainsi, des inhibiteurs de l'activité tyrosine kinase de PDGF-R, tels que les composés de la présente invention représentent une thérapie de choix, soit seul, soit en association avec des composés antagonistes d'autres facteurs de croissance impliqués dans ces pathologies comme le FGF, dans le traitement des pathologies liées à la prolifération des cellules musculaires lisses vasculaires telles que l'athérosclérose, la resténose post-angioplastie ou suite à la pose de prothèses endovasculaires (stents) ou lors de pontages aorto-coronariens.

Les composés de l'invention de par leur activité inhibitrice de l'activité TK de PDGF-R semblent d'intérêt pour traiter ces maladies vasculaires.

### F. Autres

D'autres pathologies semblent pouvoir être des indications pour les composés de l'invention dont l'hypertension artérielle pulmonaire (HTAP) idiopathique. L'HTAP caractérisée par une élévation importante et continue de la pression dans l'artère pulmonaire, conduit à l'insuffisance ventriculaire droite et, souvent, à la mort du patient. Elle est associée à l'accroissement de la prolifération et à la migration des cellules musculaires lisses des vaisseaux pulmonaires. Schermuly et coll. (2005) ont montré que l'inhibition de l'activité tyrosine kinase des récepteurs aux PDGF améliore considérablement l'évolution de la maladie. Pour cela, ils ont utilisé entre autre un modèle d'hypertension artérielle pulmonaire expérimentale chez le rat obtenu par administration de monocrotaline pendant 28 jours. Tous les rats traités ont survécus, alors que 50% d'entre eux sont morts dans le groupe témoin non traité.

Des composés hétérocycliques qui sont utiles en tant qu'inhibiteurs de l'activité kinase des récepteurs aux ligands PDGF et FLT3 ont été décrits par Supuran et al. (Expert Opinion on Therapeutic patents 2004, 14(1), 35-53).

La présente invention a pour objet des composés répondant à la formule (1) : 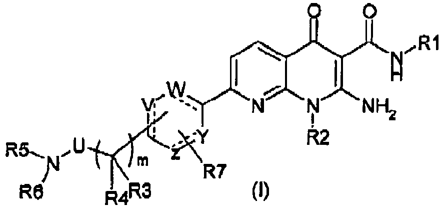 dans laquelle
**R1** représente un groupe (C1-C4) alkyle,
**R2** représente -(CH₂)ₙ-B où n'=0,1,2,3,4 et B est un groupe (C3-C5)cycloalkyle ou un groupe (C1-C4) alkyle éventuellement substitué par un ou plusieurs atomes de fluor, un groupe (C1-C4) alcoxy,
**U** représente un groupe carbonyle ou un groupe -CH₂-,
**Y, Z, V et W** représentent indépendamment les uns des autres un groupe - CH- ou un atome de carbone éventuellement substitué par un groupe R7 ou un hétéroatome tel qu'un atome d'azote ou un atome de soufre ou un atome d'oxygène; ou pas d'atome, étant entendu que la cycle doit être aromatique et comprendre entre 5 et 6 chainons,
**R3, R4** représentent indépendamment l'un de l'autre un atome d'hydrogène ou un groupe (C1-C4) alkyle linéaire, ou R3 et R4 forment ensemble avec le carbone auxquels ils sont liés un groupe (C3-C5) cycloalkyle,
**m est** un nombre entier égal à 1, 2, 3, 4,
**R5** représente un atome d'hydrogène ou un groupe (C1-C4) alkyle,
**R6** représente -(CH₂)ₙ-**L** dans lequel n= 0, 1, 2, 3 et **L** est un groupe sélectionné indépendamment parmi les groupes suivants :
o un groupe (C1-C5) alkyle éventuellement substitué par un groupe (C1-C4) alcoxy,
o un groupe (C3-C5) cycloalkyle,
o un aryle comprenant 6 atomes de carbone et éventuellement substitué par un ou plusieurs atomes d'halogènes,
o un hétéroaryle comprenant entre 5 et 6 chainons dont au moins un hétéroatome choisi parmi un atome d'azote ou de soufre, éventuellement substitué par un groupe (C1-C4) alkyle,
o un hétérocycle saturé où ledit hétérocycle comprend entre 4 et 7 chainons dont au moins un hétéroatome choisi parmi un atome d'azote, un atome d'oxygène et est éventuellement substitué en des positions quelconques, y compris sur l'atome d'azote par un ou plusieurs substituants parmi un atome de fluor, un groupe fluoroalkyle en (C1-C4), un groupe (C1-C4)alkyle linéaire ou ramifié, un groupe (C3-C5) cycloalkyle ou un groupe (C1-C4)alkyl-sulfonamide,
**R7** représente un atome d'hydrogène ou un groupe (C1-C4) alkyle ou un atome d'halogène.

La présente invention a pour objet des composés répondant à la formule (I) : 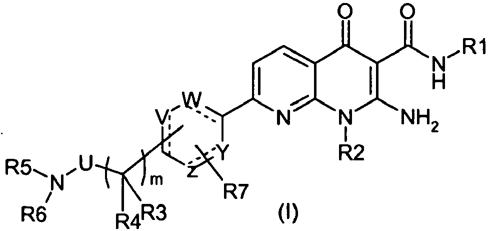 dans laquelle
**R1** représente un groupe (C1-C4) alkyle,
**R2** représente -(CH₂)ₙ-B où n'=0,1,2,3,4 et B est un groupe (C3-C5)cycloalkyle ou un groupe (C1-C4) alkyle éventuellement substitué par un ou plusieurs atomes de fluor, un groupe (C1-C4) alcoxy,
**U** représente un groupe carbonyle ou un groupe -CH₂-,
**Y, Z, V et W** représentent indépendamment les uns des autres un groupe - CH- ou un atome de carbone éventuellement substitué par un groupe R7 ou un hétéroatome tel qu'un atome d'azote ou un atome de soufre, ou un atome d'oxygène ou pas d'atome, étant entendu que le cycle doit être aromatique et comprendre entre 5 et 6 chainons,
**R3, R4** représentent indépendamment l'un de l'autre un atome d'hydrogène ou un groupe (C1-C4) alkyle linéaire, ou R3 et R4 forment ensemble avec le carbone auxquels ils sont liés un groupe (C3-C5) cycloalkyle,
**m** est un nombre entier égal à 1, 2, 3, 4,
**R5** représente un atome d'hydrogène ou un groupe (C1-C4) alkyle,
**R6** représente -(CH₂)ₙ-L dans lequel n= 0, 1, 2, 3 et L est un groupe sélectionné indépendamment parmi les groupes suivants :
o un groupe (C1-C5) alkyle éventuellement substitué par un groupe (C1-C4) alcoxy,
o un groupe (C3-C5) cycloalkyle,
o un aryle comprenant 6 atomes de carbone et éventuellement substitué par un ou plusieurs atomes d'halogènes,
o un hétéroaryle comprenant entre 5 et 6 chainons dont au moins un hétéroatome choisi parmi un atome d'azote ou de soufre, éventuellement substitué par un groupe (C1-C4) alkyle,
o un hétérocycle saturé où ledit hétérocycle comprend entre 5 et 7 chainons dont au moins un hétéroatome choisi parmi un atome d'azote, un atome d'oxygène et est éventuellement substitué en des positions quelconques, y compris sur l'atome d'azote par un ou plusieurs substituants parmi un atome de fluor, un groupe fluoroalkyle en (C1-C4), un groupe (C1-C4)alkyle linéaire ou ramifié, un groupe (C3-C5) cycloalkyle ou un groupe (C1-C4)alkyl-sulfonamide,
**R7** représente un atome d'hydrogène ou un groupe (C1-C4) alkyle ou un atome d'halogène.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.
Par exemple, quand L représente un hétérocycle, la configuration absolue d'un carbone substitué sur ledit hétérocycle peut être R ou S.

Les composés de formule (I) peuvent notamment exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme de solvats, à savoir sous forme d'associations ou de combinaisons avec une ou plusieurs molécules de solvant. De tels solvats font également partie de l'invention.

Dans le cadre de la présente invention, on entend par :
- un groupe alkyle : un groupe aliphatique saturé comprenant de 1 à 7 atomes de carbone (avantageusement, de 1 à 4 atomes de carbone) et étant linéaire ou, quand la chaîne alkyle comprend au moins 3 atomes de carbone, pouvant être ramifié ou cyclique (y compris une cyclisation seulement partielle). A titre d'exemples, on peut citer les groupes méthyle, éthyle, propyle, isopropyle, butyle, iso-butyle, tertio-butyle, méthyl-cyclopropyle, pentyle, 2,2-diméthylpropyle, hexyle, heptyle, etc ..., ainsi que les groupes cycloalkyles définis ci-dessous ;
- un groupe cycloalkyle : un groupe alkyle cyclique comprenant de 3 à 7 atomes de carbone (avantageusement de 3 à 5 atomes de carbone) et dont tous les atomes de carbones sont engagés dans le cycle. On peut citer les groupes cyclopropyle, cyclobutyle, cyclopentyle, cyclohexyle et cycloheptyle ;
- un groupe alcoxy : un groupe -O-alkyle, où le groupe alkyle est tel que défini ci-dessus ;
- un atome d'halogène : un fluor, un chlore, un brome ou un iode ;
- un groupe halogénoalkyle : un groupe comprenant un groupe alkyle tel que défini ci-dessus dont un ou plusieurs atomes d'hydrogène ont été substitués par un atome d'halogène tel que défini ci-dessus ;
- un hétéroatome : un atome d'azote, d'oxygène ou de soufre ;
- un groupe aryle : un groupe aromatique monocyclique comprenant 6 chaînons, par exemple un groupe phényle ;
- un groupe hétéroaryle : un groupe aromatique monocyclique comprenant entre 5 et 7 chaînons dont entre 1 et 3 hétéroatomes tels que précédemment définis. A titre d'exemples, on peut citer les groupes pyridine, pyrazine, pyrimidine, imidazole, pyrrole, thiophène, thiazole ;
- un hétérocycle : un groupe alkyle cyclique comprenant entre 5 et 7 chaînons dont un ou plusieurs hétéroatomes tels que précédemment définis. A titre d'exemples, on peut citer les groupes pyrrolidine, morpholine, pipéridine, pipérazine.

Parmi les composés de formule (I) objets de l'invention, on peut citer un groupe de composés qui se définit comme suit :
**R1** représente un groupe (C1-C4) alkyle,
   et/ou
**R2** représente -(CH₂)ₙ-B où n'=0,1 et B est un groupe (C3-C5) cycloalkyle ou un groupe (C1-C4) alkyle éventuellement substitué par un ou plusieurs atomes de fluor, un groupe (C1-C4) alcoxy,
   et/ou
**U** représente un groupe carbonyle ou un groupe -CH₂-,
   et/ou
**Y, Z, V et W** représentent indépendamment les uns des autres un groupe-CH- ou un atome de carbone éventuellement substitué par un groupe R7 ou un hétéroatome tel qu'un atome d'azote ou un atome de soufre, ou pas d'atome, étant entendu que le cycle doit être aromatique et comprendre entre 5 et 6 chainons,
   et/ou
**R3, R4** représentent indépendamment l'un de l'autre un atome d'hydrogène ou un groupe (C1-C4) alkyle linéaire ou R3 et R4 forment ensemble avec le carbone auxquels ils sont liés un groupe (C3-C5) cycloalkyle,
   et/ou
**m** est un nombre entier égal à 1, 2, 3, 4,
   et/ou
**R5** représente un atome d'hydrogène ou un groupe (C1-C4) alkyle,
   et/ou
**R6** représente -(CH₂)ₙ-L dans lequel n= 0, 1, 2, 3 et **L** est un groupe sélectionné indépendamment parmi les groupes suivants :
   o un groupe (C1-C5) alkyle éventuellement substitué par un groupe (C1-C4) alcoxy,
   o un groupe (C3-C5) cycloalkyle,
   o un aryle comprenant 6 atomes de carbone et éventuellement substitué par un ou plusieurs atomes d'halogènes,
   o un hétéroaryle comprenant entre 5 et 6 chainons dont au moins un hétéroatome choisi parmi un atome d'azote ou de soufre, éventuellement substitué par un groupe (C1-C4) alkyle,
   o un hétérocycle saturé où ledit hétérocycle comprend entre 5 et 7 chainons dont au moins un hétéroatome choisi parmi un atome d'azote, un atome d'oxygène et est éventuellement substitué en des positions quelconques, y compris sur l'atome d'azote par un ou plusieurs substituants parmi un atome de fluor, un groupe fluoroalkyle en (C1-C4), un groupe (C1-C4) alkyle linéaire ou ramifié, un groupe (C3-C5) cycloalkyle ou un groupe (C1-C4) alkyl-sulfonamide,
   et/ou
**R7** représente un atome d'hydrogène ou un groupe (C1-C4) alkyle ou un atome d'halogène.

Parmi les composés de formule (I) objets de l'invention, un premier sous-groupe de composés est constitué par les composés pour lesquels **U** représente un groupe carbonyle.

Parmi les composés de formule (I) objets de l'invention, un deuxième sous-groupe de composés est constitué par les composés pour lesquels **U** représente un groupe un groupe -CH₂-.

Parmi les composés de formule (I) objets de l'invention, un troisième sous-groupe de composés est constitué par les composés pour lesquels le cycle comprenant **Y, Z, V et W est** choisi parmi les groupes phényle, pyridine, thiazole, thiophène.

Parmi les composés de formule (I) objets de l'invention, un quatrième sous-groupe de composés est constitué par les composés pour lesquels **R3** représente un atome d'hydrogène.

Parmi les composés de formule (I) objets de l'invention, un cinquième sous-groupe de composés est constitué par les composés pour lesquels **R4** représente un atome d'hydrogène.

Parmi les composés de formule (I) objets de l'invention, un sixième sous-groupe de composés est constitué par les composés pour lesquels **R5** représente un atome d'hydrogène.

Parmi les composés de formule (I) objets de l'invention, un septième sous-groupe de composés est constitué par les composés pour lesquels **L** représente un groupe (C1-C5) alkyle éventuellement substitué par un groupe (C1-C4) alcoxy.

Parmi les composés de formule (I) objets de l'invention, un huitième sous-groupe de composés est constitué par les composés pour lesquels **L** représente un groupe (C3-C5) cycloalkyle.

Parmi les composés de formule (1) objets de l'invention, un neuvième sous-groupe de composés est constitué par les composés pour lesquels **L** représente un groupe hétéroaryle comprenant entre 5 et 6 chainons dont au moins un hétéroatome choisi parmi un atome d'azote ou de soufre, éventuellement substitué par un groupe (C1-C4) alkyle. Avantageusement, le groupe hétéroaryle est choisi parmi les groupes pyridine, pyrazine, pyrimidine, imidazole, pyrrole, thiazole.

Parmi les composés de formule (I) objets de l'invention, un dixième sous-groupe de composés est constitué par les composés pour lesquels **L** représente un hétérocycle saturé où ledit hétérocycle comprend entre 5 et 7 chainons dont au moins un hétéroatome choisi parmi un atome d'azote, un atome d'oxygène et est éventuellement substitué en des positions quelconques, y compris sur l'atome d'azote par un ou plusieurs substituants parmi un atome de fluor, un groupe fluoroalkyle en (C1-C4), un groupe (C1-C4) alkyle linéaire ou ramifié, un groupe (C3-C5) cycloalkyle ou un groupe (C1-C4) alkyl-sulfonamide. Avantageusement, ledit groupe hétérocyclique est choisi parmi les groupes pyrrolidine, morpholine.

Parmi les composés de formule (I) objets de l'invention, un onzième sous-groupe de composés est constitué par les composés pour lesquels **m** est égal à 1.

Parmi les composés de formule (I) objets de l'invention, un douzième sous-groupe de composés est constitué par les composés pour lesquels la chaîne - [C(R3R4)]ₘ-U-N(R5)(R6) est en position para par rapport au cycle auquelle elle est liée.

Parmi les composés de formule (I) objets de l'invention, un treizième sous-groupe de composés est constitué par les composés pour lesquels la chaîne - [C(R3R4)]ₘ-U-N(R5)(R6) est en position meta par rapport au cycle auquelle elle est liée.

Tous les groupes et sous-groupes peuvent indépendamment les uns des autres être utilisés en combinaison pour obtenir des composés selon l'invention.

Parmi les combinaisons de groupes et sous-groupes correspondant à des composés objets de l'invention, on peut citer une première combinaison correspondant à des composés pour lesquels :
**U** représente un groupe carbonyle,
**R3, R4** représentent un atome d'hydrogène,
**R5** représente un atome d'hydrogène,
**R7** représente un atome d'hydrogène,
**m** est égal à 1,
et **R1, R2, Y, Z, V, W, R6,** n sont tels que définis précédemment.

On peut citer une deuxième combinaison correspondant à des composés selon l'invention pour lesquels :
**R1** représente un groupe (C1-C4) alkyle,
**R2** représente -(CH₂)_{n'}-**B** avec n' = 0 ou 1 et **B** est un groupe (C3-C5) cycloalkyle ou un groupe (C1-C4) alkyle,
**U** représente un groupe carbonyle,
**Y, Z, V, W** représentent, indépendamment les uns des autres, un hétéroatome, un atome de carbone substitué par un groupe R7 ou un groupe -CH-,
**R7** représente un atome d'hydrogène ou d'halogène ou un groupe (C1-C4) alkyle,
**R3** représente un atome d'hydrogène,
**R4** représente un atome d'hydrogène ou un groupe (C1-C4) alkyle,
**R5** représente un atome d'hydrogène,
**R6** représente -(CH₂)ₙ-L où L représente un groupe sélectionné parmi les groupes suivants :
   o aryle comprenant 6 atomes de carbone et éventuellement substitué par un ou plusieurs atomes d'halogènes,
   o hétéroaryle comprenant entre 5 et 6 chainons dont au moins un hétéroatome choisi parmi un atome d'azote ou de soufre,
   o hétérocycle saturé où ledit hétérocycle comprend entre 5 et 6 chainons dont au moins un hétéroatome choisi parmi un atome d'azote, un atome d'oxygène et est éventuellement substitué en des positions quelconques, y compris sur l'atome d'azote par un ou plusieurs substituants parmi un atome de fluor, un groupe fluoroalkyle en (C1-C4), groupe (C1-C4) alkyle ou un groupe (C3-C5) cycloalkyle,
m est égal à 1,
et n est tel que défini précédemment.

On peut citer une troisième combinaison correspondant à des composés selon l'invention pour lesquels :
**R1** représente un groupe (C1-C4) alkyle,
**R2** représente -(CH₂)ₙ-**B** avec n'= 0, 1, 2, 3, 4 et **B** est un groupe un groupe (C1-C4) alkyle,
U représente un groupe carbonyle,
**Y, Z, V, W** représentent, indépendamment les uns des autres, un hétéroatome, un atome de carbone substitué par un groupe R7 ou un groupe -CH-,
**R7** représente un atome d'hydrogène ou d'halogène ou un groupe (C1-C4) alkyle,
**R3** représente un atome d'hydrogène,
**R4** représente un atome d'hydrogène,
**R5** représente un atome d'hydrogène,
**R6** représente -(CH₂)ₙ-**L** où **L** représente un groupe sélectionné parmi les groupes suivants :
   o aryle comprenant 6 atomes de carbone et éventuellement substitué par un ou plusieurs atomes d'halogènes,
   o hétéroaryle comprenant entre 5 et 6 chainons dont au moins un hétéroatome choisi parmi un atome d'azote ou de soufre,
   o hétérocycle saturé où ledit hétérocycle comprend entre 5 et 6 chainons dont au moins un hétéroatome choisi parmi un atome d'azote, un atome d'oxygène et est éventuellement substitué en des positions quelconques, y compris sur l'atome d'azote par un ou plusieurs substituants parmi un atome de fluor, un groupe fluoroalkyle en (C1-C4) ou un groupe (C1-C4) alkyle,
m est égal à 1,
et n est tel que défini précédemment.

On peut citer une quatrième combinaison correspondant à des composés selon l'invention pour lesquels :
**R1** représente un groupe (C1-C4) alkyle,
**R2** représente un groupe -(CH₂)ₙ-**B** où n'= 0, 1, 2, 3, 4 et **B** est un groupe (C1-C4) alkyle,
**U** représente un groupe carbonyle,
**Y, Z, V, W** représentent, indépendamment les uns des autres, un hétéroatome, un groupe -CH-,
**R3** représente un atome d'hydrogène,
**R4** représente un atome d'hydrogène,
**R5** représente un atome d'hydrogène,
**R6** représente -(CH₂)ₙ-**L** où **L** représente un groupe sélectionné parmi les groupes suivants :
   o aryle comprenant 6 atomes de carbone et éventuellement substitué par un ou plusieurs atomes d'halogènes,
   o hétéroaryle comprenant entre 5 et 6 chainons dont au moins un hétéroatome choisi parmi un atome d'azote ou de soufre,
   o hétérocycle saturé où ledit hétérocycle comprend entre 5 et 6 chainons dont au moins un hétéroatome choisi parmi un atome d'azote, un atome d'oxygène et est éventuellement substitué en des positions quelconques, y compris sur l'atome d'azote par un ou plusieurs substituants parmi un atome de fluor, un groupe (C1-C4) alkyle ou un groupe (C3-C5) cycloalkyle,
et m et n sont tels que définis précédemment.

On peut citer une cinquième combinaison correspondant à des composés selon l'invention pour lesquels :
**R1** représente un groupe (C1-C4) alkyle,
**R2** représente un groupe -(CH₂)ₙ-**B** où n'=0, 1, 2, 3, 4 et **B** est un groupe (C1-C4) alkyle,
**U** représente un groupe carbonyle,
**Y, Z, V, W** représentent un groupe -CH-,
**R3, R4** représentent un atome d'hydrogène,
**R5** représente un atome d'hydrogène,
**R7** est un atome d'hydrogène,
**R6** représente -(CH₂)ₙ-**L** où **L** représente un groupe (C1-C5) alkyle linéaire ou ramifié éventuellement substitué par un groupe (C1-C4) alcoxy ou un groupe (C3-C5) cycloalkyle,
m est égal à 1,
et n est tel que défini précédemment.

Une sixième combinaison correspond à des composés selon l'invention pour lesquels :
**R1** représente un groupe (C1-C4) alkyle,
**R2** représente un groupe -(CH₂)ₙ-**B** où n'= 0, 1, 2, 3, 4 et **B** est un groupe (C1-C4) alkyle,
**U** représente un groupe carbonyle,
**Y, Z, V, W** représentent un groupe -CH-,
**R3, R4** représentent un atome d'hydrogène,
**R5** représente un atome d'hydrogène,
**R7** est un atome d'hydrogène,
**R6** représente -(CH₂)ₙ-**L** où **L** représente un groupe aryle éventuellement substitué par un ou plusieurs atomes d'halogènes,
m est égal à 1,
et n est tel que défini précédemment.

Une septième combinaison correspond à des composés selon l'invention pour lesquels :
**R1** représente un groupe (C1-C4) alkyle,
**R2** représente -(CH₂)ₙ-**B** avec n' = 0 ou 1 et **B** est un groupe (C3-C5) cycloalkyle ou un groupe (C1-C4) alkyle éventuellement substitué par un ou plusieurs atomes de fluor,
**U** représente un groupe carbonyle,
**Y, Z, V, W** représentent, indépendamment les uns des autres, un hétéroatome, un groupe -CH-, un atome de carbone éventuellement susbtitué par un groupe R7 ou pas d'atome,
**R3, R4** représentent un atome d'hydrogène, ou R3 et R4 forment ensemble avec le carbone auxquels ils sont liés un groupe (C3-C5) cycloalkyle,
**R5** représente un atome d'hydrogène,
**R6** représente -(CH₂)ₙ-**L** où **L** représente un groupe hétérocycle saturé éventuellement substitué en des positions quelconques, y compris sur l'atome d'azote par un ou plusieurs substituants parmi un atome de fluor, un groupe fluoroalkyle en (C1-C4), un groupe (C1-C4) alkyle linéaire ou ramifié, un groupe (C3-C5) cycloalkyle ou un groupe (C1-C4) alkyl-sulfonamide,
**R7,** m et n sont tels que définis précédemment.

Une huitième combinaison correspond à des composés selon l'invention pour lesquels :
**R1** représente un groupe (C1-C4) alkyle,
**R2** représente un groupe -(CH₂)ₙ-**B** où n'= 0, 1, 2, 3, 4 et **B** est un groupe (C1-C4) alkyle éventuellement substitué par un groupe (C1-C4) alcoxy, **U** représente un groupe carbonyle,
**Y, Z, V, W** représentent, indépendamment les uns des autres, un hétéroatome ou un atome de carbone ou un groupe CH-,
**R7** est un atome d'hydrogène,
**R3, R4** représentent un atome d'hydrogène,
**R5** représente un atome d'hydrogène,
**R6** représente -(CH₂)ₙ-**L** où **L** représente un groupe hétéroaryle, éventuellement substitué par un (C1-C4) alkyle,
m est égal à 1,
et n est tel que défini précédemment.

Une neuvième combinaison correspond à des composés selon l'invention pour lesquels :
**R1** représente un groupe (C1-C4) alkyle,
**R2** représente un groupe -(CH₂)ₙ-**B** où n'= 0, 1, 2, 3, 4 et **B** est un groupe (C1-G4) alkyle,
**U** représente un groupe -CH₂-,
**Y, Z, V, W** représentent un groupe -CH-
**R3, R4** représentent un atome d'hydrogène,
**R5** représente un atome d'hydrogène,
**R6** représente -(CH₂)ₙ-**L** où **L** représente un groupe hétéroaryle,
m est égal à 1,
et n est tel que défini précédemment.

Parmi les composés de formule (I) objets de l'invention, on peut notamment citer les composés suivants :
2-amino-1-ethyl-N-methyl-4-oxo-7-{4-[2-oxo-2-(phenylamino)ethyl]phenyl}-1,4-dihydro-1,8-naphthyridine-3-carboxamide (composé n°1)
2-amino-1-ethyl-7-[4-(2-{[(1-ethylpyrrolidin-2-yl)methyl](methyl)amino}-2-oxoethyl)phenyl]-N-methyl-4-oxo-1,4-dihydro-1,8-naphthyridine-3-carboxamide (composé n°2)
2-amino-1-ethyl-N-methyl-4-oxo-7-{4-[2-oxo-2-(pyridin-3-ylamino)ethyl]phenyl}-1,4-dihydro-1,8-naphthyridine-3-carboxamide (composé n°3)
2-amino-1-ethyl-N-methyl-4-oxo-7-{4-[2-oxo-2-(pyridin-2-ylamino)ethyl]phenyl}-1,4-dihydro-1,8-naphthyridine-3-carboxamide (composé n°4)
2-amino-7-(4-{2-[(2-chlorophenyl)amino]-2-oxoethyl}phenyl)-1-ethyl-N-methyl-4-oxo-1,4-dihydro-1,8-naphthyridine-3-carboxamide (composé n°5)
2-amino-7-(4-{2-[(3,5-difluorophenyl)amino]-2-oxoethyl}phenyl)-1-ethyl-N-methyl-4-oxo-1,4-dihydro-1,8-naphthyridine-3-carboxamide (composé n°6)
2-amino-1-ethyl-N-methyl-4-oxo-7-(4-{2-oxo-2-[(pyridin-4-ylmethyl)amino]ethyl}phenyl)-1,4-dihydro-1,8-naphthyridine-3-carboxamide (composé n°7)
2-amino-1-ethyl-N-methyl-4-oxo-7-(4-{2-oxo-2-[(pyridin-2-ylmethyl)amino]ethyl}phenyl)-1,4-dihydro-1,8-naphthyridine-3-carboxamide (composé n°8)
2-amino-1-ethyl-7-(4-{2-[(2-methoxyethyl)amino]-2-oxoethyl}phenyl)-N-methyl-4-oxo-1,4-dihydro-1,8-naphthyridine-3-carboxamide (composé n°9)
2-amino-7-{4-[2-(cyclopropylamino)-2-oxoethyl]phenyl}-1-ethyl-N-methyl-4-oxo-1,4-dihydro-1,8-naphthyridine-3-carboxamide (composé n°10)
2-amino-1-ethyl-N-methyl-7-(4-{2-[(1-methylethyl)amino]-2-oxoethyl}phenyl)-4-oxo-1,4-dihydro-1,8-naphthyridine-3-carboxamide (composé n°11)
2-amino-7-{4-[2-(cyclopentylamino)-2-oxoethyl]phenyl}-1-ethyl-N-methyl-4-oxo-1,4-dihydro-1,8-naphthyridine-3-carboxamide (composé n°12)
2-amino-1-ethyl-N-methyl-4-oxo-7-{4-[2-oxo-2-(pyrazin-2-ylamino)ethyl]phenyl}-1,4-dihydro-1,8-naphthyridine-3-carboxamide (composé n°13)
2-amino-1-ethyl-7-{4-[2-({[(2R)-1-ethylpyrrolidin-2-yl]methyl}amino)-2-oxoethyl]phenyl}-N-methyl-4-oxo-1,4-dihydro-1,8-naphthyridine-3-carboxamide (composé n°14)
2-amino-1-ethyl-7-{4-[2-({[(2S)-1-ethylpyrrolidin-2-yl]methyl}amino)-2-oxoethyl]phenyl}-N-methyl-4-oxo-1,4-dihydro-1,8-naphthyridine-3-carboxamide (composé n°15)
2-amino-1-ethyl-N-methyl-4-oxo-7-{4-[2-oxo-2-(pyrimidin-4-ylamino)ethyl]phenyl}-1,4-dihydro-1,8-naphthyridine-3-carboxamide (composé n°16)
2-amino-1-ethyl-N-methyl-4-oxo-7-{4-[2-oxo-2-(pyridin-4-ylamino)ethyl]phenyl}-1,4-dihydro-1,8-naphthyridine-3-carboxamide (composé n°17)
2-amino-1-ethyl-N-methyl-7-(4-{2-[(2-morpholin-4-ylethyl)amino]-2-oxoethyl}phenyl)-4-oxo-1,4-dihydro-1,8-naphthyridine-3-carboxamide (composé n°18)
2-amino-1-ethyl-N-methyl-4-oxo-7-{5-[2-oxo-2-(pyridin-2-ylamino)ethyl]pyridin-2-yl}-1,4-dihydro-1,8-naphthyridine-3-carboxamide (composé n°19)
2-amino-1-ethyl-N-methyl-7-{4-[1-methyl-2-oxo-2-(pyridin-2-ylamino)ethyl]phenyl}-4-oxo-1,4-dihydro-1,8-naphthyridine-3-carboxamide (composé n°20)
2-amino-1-ethyl-N-methyl-4-oxo-7-{6-[2-oxo-2-(pyridin-2-ylamino)ethyl]pyridin-3-yl}-1,4-dihydro-1,8-naphthyridine-3-carboxamide (composé n°21)
2-amino-1-ethyl-7-[4-(2-{[(4-ethylmorpholin-3-yl)methyl]amino}-2-oxoethyl)phenyl]-N-methyl-4-oxo-1,4-dihydro-1,8-naphthyridine-3-carboxamide (composé n°22)
2-amino-1-ethyl-7-[6-(2-{[(4-ethylmorpholin-3-yl)methyl]amino}-2-oxoethyl)pyridin-3-yl]-N-methyl-4-oxo-1,4-dihydro-1,8-naphthyridine-3-carboxamide (composé n°23)
2-amino-1-ethyl-7-{4-[2-({[(2S)-1-ethyl-4,4-difluoropyrrolidin-2-yl]methyl}amino)-2-oxoethyl]phenyl}-N-methyl-4-oxo-1,4-dihydro-1,8-naphthyridine-3-carboxamide (composé n°24)
2-amino-1-ethyl-7-[4-(2-{[(4-ethylmorpholin-3-yl)methyl]amino}-1-methyl-2-oxoethyl)phenyl]-N-methyl-4-oxo-1,4-dihydro-1,8-naphthyridine-3-carboxamide (composé n°25)
2-amino-1-ethyl-7-{4-[2-({[(2S,4R)-1-ethyl-4-fluoropyrrolidin-2-yl]methyl}amino)-2-oxoethyl]phenyl}-N-methyl-4-oxo-1,4-dihydro-1,8-naphthyridine-3-carboxamide (composé n°26)
2-amino-1-ethyl-7-{4-[2-({[(2R)-1-(2-fluoroethyl)pyrrolidin-2-yl]methyl}amino)-2-oxoethyl]phenyl}-N-methyl-4-oxo-1,4-dihydro-1,8-naphthyridine-3-carboxamide (composé n°27)
2-amino-1-ethyl-N-methyl-7-{4-[2-({[(2R)-1-(methylsulfonyl)pyrrolidin-2-yl]methyl}amino)-2-oxoethyl]phenyl}-4-oxo-1,4-dihydro-1,8-naphthyridine-3-carboxamide (composé n°28)
2-amino-1-ethyl-7-{5-[2-({[(2R}-1-(2-fluoroethyl)pyrrolidin-2-yl]methyl}amino)-2-oxoethyl]pyridin-2-yl}-N-methyl-4-oxo-1,4-dihydro-1,8-naphthyridine-3-carboxamide (composé n°29)
2-amino-1-ethyl-N-methyl-4-oxo-7-{4-[2-oxo-2-({[(2R)-1-(2,2,2-trifluoroethyl)pyrrolidin-2-yl]methyl}amino)ethyl]phenyl}-1,4-dihydro-1,8-naphthyridine-3-carboxamide (composé n°30)
2-amino-7-{4-[2-({[(2R)-1-(2,2-difluoroethyl)pyrrolidin-2-yl]methyl}amino)-2-oxoethyl]phenyl}-1-ethyl-N-methyl-4-oxo-1,4-dihydro-1,8-naphthyridine-3-carboxamide (composé n°31)
2-amino-1-ethyl-N-methyl-7-{4-[2-({[4-(1-methylethyl)morpholin-3-yl]methyl}amino)-2-oxoethyl]phenyl}-4-oxo-1,4-dihydro-1,8-naphthyridine-3-carboxamide (composé n°32)
2-amino-7-[4-(2-{[(4-cyclopropylmorpholin-3-yl)methyl]amino}-2-oxoethyl)phenyl]-1-ethyl-N-methyl-4-oxo-1,4-dihydro-1,8-naphthyridine-3-carboxamide (composé n°33)
2-amino-7-{5-[2-({[(2R)-1-(2,2-difluoroethyl)pyrrolidin-2-yl]methyl}amino)-2-oxoethyl]pyridin-2-yl}-1-ethyl-N-methyl-4-oxo-1,4-dihydro-1,8-naphthyridine-3-carboxamide (composé n°34)
2-amino-1-ethyl-N-methyl-4-oxo-7-{4-[2-oxo-2-(1,3-thiazol-2-ylamino)ethyl]phenyl}-1,4-dihydro-1,8-naphthyridine-3-carboxamide (composé n°35)
2-amino-1-ethyl-N-methyl-7-[4-(2-{[(1-methyl-1H-imidazol-5-yl)methyl]amino)-2-oxoethyl)phenyl]-4-oxo-1,4-dihydro-1,8-naphthyridine-3-carboxamide (composé n°36)
2-amino-1-ethyl-N-methyl-4-oxo-7-(4-{2-oxo-2-[(2-pyridin-3-ylethyl)amino]ethyl}phenyl)-1,4-dihydro-1,8-naphthyridine-3-carboxamide (composé n°37)
2-amino-1-ethyl-N-methyl-7-(4-{2-[(3-morpholin-4-ylpropyl)amino]-2-oxoethyl}phenyl)-4-oxo-1,4-dihydro-1,8-naphthyridine-3-carboxamide (composé n°38)
2-amino-1-ethyl-N-methyl-4-oxo-7-(4-{2-oxo-2-[(2-pyridin-2-ylethyl)amino]ethyl}phenyl)-1,4-dihydro-1,8-naphthyridine-3-carboxamide (composé n°39)
2-amino-1-ethyl-N-methyl-4-oxo-7-(4-{2-oxo-2-[(2-phenylethyl)amino]ethyl}phenyl)-1,4-dihydro-1,8-naphthyridine-3-carboxamide (composé 40)
2-amino-1-ethyl-N-methyl-4-oxo-7-(4-{2-oxo-2-[(3-phenylpropyl)amino]ethyl}phenyl)-1,4-dihydro-1,8-naphthyridine-3-carboxamide (composé 41)
2-amino-1-ethyl-N-methyl-7-[4-(2-{[2-(1-methyl-1H-pyrrol-2-yl)ethyl]amino}-2-oxoethyl)phenyl]-4-oxo-1,4-dihydro-1,8-naphthyridine-3-carboxamide (composé n°42)
2-amino-1-ethyl-7-[4-(2-{[(1-ethylpyrrolidin-2-yl)methyl]amino}-2-oxoethyl)-1,3-thiazol-2-yl]-N-methyl-4-oxo-1,4-dihydro-1,8-naphthyridine-3-carboxamide (composé n°43)
2-amino-1-(3-methoxypropyl)-N-methyl-4-oxo-7-{4-[2-oxo-2-(pyridin-2-ylamino)ethyl]phenyl}-1,4-dihydro-1,8-naphthyridine-3-carboxamide (composé n°44)
2-amino-7-[4-(2-{[1-(2,2-difluoroethyl)pyrrolidin-3-yl]amino}-2-oxoethyl)phenyl]-1-ethyl-N-methyl-4-oxo-1,4-dihydro-1,8-naphthyridine-3-carboxamide (composé n°45)
2-amino-1-ethyl-7-[4-(3-{[(1-ethylpyrrolidin-2-yl)methyl]amino}-3-oxopropyl)phenyl]-N-methyl-4-oxo-1,4-dihydro-1,8-naphthyridine-3-carboxamide (composé n°46)
2-amino-1-ethyl-7-[4-(4-{[(1-ethylpyrrolidin-2-yl)methyl]amino}-4-oxobutyl)phenyl]-N-methyl-4-oxo-1,4-dihydro-1,8-naphthyridine-3-carboxamide (composé n°47)
2-amino-1-ethyl-7-[4-(2-{[(1-ethylpyrrolidin-2-yl)methyl]amino}-2-oxoethyl)phenyl]-4-oxo-N-propyl-1,4-dihydro-1,8-naphthyridine-3-carboxamide (composé n°48)
2-amino-7-[4-(2-{[(1-ethylpyrrolidin-2-yl)methyl]amino}-2-oxoethyl)phenyl]-N-methyl-4-oxo-1-(2,2,2-trifluoroethyl)-1,4-dihydro-1,8-naphthyridine-3-carboxamide (composé n°49)
2-amino-1-cyclopentyl-7-[4-(2-{[(1-ethylpyrrolidin-2-yl)methyl]amino}-2-oxoethyl)phenyl]-N-methyl-4-oxo-1,4-dihydro-1,8-naphthyridine-3-carboxamide (composé n°50)
2-amino-1-ethyl-7-[4-(5-{[(1-ethylpyrrolidin-2-yl)methyl]amino}-5-oxopentyl)phenyl]-*N*-methyl-4-oxo-1,4-dihydro-1,8-naphthyridine-3-carboxamide (composé n°51)
1-ethyl-7-[5-(2-{[(1-ethylpyrrolidin-2-yl)methyl]amino}-2-oxoethyl)thiophen-2-yl]-*N*,2-dimethyl-4-oxo-1,4-dihydro-1,8-naphthyridine-3-carboxamide (composé n°52)
2-amino-7-{4-[2-({[(2R)-1-ethylpyrrolidin-2-yl]methyl}amino)-2-oxoethyl]phenyl}-N-methyl-1-(2-methylpropyl)-4-oxo-1,4-dihydro-1,8-naphthyridine-3-carboxamide (composé 53)
2-amino-1-ethyl-7-{4-[1-({[(2R)-1-ethylpyrrolidin-2-yl]methyl}carbamoyl)cyclopropyl]phenyl}-N-methyl-4-oxo-1,4-dihydro-1,8-naphthyridine-3-carboxamide (composé 54)
2-amino-1-ethyl-7-{2-[2-({[(2R)-1-ethylpyrrolidin-2-yl]methyl}amino)-2-oxoethyl]-1,3-thiazol-4-yl}-N-methyl-4-oxo-1,4-dihydro-1,8-naphthyridine-3-carboxamide (composé 55)
2-amino-1-ethyl-7-{4-[2-({[(2R)-1-ethylpyrrolidin-2-yl]methyl}amino)-2-oxoethyl]-2-fluorophenyl}-N-methyl-4-oxo-1,4-dihydro-1,8-naphthyridine-3-carboxamide (composé 56)
2-amino-7-[3-chloro-4-(2-{[(1-ethylpyrrolidin-2-yl)methyl]amino}-2-oxoethyl)phenyl]-1-ethyl-N-methyl-4-oxo-1,4-dihydro-1,8-naphthyridine-3-carboxamide (composé 57)
2-amino-7-[3-fluoro-4-(2-{[(1-ethylpyrrolidin-2-yl)methyl]amino}-2-oxoethyl)phenyl]-1-ethyl-N-methyl-4-oxo-1,4-dihydro-1,8-naphthyridine-3-carboxamide (composé 58)
2-amino-7-[3-methyl-4-(2-{[(1-ethylpyrrolidin-2-yl)methyl]amino}-2-oxoethyl)phenyl]-1-ethyl-N-methyl-4-oxo-1,4-dihydro-1,8-naphthyridine-3-carboxamide (composé 59)
2-amino-1-(cyclopropylmethyl)-7-[4-(2-{[(1-ethylpyrrolidin-2-yl)methyl]amino}-2-oxoethyl)phenyl]-N-methyl-4-oxo-1,4-dihydro-1,8-naphthyridine-3-carboxamide (composé 60)
2-amino-1-ethyl-7-[4-(2-{[(1-ethylpyrrolidin-2-yl)methyl]amino}-2-oxoethyl)-2-methylphenyl]-*N*-methyl-4-oxo-1,4-dihydro-1,8-naphthyridine-3-carboxamide (composé 61)
2-amino-1-ethyl-7-[3-(2-{[(1-ethylpyrrolidin-2-yl)methyl]amino}-2-oxoethyl)phenyl]-N-methyl-4-oxo-1,4-dihydro-1,8-naphthyridine-3-carboxamide (composé n°62)
2-amino-1-ethyl-N-methyl-4-oxo-7-{3-[2-oxo-2-(pyridin-2-ylamino)ethyl]phenyl}-1,4-dihydro-1,8-naphthyridine-3-carboxamide (composé n°63)
2-amino-1-ethyl-*N*-methyl-4-oxo-7-{4-[2-(pyridin-2-ylamino)ethyl]phenyl}-1,4-dihydro-1,8-naphthyridine-3-carboxamide (composé 64).

Il est à noter que les composés ci-dessus ont été nommés en nomenclature IUPAC par l'intermédiaire du logiciel ACDLABS 10.0 ACD/name (Advanced Chemistry development).

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et coll., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, 1985, p. 310-316.

Conformément à l'invention, on peut préparer les composés de formule générale (I) selon le procédé qui suit.

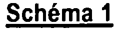

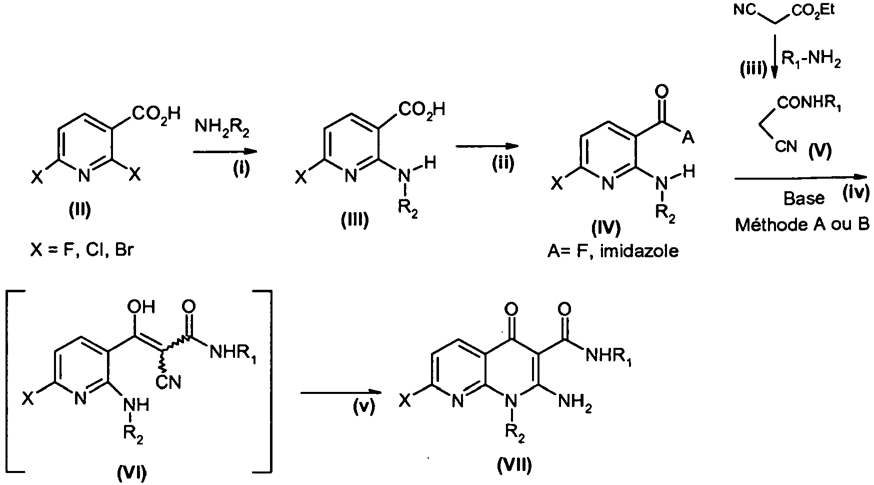

Selon le schéma 1, un acide 2,6-dihalogénonicotinique de formule (II) est mono-substitué en position 2 par une amine de formule R₂-NH₂ (où R₂ est tel que défini précédemment en rapport avec les composés de formule (I), à température ambiante, ou à une température de 50°C à 100°C, en chauffage classique ou par micro-onde et dans un solvant protique tel qu'un alcool par exemple éthanol, n-butanol, *tert*-butanol ou l'eau. L'acide (III), issu de l'étape (i), est ensuite activé en dérivé de formule (IV) soit sous forme de fluorure d'acide par l'action du fluorure de cyanuryle à température ambiante, en présence d'une base telle que la triéthylamine ou la pyridine et dans un solvant tel que le dichlorométhane ou le THF, comme décrit par G. OLAH et coll. dans Synthesis (1973), 487, ou sous forme d'imidazolide par l'action du carbodiimidazole dans un solvant tel que le DMF ou le THF ou par d'autres méthodes connues de l'Homme de l'art, telles que celles décrites par MUKAIYAMA et TANAKA dans Chem. Lett. (1976), 303 ou par ISHIKAWA et SASAKI dans Chem. Lett. (1976), 1407.

Le fluorure d'acide ou l'imidazolide de formule (IV) obtenus à l'issue de l'étape (ii), très réactifs mais stables, sont mis ensuite en réaction avec un cyanoacétamide N-substitué de formule (V) selon les methodes A ou B.

Suivant la méthode A, deux équivalents d'une base telle que l'hydrure de sodium ou le tert-butoxyde de potassium, sont utilisés pour l'étape (iv) de condensation du dérivé cyanoacétamide N-substitué (V), avec un composé de formule (IV), après une nuit à température ambiante, on obtient un β-céto-cyanoacétamide de formule (VI), qui est ensuite cyclisé en pyridino-pyridinone de formule (VII) par chauffage à une température comprise entre 90 et 125°C dans un solvant polaire tel que le n-butanol, le DMSO ou le DMF.

La methode B est similaire à la méthode A pour l'étape de condensation (iv) mais au mélange réactionnel, un troisième équivalent de la base utilisée est additionné et le composé de formule (VI) formé, se cyclise in situ, à température ambiante, pour fournir directement le composé pyridino-pyridinone de formule (VII).

Les N-alkylcyanoacétamides de formule (V) sont préparés selon l'étape (iii) en faisant réagir le cyanoacétate d'ethyle avec un excès d'amine de formule R₁-NH₂ (où R₁ est tel que défini précédemment en rapport avec les composés de formule (I) objets de l'invention) dans un solvant tel que le THF ou l'éthanol, à température allant de la température ambiante à la température de reflux du solvant.

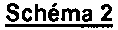

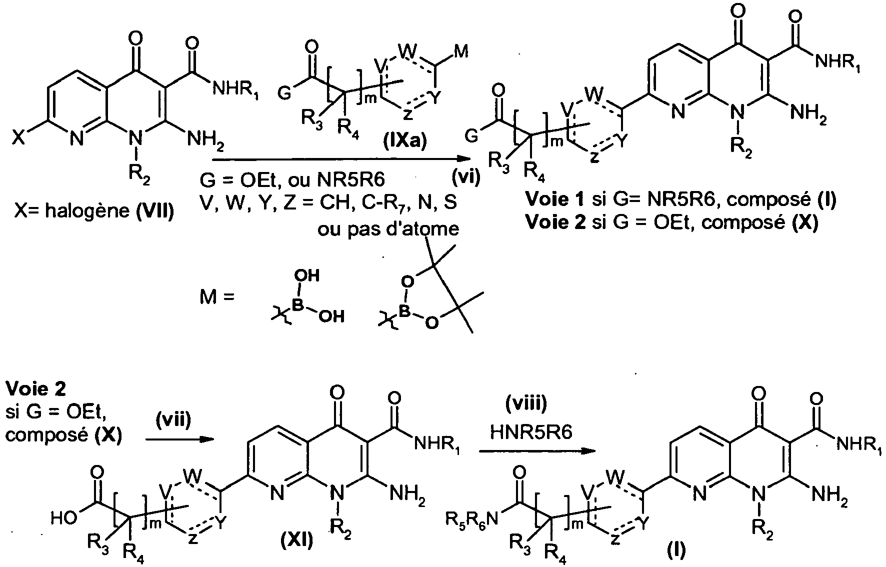

Pour l'obtention des composés de formule (I) objet de la présente invention deux méthodes sont utilisables à partir de l'intermédiaire halogéné de formule (VII). Suivant la méthode 1 décrite dans le schéma 2, l'intermédiaire (VII) est engagé à l'étape (vi) dans une réaction de couplage de SUZUKI avec un acide boronique ou un ester boronique de bispinacol (IXa) et m, R3 et R4 et V, W, Y, Z sont tels que défini précédemment en rapport avec les composés de formule (I) objets de l'invention étant entendu que le cycle doit comprendre entre 5 et 6 chainons, et G est soit un groupement (C1-C4)alcoxy tel que OEt ou un motif -NR5R6, où R5 et R6 sont tels que définis pour le composé de formule (I). Cette réaction (vi) est faite en présence d'un complexe de palladium (à l'état d'oxydation (0) ou (II)) tel que par exemple Pd(PPh₃)₄, PdCl₂(PPh₃)₂, Pd₂dba₃, Xphos ou PdCl₂(dppf), dans un solvant polaire, protique ou non tel que le DME, l'éthanol, le DMF, le dioxanne, ou des mélanges de ces solvants, en présence d'une base telle que le carbonate de césium, l'hydrogénocarbonate de sodium aqueux, ou K₃PO₄, en chauffant classiquement entre 80 et 120 °C ou bien sous l'action des micro-ondes entre 130 et 170°C.

Dans le cas où G est un motif NR5R6, où R5 et R6 sont tels que définis pour le composé de formule (I), c'est la voie 1, le composé de formule (I) objet de l'invention est directement obtenu à l'issu de ce couplage (vi).

Dans le cas où G est un groupement (C1-C4)alcoxy tel que OEt suivant la voie 2, c'est le composé (X) qui est obtenu par le couplage de Suzuki de l'étape (vi). Le composé (X) est ensuite saponifié, à l'étape (vii) en présence d'un réactif nucléophile tel que LiOH ou de NaOH en solution aqueuse, dans un solvant tel qu'un solvant polaire tel que le THF, DMF, le MeOH ou EtOH, à une température allant de température ambiante à 80°C, pour donner le composé (XI) qui est ensuite engagé dans une réaction de couplage peptidique, dans l'étape (viii), avec l'amine HNR5R6 choisie, où R5 et R6 sont tels que définis pour le composé de formule (I), en présence d'un agent de couplage tel que TBTU, HBTU ou le CDI et d'une base, par exemple la diisopropyléthylamine, la triéthylamine ou le NaHCO₃, dans un solvant aprotique tel que le dichlorométhane, le THF ou le DMF ou par d'autres méthodes connues de l'Homme de l'art, telles que celles décrites par « Principales of Peptide Synthesis », 2^{nd} Ed 1993 M Bodanszky, Springer Laboratory , pour donner le composé de formule (I) objet de l'invention.

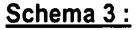

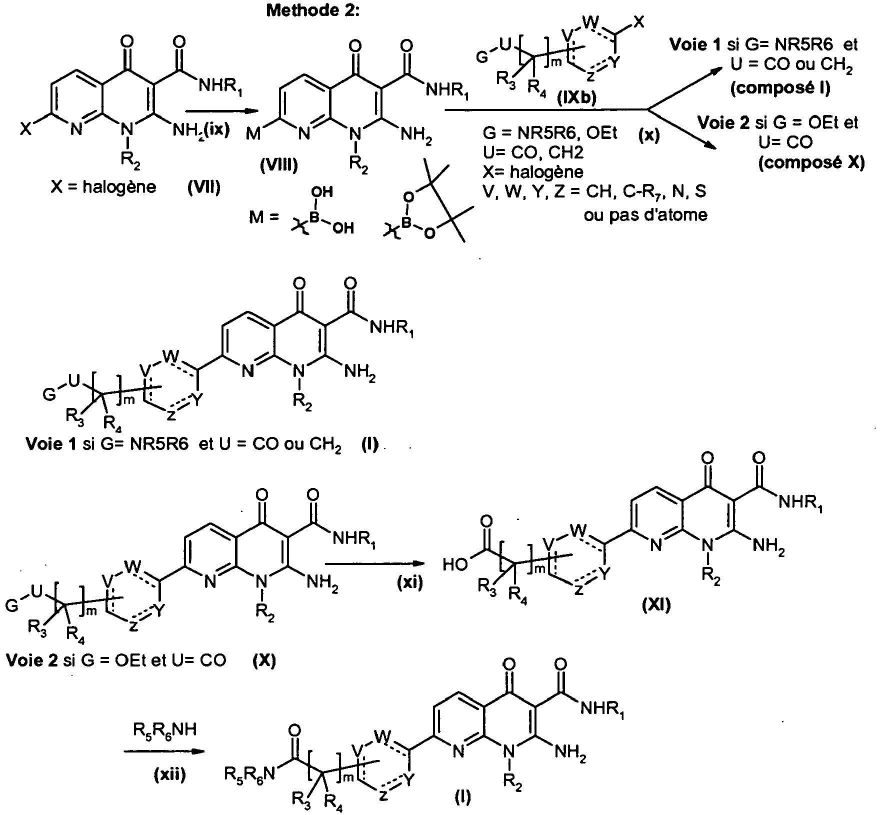

Pour l'obtention des composés de formule (I) objet de la présente invention, une seconde méthode est utilisable à partir de l'intermédiaire halogéné de formule (VII), cette méthode 2 est décrite dans le schéma 3. L'intermédiaire halogéné de formule (VII) peut être transformé en dérivé d'ester boronique de bispinacol ou d'acide boronique de formule (VIII) suivant l'étape (ix), par réaction avec le bis(pinacolato)diborane en présence de [1,1'-bis(diphénylphosphino)ferrocène] dichloropalladium (II) et d'acétate de potassium ou de carbonate de potassium dans un solvant polaire tel que le DMSO, le DMF, le DME ou le dioxanne, à une température comprise entre 50 et 100°C, selon la méthodologie décrite par ISHIYAMA, T. et coll. dans J. Org. Chem., 1995, 60, 7508-7510 et GIROUX, A. et coll. dans Tet. Lett., 1997, 38, 3841-3844. Dans l'étape (x) suivante, le composé acide ou ester boronique (VIII) est engagé dans une réaction de type Suzuki, avec un composé aromatique halogéné de formule (IXb) où X, m, R₃ et R₄, U, et V, W, Y, Z sont tels que défini précédemment en rapport avec les composés de formule (I) objets de l'invention étant entendu que le cycle doit comprendre entre 5 et 6 chainons et G est soit un groupement (C1-C4)alcoxy tel que OEt ou un motif - NR5R6, où R5 et R6 sont tels que définis pour le composé de formule (I). Pour cette réaction (x) les conditions de couplage de SUZUKI décrites à l'étape (vi) du schéma 2, peuvent être appliquées.

Dans le cas où G est un motif NR5R6, où R5 et R6 sont tels que définis précédemment et U est soit un carbonyle soit un motif méthylène, le composé de formule (I) objet de l'invention est directement obtenu à l'issu de l'étape de couplage (x) (voie 1).

Dans le cas où G est un groupement OEt et U est un carbonyle, c'est la voie 2 qui est suivie, et c'est le composé (X) qui est obtenu par ce couplage de Suzuki (x). Ce composé (X) est ensuite engagé dans l'étape (xi) donnant le composé acide (XI) lui-même transformé dans l'étape (xii) en composé de fomule (I) objet de l'invention. Les étapes (xi) et (xii) sont identiques respectivement aux étapes (vii) et (viii) décrites précédemment.

Quand les modes de préparation, des composés de départ, des réactifs, tel que les amines HNR5R6 ou des composés de formule IXa et IXb, utilisés dans les schémas 1, 2 et 3 ne sont pas décrits, ils sont soit disponibles commerçialement ou bien peuvent être préparés selon des méthodes qui sont décrites dans la littérature ou connues de l'Homme du métier.

Si nécessaire, certaines fonctions réactives situées sur le groupe R₂, R₅ ou R₆ peuvent être protégées au cours de ces réactions par des groupes protecteurs, tels que décrits dans « Protective Groups in Organic Synthesis », Green et coll., 2nd Edition (John Wiley & Sons, Inc., New York).

L'invention, selon un autre de ses aspects, a également pour objet les composés de formules (VIII), (X), (XI). Ces composés sont utiles comme intermédiaires de synthèse des composés de formule (I).

Les exemples suivants illustrent la préparation de certains composés conformes à l'invention. Ces exemples ne sont pas limitatifs et ne font qu'illuster la présente invention. Les numéros des composés exemplifiés renvoient à ceux donnés dans le tableau ci-après, qui illustre les structures chimiques et les propriétes physiques de quelques composés selon l'invention.

Les abréviations et formules brutes suivantes sont utilisées :
- AcOEt: Acétate d'éthyle
- CDI: Carbonyldiimidazole
- DCM: Dichlorométhane
- °C: degré Celsius
- DME: Diméthoxyéthane
- DMF: Diméthylformamide
- DMSO: Diméthyl sulfoxide
- EDC HCl: Chlorhydrate de N-[3-(diméthylamino)propyl-N'-éthyl carbodiimide
- FAMSO: méthylthiométhyl sulfoxide de méthyle
- HBTU: Hexafluorophosphate de O-(-benzotriazol-1-yl)-N, N, N', N'-tetraméthyluronium.
- h: Heure(s)
- HCl: Acide chlorhydrique
- LiOH: Hydroxyde de lithium
- Na₂CO₃: Carbonate de sodium
- NH₄Cl: Chlorure d'ammonium
- NaHCO₃: Hydrogénocarbonate de sodium
- Na₂SO₄: Sulfate de sodium
- NaCl: Chlorure de sodium
- NaOH: Hydroxyde de sodium
- NH₄OH: hydoxyde d'ammonium
- Na₂SO₄: Sulfate de sodium
- min.: minutes
- ml: millilitre
- P₂O₅: di-phosphore pentaoxide
- TBTU: Tétrafluoroborate de N-[(1H-benzotriazol-1-yloxy) (diméthylamino)méthylidène]-N-méthylméthanaminium
- THF: Tétrahydrofurane
- T.A.: Température ambiante
- Xphos: 2-Dicyclohexylphosphinol-2',4',6'-triisopropylbiphényle
Appareil de micro-ondes utilisé: Biotage, initiator

### Condition d'analyse :

### Conditions de couplage LC/UV/MS :

Instrument (Agilent) : chaîne HPLC : Série 1100, Spectromètre de masse MSD SL (Agilent), Logiciel : Chemstation version B.01.03 de Agilent

### LClUV

Colonne : Symmetry C18 3.5µm (2.1 x 50 mm) (Waters), Temp. colonne :25°C,
Post run : 5 min Détection UV : 220 nm. Volume d'injection : 2µl d'une solution à 0,5 mg/ml

### Condition 1 : pH 3 gradient 15 minutes

Eluants : A : H₂O + 0,005 % TFA / B : CH₃CN + 0,005 % TFA, Débit : 0.4 ml/min. Gradient : 0 à 10 min 0 à 100% B et de 10 à 15 min 100 B%

### Condition 2 : pH 3 gradient 30 minutes

Colonne : Symmetry C18 3.5µm (2.1 x 50 mm) (Waters), Temp. colonne :25°C, Eluants : A : H₂O + 0,005 % TFA / B : CH₃CN + 0,005 % TFA, Débit : 0.4ml/min. Gradient : 0 à 30 min 0 à 100% B et de 30 à 35 min 100 B%
Post run : 6 min. Détection UV : 220 nm. Volume d'injection : 2µl d'une solution à 0,5 mg/ml

### Condition 3 : pH 7 gradient 20 minutes

Colonne : X terra MS C18 3.5 µm (2.1X50 mm), Temp colonne : 20°C Eluants : A H₂O + AcNH₄ (5 nM) + 3% CH₃CN / B : CH₃CN . Gradient 0 à 20 min 0 à 100% de B. Détection UV : 210 nm.

### Condition GC Cl/CH4+) : ionisation Cl/CH4 +, 30 minutes

Colonne : Agilent HP-5MS 30 m x 250 µm film 0,25 µm d'épaisseur. Température 250 °C, Gaz vecteur : Helium, débit constant 1,4 ml/ min

### Spectrométrie de masse (MS)

mode d'ionisation : Electrospray mode positif ESI+, Gamme de masse : 90-1500 uma
Spray Chamber Gas temp. : 350°C Drying gas (N₂) :10.0 l/min Neb. pressure : 30 psig Vcap : 4000 V

Les spectres RMN ¹H ont été obtenu en utilisant des Spectromètres RMN Bruker 250, 300 ou 400 MHz dans du DMSO-d6, en utilisant le pic du DMSO-d5 comme référence. Les déplacements chimiques δ sont exprimés en partie par million (ppm). Les signaux observés sont exprimés ainsi : s = singulet ; d = doublet ; t = triplet ; m = massif ou large singulet ; H = proton.

Les points de fusions inférieurs à 260°C ont été mesurés avec un appareil banc Koffler et les points de fusions supérieurs à 260°C ont été mesurés avec un appareil Buchi B-545.

Les pouvoirs rotatoires ont été mesurés sur un polarimètre de type: Polarimeter Perkin-Elmer, energy 55µA.

### Exemple 1 : Chlorhydrate de 2-amino-1-ethyl-7-{4-[2-({[(2R)-1-(2-fluoroethyl)pyrrolidin-2-yl]methyl}amino)-2-oxoethyl]phenyl}-N-methyl-4-oxo-1,4-dihydro-1,8-naphthyridine-3-carboxamide (composé n°27)

### 1.1 : 6-chloro-2-(ethylamino)pyridine-3-carboxylic acid :

Une solution de 18,0 g (84,4 mmol) d'acide 2,6-dichloronicotinique dans 180 ml (3,45 mol) d'une solution d'éthylamine à 70 % dans l'eau est chauffée à 50°C pendant 10 heures. L'excès d'amine est alors évaporé sous pression réduite, puis une solution aqueuse d'acide acétique à 10 % est additionnée jusqu'à la précipitation du produit. Le solide beige est essoré, rincé avec de l'eau froide et séché à l'étuve. On obtient 10,5 g du produit attendu. Rendement = 62 %. Point de fusion : 158-160°C. MH⁺: 201,1 (tr: 7.7 min, condition 1).

### 1.2 : 6-chloro-2-(ethylamino)pyridine-3-carbonyl fluoride

A une suspension de 10,5 g (52,3 mmol) du composé obtenu à l'issue de l'étape 1.1 dans le dichlorométhane (250 ml), 4,2 ml (52,3 mmol) de pyridine et 8,4 ml (99,6 mmol) de fluorure cyanurique sont ajoutés successivement. Le mélange est agité pendant 3 heures à température ambiante puis filtré. Le solide est rincé au dichlorométhane (100 ml) et le filtrat est lavé deux fois à l'eau glacée (60 ml). La phase organique est séchée sur Na₂SO₄ puis concentrée sous pression réduite. 10,44 g de produit sont obtenus, sous forme d'huile orange. Rendement = 99 %. Le produit est utilisé sans purification dans l'étape suivante.

### 1.3 : 2-cyano-N-methylacetamide

A 10,9 g (353,6 mmol) d'une solution de méthylamine dans le THF refroidie à 0°C, 20 g (176,8 mmol) d'éthyle cyanoacétate sont ajoutés goutte à goutte puis le mélange réactionnel est agité à température ambiante pendant une nuit. Les solvants sont évaporés sous pression réduite et le produit est purifié par recristallisation dans le toluène. 16,8 g de produit sont obtenus, sous forme d'un solide beige. Rendement = 96 %. Point de fusion = 99°C.

### Méthode A (1.4 et 1.5 ci-après)_{.}

### 1.4 : 3-[6-chloro-2-(ethylamino)pyridin-3-yl]-2-cyano-3-hydroxy-N-methylprop-2-enamide 2-enamide

A une solution, refroidie à 0-5°C, de 9,80 g (100 mmol), du composé obtenu à l'issue de l'étape 1.3, dans 100 ml de DMF anhydre, 3,98 g (100 mmol) d'hydrure de sodium à 60 % dans de l'huile minérale sont ajoutés par petites quantités. Après la fin du dégagement d'hydrogène, le mélange est agité pendant 10 minutes à température ambiante, puis refroidit à nouveau à 0-5°C. Une solution de 10,1 g (49,8 mmol) du composé obtenu à l'issue de l'étape 1.2, dans 60 ml de DMF, est additionnée et le mélange est agité à température ambiante pendant une nuit puis 2,85 ml (49,8 mmol) d'acide acétique sont ajoutés. Le DMF est évaporé sous pression réduite puis le résidu est repris dans de l'eau et le produit est extrait deux fois avec un mélange dichlorométhane : méthanol dans des proportions de 95 pour 5, puis une fois avec un mélange acétate d'éthyle : THF (2:1). Les phases organiques combinées sont séchées sur MgSO₄, puis les solvants sont évaporés sous pression réduite. On obtient 19,0 g de produit utilisé tel quel dans l'étape suivante.

### 1.5 : 2-amino-7-chloro-1-ethyl-N-methyl-4-oxo-1,4-dihydro-1,8-naphthyridine-3-carboxamide

Une solution de 19,0 g (49,8 mmol) du produit brut obtenu à l'issue de l'étape 1.4 dans 600 ml de n-butanol est chauffée pendant 48 heures à 110°C. Le solvant est évaporé sous pression réduite et le solide obtenu est trituré dans du méthanol. Le solide est ensuite filtré et séché à l'étuve. On obtient 7,9 g du produit attendu sous forme de solide jaune pâle. Rendement = 57 %. Point de fusion : 283-286°C. MH⁺: 281.2 (tr= 6,99 min, condition 1)

### Méthode B (1.6 ci-après au lieu de 1.4 et 1.5).

### 1.6 : 2-amino-7-chloro-1-éthyl-N-méthyl-4-oxo-1,4-dihydro-1,8-naphthyridine-3-carboxamide

A une solution refroidie à 0-5 °C de 0,48 g (4,9 mmol) du composé obtenu à l'issue de l'étape 1.3, dans du DMF anhydre (7 ml), 0,4 g (9,95 mmol) d'hydrure de sodium à 60 % dans l'huile minérale sont ajoutés par petites portions. Le mélange est agité à cette température pendant dix minutes puis une solution de 1,0 g (4,93 mmol) du composé obtenu à l'issue de l'étape 1.2 dans du DMF anhydre (5 ml) est ajoutée. Le mélange réactionnel est agité pendant 1 nuit à température ambiante puis 0,2 g (4,9 mmol) d'hydrure de sodium à 60 % sont rajoutés par petites portions. L'agitation est poursuivie à cette température pendant 30 minutes puis 0,56 ml (9,8 mmol) d'acide acétique sont additionnés, suivi de 60 ml d'eau et le solide est filtré, rincé à l'eau puis séché à l'étuve. On obtient 1,30 g du produit attendu. Rendement = 94 %. Point de fusion : 283-284°C. MH⁺: 281,2 (tr= 6,99 min, condition 1)

### 1.7 [7-amino-8-ethyl-6-(methylcarbamoyl)-5-oxo-5,8-dihydro-1,8-naphthyridin-2-yl]boronic acid

Une suspension de 8 g (0,03 mol) du composé obtenu à l'issue de l'étape 1.5 ou 1.6 (selon que la méthode A ou B a été employée), de 8,0 g (0,03 mol) de bis(pinacolato) diborane et de 8,5 g (0,08 mol) d'acétate de potassium dans le diméthylsulfoxyde (130 ml), est dégazée à l'argon pendant 15 minutes. 1,4 g (1,7 mmol) de [1,1'-bis(diphenylphosphino)ferrocène]dichloropalladium(II), complexé avec du dichlorométhane (1 :1) est additionné et le mélange est chauffé à 80°C pendant 30 minutes, sous argon, puis refroidi et dilué avec 1,1 l d'eau et acidifié à pH = 4 par ajout d'acide acétique (50 ml). Le mélange est filtré et le précipité noir est lavé à l'eau (40 ml) puis à l'éther (60 ml). Le résidu noir est repris dans 575 ml d'une solution NaOH (1N) et le mélange est filtré sur célite 545. Le filtrat est acidifié avec 60 ml d'acide acétique et le précipité est filtré, lavé à l'eau et à l'éther puis séché à l'étuve. 6,85 g de produit sont obtenu sous forme d'une poudre blanche. Rendement = 83%. Point de fusion : 335°C. MH⁺: 291,2 (tr= 5,3 min, condition 1) RMN ¹H (250MHZ, DMSO-d₆), δ (ppm) : 11,69 (s, 1 H); 11,12 (q, 1 H, 4,67 Hz); 8,47 (s, 2H); 8,44 (d, 1H, 7,7Hz); 7,9(s, 1H); 7,75(d, 1H, 7,7Hz); 4,72 (m, 2H); 2,8 (d, 3H, 4,67 Hz); 1,22(t, 3H, 6,9 Hz).

### 1.8: 1-trityl-D-prolinamide

A froid (bain de glace) et sous atmosphère inerte, 10 ml de triéthylamine (69,7 mmol) sont ajoutés à 5 g (33,2 mmol) de chlorhydrate de prolinamide en suspension dans le chloroforme (50 ml), 9,7 g (34,9 mmol) de chlorure de trityle sont ensuite additionnés par portion. La réaction est laissée sous agitation à température ambiante pendant 2 jours. Le mélange réactionnel est dissout dans le dichlorométhane (200 ml) et lavé successivement 2 fois avec une solution d'HCl (1N) (150 ml), à l'eau (150 ml), avec une solution de NaHCO₃ saturée (150 ml) et une solution de NaCl saturée (150 ml). La phase organique est séchée (Na₂SO₄), filtrée et évaporée sous pression réduite. Le résidu est recristallisé dans 20 ml d'éthanol chaud, 11 g du composé sont obtenus sous la forme de cristaux blancs. Rendement = 93 %. Point de fusion : 162°C

### 1.9: 1-[(2R)-1-tritylpyrrolidin-2-yl]methanamine

A froid (bain de glace) et sous atmosphère inerte, 60 ml (61,6 mmol) d'une solution d'hydrure de lithium et d'aluminium (1 N) dans le THF sont ajoutés goutte à goutte à 11 g (30,8 mmol) du composé obtenu à l'issue de l'étape 1.8 en suspension dans le THF (60 ml). Après addition le mélange réactionnel est chauffé 3 heures à 70°C, puis refroidi à 0°C. Sont alors additionnés goutte à goutte, successivement 2,8 ml d'eau (formation d'un précipité), 2,8 ml de solution de NaOH (1N) et 8,3 ml d'eau. Ce mélange est agité 30 minutes puis filtré, le solide est rincé au THF (10 ml) puis le filtrat est concentré sous pression réduite. 10 g de composé sont obtenus sous forme de poudre jaune vif et utilisé tel que dans l'étape suivante. Rendement = 95 %. Point de fusion : 100°C.

### 1.10: 2-(4-iodophenyl)-N-[(2R)-pyrrolidin-2-ylmethyl]acetamide

Sous atmosphère inerte et à température ambiante, 4 ml (43,8 mmol) de chlorure d'oxalyle sont ajoutés à 3,8 g (14,6 mmol) d'acide 4-iodophényle acétique en suspension dans le dichlorométhane (54 ml). Deux gouttes de DMF sont introduites et le mélange réactionnel est agité une heure à température ambiante, puis est concentré sous pression réduite. Le résidu est repris dans le toluène et concentré à nouveau. Le chlorure d'acide ainsi obtenu est mis en solution dans le dichlorométhane (10 ml) et additionné goutte à goutte à 5 g (14,6 mmol) du composé obtenu à l'issue de l'étape 1.9 en suspension dans le dichlorométhane (30 ml) en présence de 2,45 g (29,2 mmol) de NaHCO₃ à froid (bain de glace) et sous atmosphère inerte. Le milieu réactionnel est agité pendant une nuit à température ambiante, puis filtré sur célite et concentré sous pression réduite. 8,5 g de produit sont obtenus sous forme de mousse jaune, et directement utilisés dans l'étape suivante sans purification. MH⁺: 586

8,5 g (14,6 mmol) de ce composé, obtenu à l'issue de l'étape précédente sont repris dans un mélange éthanol/ HCl 35% (41 ml / 3,2 ml) à froid (bain de glace). Le milieu réactionnel est agité 2 heures à température ambiante. Le milieu réactionnel est concentré sous pression réduite, repris dans l'eau (30 ml) et extrait à l'éther (30 ml x2). La phase aqueuse, refroidie, est basifiée à l'aide d'une solution de NaOH 35% versée goutte à goutte jusqu'à pH=10. Le produit est alors extrait au dichlorométhane (100 ml x 6). La phase organique est séchée (Na₂SO₄), filtrée et évaporée sous pression réduite. 4,6 g de composé sont obtenus, sous forme de poudre blanche.
Rendement = 92% pour les trois étapes. Point de fusion : 120°C. MH⁺ : 345 (tr: 4,65 min, condition 1)

### 1.11: N-{[(2R)-1-(2-fluoroethy)pyrrolidin-2-yl]methyl}-2-(4-iodophenyl)acetamide

Dans un tube scellé, 0,53 g de NaHCO₃ (6,4 mmol) et 0,49 g de 1-iodo,-2-fluoroéthane (2,8 mmol) sont ajoutés à 0,88 g (2,6 mmol) du composé obtenu à l'issue de l'étape 1.10 en solution dans le DMF (6 ml). Le tube est scellé et chauffé à 90°C pendant 5 heures. Le mélange réactionnel est refroidi puis le DMF est évaporé sous pression réduite, et le résidu obtenu est repris dans l'eau (10 ml) et extrait à l'acétate d'éthyle (20 ml x 2). La phase organique est séchée sur sulfate de sodium, filtrée et évaporée sous pression réduite. Le résidu obtenu est purifié par flash chromatographie sur gel de silice (éluant : dichlorométhane/méthanol, 1 % NH₄OH), avec un gradient de 0% à 10% de méthanol. 0,82 g de composé sont obtenus, sous forme de poudre beige. Rendement= 85%. Point de fusion : 96°C. MH⁺: 391 (tr: 5.1 min, condition 1)

### 1.12: Chlrohydrate de 2-amino-1-ethyl-7-{4-[2-({[(2R)-1-(2-fluoroethyl)pyrrolidin-2-yl]methyl)amino)-2-oxoethyl]-phenyl}-N-methyl-4-oxo-1,4-dihydro-1,8-naphthyridine-3-carboxamide (composé n°27)

Dans un tube à sceller, 2,1 ml d'une solution aqueuse de NaHCO₃ saturée sont ajoutés à 0,19 g (0,51 mmol) du composé obtenu à l'issue de l'étape 1.11 et à 0,15 g (0,53 mmol) du composé obtenu à l'issue de l'étape 1.7 en solution dans un mélange de 1,2-diméthoxyéthane/éthanol 2/1 (6 ml). Après 15 minutes de dégazage à l'argon, 0,03 g (0,03 mmol) de Palladium tétrakistriphénylphosphine sont ajoutés. Le milieu réactionnel est chauffé à 100°C pendant 3 heures. Le milieu réactionnel est refroidi à température ambiante, puis de l'eau (10 ml) est ajouté, le précipité formé est alors filtré, rincé à l'eau (5 ml X2), puis au dichlorométhane (5 ml X2) et séché à l'étuve. Il est ensuite purifié par flash chromatographie sur gel de silice (éluant : dichlorométhane/méthanol, 1% NH₄OH), avec un gradient de 0% à 10% de méthanol. 0,05 g de produit sont obtenus sous forme de poudre blanche. Les 0,05 g (0,1 mmol) de ce composé ainsi obtenus, sont mis en suspension dans 3 ml de méthanol, et 0,1 ml (0,1 mmol) d'une solution d'HCl (1N) dans l'éther sont ajoutés. Le milieu réactionnel est agité à température ambiante 30 minutes puis 5 ml d'éther sont ajoutés, le précipité formé est collecté par filtration et séché à l'étuve. 0,031g de produit sont obtenu sous forme de poudre blanche. Rendement de 19%.
Point de fusion : 180°C. MH⁺: 509,3 (tr: 4.6 min, condition 1)
RMN ¹H (400MHZ, DMSO-d₆), δ (ppm) : 11,71 (large s, 1H); 11,16 (d, 1H); 10,36 (large s, 1 H); 8,61 (m, 1 H); 8,53 (d, 1 H, 8,1 Hz); 8,16 (d, 2H, 8,3 Hz, ); 7,96 (s+d,2H, 8,1 Hz); 7,47 (d, 2H, 8,3 Hz); 4,83 (large m, 2H); 4,61 (m, 2H); 3,84-3,37 (très large massif, 8H); 3,15 (m, 1 H); 2,82 (d, 3H, 4,6 Hz); 2,15-1,68 (très large m, 4H); 1,33 (t, 3H, 6,9Hz).

### Exemple 2: Chlorhydrate de 2-amino-1-ethyl-N-methyl-7-{4-[2-({[4-(1-methylethyl)morpholin-3-yl]methyl}amino)-2-oxoethyl]phenyl}-4-oxo-1,4-dihydro-1,8-naphthyridine-3-carboxamide (composé n°32)

### 2.1: 4-(1-methylethyl)morpholine-3-carboxamide

A une solution de 1,1 g (6,3 mmol) de morpholine-3-carboxamide (dont une synthèse est décrite dans WO2005026156, Hennequin L.F.A et coll) dans l'acétonitrile (16 ml) 1,86 g (22,2 mmol) d'hydrogénocarbonate en poudre et 0,7 ml (7,0 mmol) d'iodure d'isopropyle sont ajoutés successivement puis le mélange est chauffé à 150°C sous micro-ondes pendant 30 minutes. Le mélange réactionnel est refroidi à température ambiante, dilué avec du dichlorométhane (30 ml) et filtré sur célite. Le filtrat est concentré sous pression réduite et 1,1 g de composé sont obtenus sous forme de poudre beige, utilisés sans purification dans l'étape suivante. Rendement : 97%. MH⁺: 173,2 (tr: 0,72 min, condition 1)

### 2.2 : Chlorhydrate de 1-[4-(1-methylethyl)morpholin-3-yl]methanamine

Sous atmosphère inerte à une solution de 1,1 g (6,15 mmol) du composé obtenu à l'issue de l'étape 2.1, dans le THF anhydre, 15,4 ml d'une solution (1M) d'hydrure de lithium et d'aluminium dans le THF sont ajoutés goutte à goutte, puis le mélange réactionnel est chauffé à 70°C pendant 2 heures, puis refroidi à température ambiante. 0,6 ml d'eau, 0,6 ml de soude (1N) et 1,8 ml d'eau sont additionnés lentement et successivement, puis le mélange réactionnel est agité 30 minutes à température ambiante, le précipité formé est éliminé par filtration, rincé au THF (20 ml), le filtrat est concentré sous pression réduite, puis 6,2 ml d'une solution d'HCl (1M) dans le diéthyle éther sont ajoutés, le mélange est agité à température ambiante pendant 30 minutes puis concentré sous pression réduite. 1,0 g de composé chlorhydraté sont obtenus sous forme d'une poudre marron et utilisés sans purification dans l'étape suivante. Rendement : 86%. MH⁺: 159.4 (tr: 0.53 min, condition 1)

### 2.3 : ethyl [4-(4,4,5,5-tetramethyl-1,3,2-dioxaborolan-2-yl)phenyl]acetate

Un mélange de 9,5 g (32,8. mmol) de (4-iodo-phényl)-acétate d'éthyle et de 9,16 g (36,1 mmol) de bispinacolatodiborane en solution dans le diméthylsulfoxide anhydre (65 ml) est dégazé à l'argon 15 minutes, puis 27,8 g (98,4 mmol) d'acétate de potassium et 1,34 g (1,64 mmol) de palladium dichloro(phosphinoferrocène) sont ajoutés et le mélange réactionnel est chauffé à 55°C pendant 1h30 sous argon. Le mélange réactionnel est dilué avec 220 ml d'acétate d'éthyle, puis la phase organique est lavée trois fois avec de l'eau (200 ml), puis séchée sur Na₂SO₄, et concentrée sous pression réduite. 10,8 g de composé sont obtenus sous forme d'une huile marron impure, mais utilisée telle que dans l'étape suivante. MH⁺: 291.2 (tr: 9.4 min, condition 1)

### 2.4: ethyl {4-[7-amino-8-ethyl-6-(methylcarbamoyl)-5-oxo-5,8-dihydro-1,8-naphthyridin-2-yl]phenyl}acetate

A 6,16 g (21,9 mmol) du composé obtenu à l'issue de l'étape 1.5 ou 1.6 (selon que la methode A ou B a été employée) et à 7,64 g (26,3 mmol) du composé obtenu à l'issue de l'étape 2.3 en solution dans un mélange de 1,2-diméthoxyéthane/ éthanol 2/1 (275 ml), 88 ml d'une solution aqueuse de NaHCO₃ saturée sont ajoutés. Après 15 minutes de dégazage à l'argon, 1,27 g (1,1 mmol) de Palladium tétrakistriphénylphosphine sont ajoutés. Le milieu réactionnel est chauffé à 100°C pendant 4 heures. Le milieu réactionnel est refroidi à température ambiante, puis de l'eau (300 ml) est ajoutée, le précipité formé est alors filtré, rincé à l'eau (20 ml X2), lavé avec de l'acétate d'éthyle (50 ml) puis séché sous vide sur P₂O₅. 5,3 g de produit sont obtenus sous forme de poudre blanche. Rendement : 59%. Point de fusion 195°C. MH⁺: 409,1 (tr: 5,89 min, condition 1)

### 2.5: {4-[7-amino-8-ethyl-6-(methylcarbamoyl)-5-oxo-5,8-dihydro-1,8-naphthyridin-2-yl]phenyl}acetic acid

A une suspension de 0,87 g (2,1 mmol) du composé obtenu à l'issue de l'étape 2.4 dans un mélange 1/1/1 de THF/ méthanol/eau (12 ml), 0,134 g (3,2 mmol) d'hydroxyde de lithium sont ajoutés en une portion. Le mélange réactionnel est chauffé à 70°C pendant 3 heures, puis refroidi à température ambiante. De l'eau est ajoutée (10 ml) et le milieu est acidifié avec une solution d'HCl (1 N) jusqu'à pH=2, le précipité formé est isolé par filtration et séché sous vide sur P₂O₅. 0,78 g de produit sont obtenus sous forme de poudre blanche. Rendement 96%. Point de fusion 260°C. MH⁺ : 381,1 (tr: 6,74 min, condition 1)

### 2.6: Chlorhydrate de 2-amino-1-ethyl-N-methyl-7-{4-[2-({[4-(1-methylethyl)morpholin-3-yl]methyl}amino)-2-oxoethyl]phenyl}-4-oxo-1,4-dihydro-1,8-naphthyridine-3-carboxamide (composé n°32)

Sous atmosphère inerte, 0,18 g de N,N-carbodiimidazole sont ajoutés à une suspension de 0,4 g (1,1 mmol) du composé obtenu à l'issue de l'étape 2.5 dans le DMF (5 ml), le mélange réactionnel est agité à température ambiante 1 heure et demi puis 0,225 g (1,2 mmol) du composé obtenu à l'issue de l'étape 2.2 en solution dans le DMF (1 ml), et préalablement agité en présence de 0,13 g (1,3 mmol) de carbonate de sodium, sont ajoutés, puis le mélange est chauffé à 80°C pendant 2 heures. Le DMF est éliminé par évaporation sous pression réduite, puis le résidu est purifié par flash chromatagraphie sur gel de silice (éluant : dichlorométhane/ méthanol /1% d'NH₄OH), avec un gradient de 0% à 5% de méthanol. 0,3 g de produit sont obtenus, sous forme de poudre blanche. Rendement : 57%. MH⁺: 521.3
A une suspension de 0,28 g (0,54 mmol) de ce composé dans le méthanol (2 ml), 0,05 ml d'une solution concentrée d'HCl (35%) sont ajoutés. Le mélange est agité pendant une heure puis de l'éther diéthylique (10 ml) est ajouté, le solide formé est isolé par filtration puis séché sous vide. 0,26 g de produit chlorhydraté, sont obtenus sous forme d'une poudre orangée. Rendement 87%. Point de fusion 192°C. MH⁺: 521,3 (tr: 9,1 min, condition 2)
RMN ¹H (400MHZ, DMSO-d₆), δ (ppm): 11,75(s, 1H); 11,13 (q, 1H, 4,6 Hz);10,86,(s, 1H); 8,52 (d, 2H, 8,1 Hz); 8,16 (d, 2H, 8,3 Hz); 8,01 (s,1H); 7,95 (d,1H, 8,1 Hz); 7,46 (d, 2H, 8,3 Hz); 4,61 (q, 2H, 6,8Hz); 3,96 (m, 3H); 3,83-3,02 (large m, 9H); 2,8 (s, 3H); 1,24 (m, 9H).

### Exemple 3: Chlorhydrate de 2-amino-1-ethyl-N-methyl-4-oxo-7-{5-[2-oxo-2-(pyridin-2-ylamino)ethyl]pyridin-2-yl}-1,4-dihydro-1,8-naphthyridine-3-carboxamide (composé n°19)

### 3.1 : 2-(6-Chloro-pyridin-3-yl)-N-pyridin-2-yl-acetamide

Sous atmosphère inerte, 6,23 g (38,5 mmol) de N,N-carbodiimidazole sont ajoutés à une suspension de 6 g (35 mmol) d'acide 2-chloropyridyle acétique dans le THF anhydre (90 ml) à température ambiante. Le mélange réactionnel est agité à cette température pendant 2 heures puis 5,43 g (57,7 mmol) de 2-aminopyridine sont ajoutés et le mélange est chauffé pendant 2 heures à reflux. 200 ml de dichlorométhane sont ajoutés au mélange réactionnel, refroidi à température ambiante, la phase organique ainsi obtenue est lavée avec une solution saturée de chlorure d'ammonium, puis avec une solution aqueuse de soude (1N), séchée sur Na₂SO₄, filtrée et concentrée sous pression réduite. Le résidu est purifié par flash chromatographie sur gel de silice (Eluant dichlorométhane / acétate d'éthyle de 0% a 70% d'acétate d'éthyle). 5,6 g de composé sont obtenus sous forme d'une poudre blanche. Rendement 65%. Point de fusion : 130°C. MH⁺: 248,1 (tr: 5,45 min, condition 1).

### 3.2: Chlorhydrate de 2-amino-1-ethyl-N-methyl-4-oxo-7-{5-[2-oxo-2-(pyridin-2-ylamino)ethyl]pyridin-2-yl}-1,4-dihydro-1,8-naphthyridine-3-carboxamide (composé n°19

Même mode opératoire que celui décrit dans l'exemple 1, étape 1.12, à partir de 0,32 g (1,3 mmol) du composé obtenu à l'issue de l'étape 3.1, de 0,41 g (1,4 mmol) du composé obtenu à l'issue de l'étape 1.7, de 74 mg (0,06 mmol) de palladium tétrakistriphénylphosphine, de 14,5 ml d'une solution saturée d'hydrogénocarbonate dans un mélange de 1,2-diméthoxyéthane/éthanol (2/1) (19 ml), et de 0,26 ml d'une solution d'HCl (1M) dans l'éther, 0,095 g de produit chlorhydraté, sont obtenus sous forme d'une poudre jaune. MH⁺: 458,3 (tr: 6,3 min, condition 1). Rendement : 16%. Point de fusion : 268-288°C(décomposition)
RMN ¹H (400 MHZ, DMSO-d₆), δ (ppm) : 11,77(s, 1H); 11,3(s, 1H); 11,12(s, 1H); 8,74(d, 1H, 2Hz); 8,62(d, 1H, 8,1Hz); 8,49(d, 1H, 8,2Hz); 8,36(m, 1H); 8,35(d, 1H, 8,1 Hz); 8,05(s+ dd, 2H, 2- 8,2Hz); 8(d, 1H, 8,4Hz); 7,9(dd, 1H, 7,2- 8,4Hz); 7,2(dd, 1 H, 5,2- 7,2Hz); 6,33(2HCl); 4,64(m, 2H); 3,97(s, 2H); 2,82(s, 3H); 1,33(t, 3H, 6,8Hz).

### Exemple 4 : 2-amino-1-ethyl-N-methyl-4-oxo-7-{6-[2-oxo-2-(pyridin-2-ylamino)ethyl]pyridin-3-yl}-1,4-dihydro-1,8-naphthyridine-3-carboxamide (composé n°21)

### 4.1 : 2-(5-Bromo-pyridin-2-yl)-N-pyridin-2-yl-acetamide

Sous atmposhère inerte, à 0,26 g (0,95 mmol) d'acide (5-bromo-pyridin-2-yl)-acétique (dont une synthèse est décrite dans Tetrahedron, 1997, 53(24) 8257-8268 Gurnos J. et coll) en solution dans le THF anhydre (4 ml), sont ajoutés successivement 0,09 g (0,95 mmol) de 2-aminopyridine, 0,31 g (2,4 mmol) de diisopropyléthylamine et 0,35 g (1,1 mmol) de TBTU. Le mélange réactionnel est agité 3 heures à température ambiante. Le mélange réactionnel est concentré sous pression réduite et le résidu est repris dans l'acétate d'éthyle (20 ml), puis la phase organique ainsi obtenue est lavée avec une solution saturée de chlorure d'ammonium (15 ml) et avec une solution saturée d'hydrogénocarbonate (15 ml), puis est séchée sur Na₂SO₄, filtrée et concentrée sous pression réduite. Le résidu est purifié par flash chromatographie sur gel de silice (éluant : dichlorométhane/acétate d'éthyle, 1% NH₄OH), avec un gradient de 0% à 20% d'acétate d'éthyle, 1% NH₄OH. 0,125 g de composé sont obtenus sous forme d'une poudre blanche. Rendement : 45%. Point de fusion : 131°C. MH⁺: 292-294 (tr: 6,1 min, condition 1)

### 4.2 : 2-amino-1-ethyl-N-methyl-4-oxo-7-{6-[2-oxo-2-(pyridin-2-ylamino)ethyl]pyridin-3-yl}-1,4-dihydro-1,8-naphthyridine-3-carboxamide (composé n°21

Même mode opératoire que celui décrit dans l'exemple 1, étape 1.12, à partir de 0,42 g (1,4 mmol) du composé obtenu à l'issue de l'étape 4.1, de 0,46 g (1,6 mmol) du composé obtenu à l'issue de l'étape 1.7, de 83 mg (0,07 mmol) de palladium tétrakistriphénylphosphine, de 15 ml d'une solution saturée d'hydrogénocarbonate dans un mélange de 1,2-diméthoxyéthane/éthanol (2/1) (24 ml), 0,03 g de produit sont obtenus sous forme de poudre blanche. Rendement : 5%.
Point de fusion : 266°C. MH+ 458,1 (tr: 6,5 min, condition 1)
RMN ¹H (400MHZ, DMSO-d₆), δ (ppm) : 11,76 (s, 1H); 11,10 (q, 1H, 4,6 Hz); 10,78 (s, 1 H); 9,32 (d, 1 H, 2,3 Hz); 8,57 (d, 1 H, 8,1 Hz); 8,53 (dd, 1 H, 2,3-8,2 Hz); 8,34 (d, 1 H, 5Hz); 8,07 (d, 1 H, 8,2Hz); 8,04 (d+s, 2H, 8,2 Hz); 7,78 (dd, 1H, 7,4-8,2 Hz); 7,59 (d, 1 H, 8,1 Hz); 7,12 (dd, 1 H, 5, 7,4Hz); 4,61 (m, 2H); 4,05 (s, 2H); 2,82 (d, 3H, 4,6 Hz); 1,3 (t, 3H, 6,8Hz).

### Exemple 5: Chlorhydrate de 2-amino-7-[4-(2-{[(4-cyclopropylmorpholin-3-yl)methyl]amino}-2-oxoethyl)phenyl]-1-ethyl-N-methyl-4-oxo-1,4-dihydro-1,8-naphthyridine-3-carboxamide (composé n°33)

### 5.1 : 4-cyclopropylmorpholine-3-carboxamide

A 1,2 g (7,2 mmol) de chlorhydrate de morpholine-3-carboxamide (dont une synthèse est décrite dans WO2005026156, Hennequin L.F.A. et coll) en solution dans le méthanol (36 ml) 2,9 g de tamis moléculaire 3A , 4,3 g (72 mmol) d'acide acétique, 7, g (43,2 mmol) de 2(1-éthoxy-cyclopropyl)oxy]triméthylsilane et 4,4 g (31,7 mmol) de cyanoborohydrure de sodium sont ajoutés successivement. Le mélange réactionnel est chauffé à 70°C pendant 3 heures et demie, puis, refroidi à température ambiante, il est filtré. Le filtrat est concentré sous pression réduite, puis le résidu est repris dans le dichlorométhane (200 ml) et lavé 3 fois avec une solution aqueuse de NaOH (1N) (100 ml). La phase organique est séchée sur Na₂SO₄, filtrée puis concentrée sous pression réduite. 0,58 g de produit sont obtenus, sous forme d'une poudre blanche. Rendement 47%. Point de fusion 116°C. MH⁺: 171,2 (tr: 1,03 min, condition 1).

### 5.2 : 1-(4-cyclopropylmorpholin-3-yl)methanamine chlorhydratée

Sous atmosphère inerte, à une solution de 0,58 g (3,4 mmol) du composé, obtenu à l'issue de l'étape 5.1, dans le THF anhydre, refroidie à 0°C, 13,6 ml (13,6 mmol) d'une solution (1 N) de triborohydure complexé au tétrahydrofurane dans le THF sont ajoutés goutte à goutte. Le mélange réactionnel est chauffé à 70°C pendant 3 h. 15 ml d'une solution (1 N) d'HCl sont ajoutés sur le mélange réactionnel refroidi à température ambiante, après 30 min d'agitation la phase aqueuse est décantée et extraite 2 fois à l'éther (15 ml) puis alcalinisée par addition d'une solution (1 N) de soude. La phase aqueuse est ensuite extraite à l'acétate d'éthyle 4 fois (20 ml) et au dichlorométhane 4 fois (20 ml). Les phases organiques réunies sont séchées sur Na₂SO₄, filtrées et concentrées sous pression réduite. Le résidu est repris dans du méthanol (4 ml) et 3,4 ml d'une solution d'HCl dans l'éther sont ajoutés, la solution est agitée 30 minutes puis 5 ml d'éther sont ajoutés, le solide formé est collecté par filtration et séché sous vide sur P₂O₅. 0,51 g de produit sont obtenus, sous forme d'une poudre blanche. Rendement 78%. MH⁺: 157,2 (tr: 0,4 min, condition 1).

### 5.3: Chlorhydrate de 2-amino-7-[4-(2-{[(4-cyclopropylmorpholin-3-yl)methyl]amino}-2-oxoethyl)phenyl]-1-ethyl-N-methyl-4-oxo-1,4-dihydro-1,8-naphthyridine-3-carboxamide (composé n°33)

Même mode opératoire que celui décrit dans l'exemple 2, étape 2.6, à partir de 0,33 g (0,88 mmol) du composé obtenu à l'issue de l'étape 2.5, en suspension dans du DMF anhydre (5 ml), 0,15 g (0,92 mmol) de CDI sont ajoutés et le mélange est agité à température ambiante pendant 2 h 40. En parallèle sous atmosphère inerte, à 0,22 g (0,97 mmol) du composé obtenu à l'issue de l'étape 5.2, en solution dans le DMF (2 ml), 0,11 g (1,1 mmol) de carbonate de sodium sont ajoutés et le mélange est agité à TA pendant 2 h, puis le surnageant est prélevé et est additionné goutte à goutte sur l'intermédiaire imidazolide formé précédemment. Le mélange est ensuite chauffé à 80°C pendant 2 h. Le DMF est évaporé sous pression réduite, et le résidu est purifié par flash chromatographie sur gel de silice (éluant: dichlorométhane/méthanol) avec un gradient de 0% à 5% de méthanol. 0,13 g de produit sont obtenus sous forme de poudre blanche. Rendement : 28%.

A 0,13 g (0,25 mmol) de ce composé, en solution dans 2 ml de méthanol, 0,02 g (0,3 mmol) d'une solution HCl concentrée à 35,6% sont ajoutés. Le mélange est agité à TA pendant 1 h, le solide formé est collecté par filtration, séché sur P₂O₅ sous vide. 0,13 g de composé sont obtenus sous forme d'une poudre blanche. Rendement 92%. Point de fusion : 250°C. MH⁺: 519,2 (tr: 5,5 min, condition 1). RMN (400MHz, DMSO-d₆) 11,74(s, 1H); 11,13(q, 1H, 4,6Hz); 10,33(s, 1H); 8,53(d, 1 H, 8,1Hz); 8,38(s, 1 H); 8,16(d, 2H, 8,1Hz); 8,0(s, 1 H); 7,97(d, 1 H, 8,1Hz); 7,45(d, 2H, 8,1Hz); 4,61(m, 2H); 4,03-3,79(m, 3H); 3,69-3,24(m, 8H); 2,95(m, 1H); 2,81(s, 3H); 1,32(t, 3H, 6,9Hz); 1,16-0,92(m, 4H).

### Exemple 6: Chlorhydrate de 2-amino-1-ethyl-7-{4-[2-({[(2S,4R)-1-ethyl-4-fluoropyrrolidin-2-yl]methyl}amino)-2-oxoethyl]phenyl}-N-methyl-4-oxo-1,4-dihydro-1,8-naphthyridine-3-carboxamide (composé n°26)

### 6.1 : (4R)-1-ethyl-4-fluoro-L-prolinamide

Même mode opératoire que celui décrit dans l'exemple 2, étape 2.1, à partir de 0,88 g (5,2 mmol) de 4(R)-fluoro-2(S)-pyrrolinecarboxamide (dont une synthèse est décrite dans Bioorg Med Chem 12(23), 2004, 6053-6061, Fukushima H. et coll), de 0,90 g (5,7 mmol) d'iodure d'éthyle et de 1,53 g (18,3 mmol) de NaHCO₃ dans le DMF anhydre (17 ml), 0,84 g de produit sont obtenus sous forme de poudre blanche. Rendement : 100%. Point de fusion : 127°C. MH⁺: 161,2 (tr: 0,36 min, condition 1).

### 6,2 : 1-[(2S,4R)-1-ethyl-4-fluoropyrrolidin-2-yl]methanamine chlorhydratée

Même mode opératoire que celui décrit dans l'exemple 5, étape 5.2, à partir de 0,69 g (4,3 mmol) de composé obtenu à l'issue de l'étape 6.1, de 17,3 ml (17,3 mmol) d'une solution (1M dans le THF) de trihydrure de bore complexé au tétrahydrofuranne, dans le THF anhydre (30 ml), puis de 4,3 ml d'une solution d'HCl (1 M) dans l'éther, 0,33 g de produit sont obtenus sous forme d'huile jaune et utilisés sans autre purification dans l'étape suivante. Rendement : 52%. MH⁺: 147,3 (tr: 0,36 min, condition 1).

### 6.3: Chlorhydrate de 2-amino-1-ethyl-7-{4-[2-({[(2S,4R)-1-ethyl-4-fluoropyrrolidin-2-yl]methyl}amino)-2-oxoethyl]phenyl}-N-methyl-4-oxo-1,4-dihydro-1,8-naphthyridine-3-carboxamide (composé n°26)

Sous atmosphère inerte, à une suspension de 0,85 g (2,2 mmol) du composé obtenu à l'issue de l'étape 2.5, dans le dichlorométhane (9 ml), 0,45 g (4,4 mmol) de triéthylamine sont ajoutés. Le mélange est refroidi à 0°C (bain de glace) et 0,61 g (4,4 mmol) de fluorure de cyanuryle en solution dans (1 ml) de dichlorométhane sont ajoutés goutte à goutte. Le mélange est agité à TA pendant 3h30, puis dilué avec 30 ml de dichlorométhane, lavé avec 20 ml d'une solution de NaHCO₃ saturée, préalablement refroidie, séché sur Na₂SO₄, filtré et concentré. Le fluorure d'acide obtenu est utilisé sans purification dans l'étape suivante.

A 0,24 g (1,6 mmol) de composé issu de l'étape 6.2 en solution dans du DMF anhydre (4 ml) 0,41 g (4,1 mmol) de triéthylamine sont ajoutés suivi de 0,63 g (1,7 mmol) du fluorure d'acide issu de l'étape précedente. Le mélange est agité à TA pendant une nuit, puis le DMF est évaporé sous vide, le résidu est repris dans du dichlorométhane (20 ml) et la phase organique est lavée avec une solution saturée de NaHCO₃ séchée sur Na₂SO₄, filtrée et concentrée sous pression réduite. Le résidu est purifié par flash chromatographie sur gel de silice (éluant : dichlorométhane/méthanol), avec un gradient de 0% à 3% de méthanol. 0,075 g de composé sont obtenus sous forme d'une poudre blanche. Rendement : 9%. Alpha D= -12 (concentration 25 mg/ml dans le MeOH). MH⁺: 509 (tr: 5,2 min, condition 1).

A 0,07 g (0,13 mmol) du composé issu de l'étape précédente en solution dans le méthanol (2,5 ml), 0,13 ml (0,13 mmol) d'une solution HCl (1M) dans l'éther sont ajoutés. Le mélange est agité à TA pendant 1 heure, concentré sous pression réduite. Le solide est trituré dans le dichlorométhane (2 ml), filtré et séché sous vide sur P₂O₅. 0,05 g de composé sont obtenus sous forme d'une poudre jaune claire. Rendement : 59%. Point de fusion 170°C. MH⁺: 509,3 (tr: 5,2 min, condition 1).

RMN 1H (400MHZ, DMSO-d₆), δ (ppm) : 11,73(s1H); 11,12(m, 1H); 10,56(s, 1 H); 8,59(t, 1 H, 5,6Hz); 8,54(d, 1 H, 8,1 Hz); 8,16(d, 2H, 8,3Hz); 7,99(s,1 H); 7,96(d, 1H, 8,1 Hz); 7,48(d, 2H, 8,3Hz); 5,41 (dt, 1 H, 4,9- 53,2Hz); 4,61(m, 2H); 3,9(m, 1 H); 3,82-3,44(m, 6H); 3,38(m, 1 H); 3,12(m,1 H); 2,82(m, 3H); 2,36(m, 1 H); 2,02(m, 1 H); 1,33(t, 3H, 6,8Hz); 1,24(t, 3H, 7,9Hz)

### Exemple 7: Chlorhydrate de 2-amino-1-ethyl-7-{4-[2-({[(2S)-1-ethyl-4,4-difluoropyrrolidin-2-yl]methyl}amino)-2-oxoethyl]phenyl}-N-methyl-4-oxo-1,4-dihydro-1,8-naphthyridine-3-carboxamide (composé n°24)

### 7.1 : 1-ethyl-4,4-difluoro-L-prolinamide

Même mode opératoire que celui décrit dans l'exemple 2, étape 2.1, à partir de 0,2 g (1,1 mmol) de 4,4-difluoro-2(S)-pyrrolinecarboxamide (dont une synthèse est décrite dans Bioorg Med Chem 12(23) 2004 6053-6061 Fukushima H. et coll), de 0,18 g (1,2 mmol) d'iodure d'éthyle et de 0,32 g (3,7 mmol) de NaHCO₃ dans le DMF anhydre (3,5 ml), 0,18 g de produit sont obtenus sous forme de poudre blanche. Rendement : 95%. Point de fusion : 138°C. MH⁺: 179.2 (tr: 1.25 min, condition 1).

### 7,2 : chlorhydrate de 1-[(2S)-1-ethyl-4,4-difluoropyrrolidin-2-yl]methanamine:

Même mode opératoire que celui décrit dans l'exemple 2, étape 5.2, à partir de 0,18 g (1 mmol) de composé issue de l'étape 7.1, de 8,2 ml (8,2 mmol) d'une solution (1M dans le THF) de trihydrure de bore complexé au tétrahydrofurane, dans le THF anhydre (12 ml), et de 2 ml d'une solution (1M) d'HCl dans l'éther, 0,2 g de produit sont obtenus sous forme d'une poudre grise et utilisés sans autre purification. Rendement: 100%. MH⁺ : non détectable

### 7.3: Chlorhydrate de 2-amino-1-ethyl-7-{4-[2-({[(2S)-1-ethyl-4,4-difluoropyrrolidin-2-yl]methyl}amino)-2-oxoethyl]phenyl}-N-methyl-4-oxo-1,4-dihydro-1,8-naphthyridine-3-carboxamide (composé n°24)

Sous atmposhère inerte, à 0,32 g (0,85 mmol) de composé obtenu à l'issue de l'étape 2.5 en suspension dans le DMF anhydre (4 ml), sont successivement ajoutés 0,37 ml (3,8 mmol) de diisopropyléthylamine puis une solution dans le DMF (2 ml) de 0,20 g (0,85 mmol) du composé issue de l'étape 7.2 en présence de 0,3 ml (1,7 mmol) de diisopropyléthylamine, à ce mélange réactionnel, refroidi par un bain de glace, sont ajoutés 0,31 g (0,9 mmol) de TBTU. Le mélange réactionnel est agité 6 heures à 70°C, puis concentré sous pression réduite. Le résidu est purifié par flash chromatographie sur gel de silice (éluant : dichlorométhane/acétate d'éthyle, 1% NH₄OH), avec un gradient de 0% à 100% d'acétate d'éthyle, 1% NH₄OH. 0,087 g de composé sont obtenus sous forme d'une poudre rosée

A 0,087 g (0,17 mmol) du composé obtenu à l'issue de l'étape précédente, en solution dans 2 ml de méthanol, 0,17 ml (0,17 mmol) d'une solution d'HCl (1M dans l'éther) sont ajoutés. Le mélange est agité à TA pendant 1 h, puis 5 ml d'éther sont ajoutés et le solide formé est collecté par filtration, séché sur P₂O₅ sous vide. 0,065 g de produit sont obtenus sous forme de poudre rosée. Rendement : 70%. Point de fusion : 266°C. MH⁺ : 527,3 (tr: 6,04 min, condition 1).
RMN ¹H (400MHZ, DMSO-d₆), δ (ppm): 11,74(s, 1H); 11,12(q, 1H, 4,6Hz); 11,08(s, 1 H); 8,58(s, 1 H); 8,53(d, 1H, 8,1 Hz); 8,16(d, 2H, 8,3Hz); 7,98(s, 1 H); 7,96(d, 1 H, 8,1Hz); 7,47(d, 2H, 8,3Hz); 4,61(m, 2H); 4,18(q, 1H, 10,7Hz); 3,91(m, 1H); 3,82(q, 1 H, 12,9Hz); 3,59(s, 2H); 3,56(m, 3H); 3,17(m, 1 H); 2,81 (s, 3H); 2,75(m, 1 H); 2,43(m, 1H); 1,32(t, 3H, 6,8Hz); 1,21(t, 3H, 7,1 Hz).

### Exemple 8 : Chlorhydrate de 2-amino-1-ethyl-7-[4-(2-{[(4-ethylmorpholin-3-yl)methyl]amino}-2-oxoethyl)phenyl]-N-methyl-4-oxo-1,4-dihydro-1,8-naphthyridine-3-carboxamide (composé n°22)

### 8.1 : 4-Ethyl-morpholine-3-carboxamide:

Même mode opératoire que celui décrit dans l'exemple 2, étape 2.1, à partir de 0,2 g (1,1 mmol) de morpholine-3 -carboxamide (dont la synthèse est décrite dans WO2005026156, Hennequin L.F.A.. et coll), de 0,18 g (1,2 mmol) d'iodure d'éthyle et de 0,32 g (3,7 mmol) de NaHCO₃ dans le DMF anhydre (3,5 ml), 0,18 g de produit sont obtenus sous forme de poudre blanche. Rendement : 95%. Point de fusion : 127°C. MH⁺: 159,3 (tr:0,35 min, condition 1).

### 8.2 : 1-(4-ethylmorpholin-3-yl)methanamine

Sous atmosphère inerte, à 0,06 g (0,38 mmol) de composé issue de 8.1 en solution dans le THF anhydre, 0,76 ml (0,76 mmol) d'une solution (1M) dans le THF d'hydrure de lithium et d'aluminium sont ajoutés puis le mélange réactionnel est chauffé à 70°C pendant 5 h. Au mélange refroidi à TA, 28 µl d'eau, 28 µl de soude (1N), 84 µl d'eau sont ajoutés successivement pour former un « cake ». Le mélange est agité vigoureusement pendant 30 minutes puis le précipité est éliminé par filtration. Le filtrat est concentré sous pression réduite pour donner 0,06 g de produit sous forme d'une huile incolore, qui est utilisée sans purification dans l'étape suivante.
Rendement: 100%.
RMN ¹H (300MHZ, DMSO-d₆), δ (ppm) :3,82(m, 2H); 3,61 (m, 2H); 2,92(m, 2H); 2,77(m, 2H); 2,4(m, 3H); 1,53(s, 2H); 1.1(t, 3H, 7.5Hz).

### 8.3: Chlorhydrate de 2-amino-1-ethyl-7-[4-(2-{[(4-ethylmorpholin-3-yl)methyl]amino}-2-oxoethyl)phenyl]-N-methyl-4-oxo-1,4-dihydro-1,8-naphthyridine-3-carboxamide

Même mode opératoire que celui décrit dans l'exemple 7, étape 7.3, à partir de 0,14 g (0,37 mmol) de composé issu de l'étape 2.5, de 0,05 g (0,37 mmol) du composé issu de l'étape 8.2, de 0,13 g (0,4 mmol) de TBTU et de 0,12 g (0,92 mmol) de diisopropyléthylamine dans le DMF anhydre (4 ml), et de 0,17 ml d'une solution d'HCl (1M) dans l'éther. 0,07 g de produit sont obtenus sous forme de poudre blanche. Rendement : 50%. Point de fusion : 190°C. MH⁺: 507,1 (tr: 5,4 min, condition 1).
RMN ¹H (400MHZ, DMSO-d₆), δ (ppm): 11,73(s, 1H); 11,12(q, 1H, 4,6Hz); 11,03+10,76(2s, 1H); 8,52(d, 2H, 8,1Hz); 8,15(d, 2H, 8,1Hz); 7,98(large s, 1H); 7,95(d, 1H, 8,1Hz); 7,47(d, 2H, 8,1Hz); 4,61(m, 2H); 3,94(m, 2H); (3,81(m, 1H); 3,58(m, 3H); 3,38(m, 4H); 3,18(m, 3H); 2,81(d, 3H, 4,6Hz); 1,28(m, 6H).

### Exemple 9: 1-ethyl-7-[5-(2-{[(1-ethylpyrrolidin-2-yl)methyl]amino}-2-oxoethyl)thiophen-2-yl]-N,2-dimethyl-4-oxo-1,4-dihydro-1,8-naphthyridine-3-carboxamide (composé n°52)

### 9.1 : (5-chlorothiophen-2-yl)acetic acid

A 19,7 g (158,8 mmol) de FAMSO, 1,2 g (29,8 mml) d'hydroxyde de sodium en poudre sont ajoutés, le mélange est chauffé à 70°C pendant 30 min puis 3 g (19,8 mmol) de 5-chloro-2-thiophenecarboxaldéhyde sont ajoutés et le mélange est chauffé de nouveau à 70°C pendant une nuit. A température ambiante, 850 ml d'eau sont ajoutés puis la phase aqueuse est extraite à l'acétate d'éthyle (3 fois 430 ml). Les phases organiques réunies sont lavées par une solution aqueuse saturée de NaCl, séchées sur Na₂SO₄, filtrées et concentrées sous pression réduite. 6,7 g de produit sont obtenus sous forme d'une huile marron et sont utilisés sans purification dans l'étape suivante.

### MH⁺ : 253 (tr : 7,6 min condition 1)

A 6,7 g du composé obtenu à l'issue de l'étape précédente, en solution dans l'éthanol (44 ml), 33 ml (132 mmol) d'une solution d'HCl (4M) dans le dioxanne sont ajoutés et le mélange est chauffé à 80°C pendant 2h15. Le mélange refroidi à TA, est dilué avec de l'acétate d'éthyle (400 ml) et lavé avec une solution aqueuse saturée de NaHCO₃ (350 ml), puis de NaCl (200 ml), la phase organique est ensuite séchée sur Na₂SO₄, filtrée et concentrée sous pression réduite. Le résidu est purifié par flash chromatographie sur colonne de gel de silice (éluant: heptane/ dichlorométhane), avec un gradient de 0% à 50% de dichlorométhane. 1,93 g de produit sont obtenus sous forme d'une huile marron. Rendement : 32%. MH⁺ : 205,1 (tr : 5,33 min condition 1)

A 1,93 g (6,6 mmol) du composé obtenu à l'issue de l'étape précédente, en solution dans un mélange de solvant (THF/ méthanol/ eau 1/ 1/ 1) (33 ml), 0,97 g (23,1 mmol) d'hydroxyde de lithium monohydraté sont ajoutés. Le mélange est chauffé à 70°C pendant 2 h, refroidi à TA et acidifié jusqu'à pH= 1 par addition de 23 ml d'une solution aqueuse d'HCl (1N) puis extrait au dichlorométhane (2 fois 30 ml). Les phases organiques réunies sont lavées par une solution aqueuse saturée de NaCl, séchées sur Na₂SO₄, filtrées et concentrées sous pression réduite. 1,44 g de produit sont obtenus sous forme d'une poudre marron et sont utilisés sans purification dans l'étape suivante. MH⁺ : 176 (tr : 12,3 min condition GC Cl/CH4+).

### 9.2 : 2-(5-chlorothiophen-2-yl)-N-[(1-ethylpyrrolidin-2-yl)methyl]acetamide:

Même mode opératoire que celui décrit dans l'exemple 4, étape 4.1, à partir de 1,77 g (10 mmol) de composé obtenu à l'issue de l'étape précédente, de 5,4 g (40 mmol) de 1-éthylpyrrolidinylméthylamine, de 6,4 g (20 mmol) de TBTU et de 2,58 g (20 mmol) de diisopropyléthylamine dans le DMF anhydre (100 ml). 1,62 g de produit sont obtenus sous forme de poudre blanche. Rendement : 56%. Point de fusion : MH⁺: 287,1 (tr: 4,1 min, condition 1).

### 9.3 1-ethyl-7-[5-(2-{[(1-ethylpyrrolidin-2-yl)methyl]amino}-2-oxoethyl)thiophen-2-yl]-N,2-dimethyl-4-oxo-1,4-dihydro-1,8-naphthyridine-3-carboxamide (composé n°52)

Même mode opératoire que celui décrit dans l'exemple 1, étape 1.12, à partir de 1 g (3,5 mmol) de composé obtenu à l'issue de l'étape 9.2, de 1,3 g (4,5 mmol) de composé obtenu à l'issue de l'étape 1.7, de 0,24 g (0,2 mmol) de palladium tetrakistriphénylphosphine et de 14 ml d'une solution NaHCO₃ saturée, dans un mélange de DME/ EtOH/ 2/1 (35 ml). 0,05 g de produit sont obtenus sous forme de poudre blanche. Rendement : 2 %. Point de fusion : 250°C. MH⁺: 497 (tr: 5,3 min, condition 1).
RMN ¹H (400MHZ, DMSO-d₆), δ (ppm) : 11,7(s, 1 H); 11,1(q, 1 H, 4,5Hz); 8,43(d, 1 H, 8,3Hz); 8,0 (m, 1 H); 7,95 (large s, 1 H); 7,82(d, 1 H, 8,3Hz); 7,80(d, 1 H, 3,7Hz); 7,0(d, 1 H, 3,7Hz); 4,5 (m, 2H); 3,72 (s, 2H); 3,27 (m, 1 H); 3,01 (m, 1 H); 2,89 (m, 1 H); 2,8(t, 3H, 4,5Hz); 2,76 (m, 1 H); 2,42 (m, 1 H); 2,18 (m, 1 H); 2,07 (m, 1 H); 1,76 (m, 1 H); 1,61 (m, 2H); 1,48 (m, 1 H); 1,3 (t, 3H, 6,9Hz); 1,01 (t, 3H, 7,2Hz);

### Exemple 10: Chlorhydrate de 2-amino-1-ethyl-N-methyl-4-oxo-7-{4-(2-(pyridin-2-ylamino)ethyl]phenyl}-1,4-dihydro-1,8-naphthyridine-3-carboxamide (composé 64)

### 10.1 N-[2-(4-bromophenyl)ethyl]pyridin-2-amine

Dans un tube scellé un mélange de 1,9 g (20,4 mmol) de 2-aminopyridine et 6,1 g (30,6 mmol) de 4-bromophénéthyle amine en solution dans du butanol (10 ml) est chauffé à 230°C pendant 1h sous micro-ondes. Le mélange refroidi à température ambiante est versé sur de l'eau (50 ml) et extrait à l'acétate d'éthyle (3 fois 100 ml), les phases organique regroupées sont séchées sur sulfate de sodium, filtrées et concentrées. Le résidu est purifié par flash chromatographie sur colonne de gel de silice (éluant : dichlorométhane/ Acétate d'éthyle, de 0% à 50% d'acétate d'éthyle), 1,4 g de produit sont obtenus sous forme d'une poudre blanche. Rendement : 24%. Point de fusion : 71°C. MH⁺ : 278,1 (tr : 3.71 min, condition 1).

### 10.2 : Chlorhydrate de 2-amino-1-ethyl-N-methyl-4-oxo-7-{4-[2-(pyridin-2-ylamino)ethyl]phenyl}-1,4-dihydro-1,8-naphthyridine-3-carboxamide (composé 64)

Même mode opératoire que celui décrit dans l'exemple 1, étape 1.12, à partir de 0.3 g (1,1 mmol) de composé obtenu à l'issue de l'étape 10.1, de 0,345 g (1,2 mmol) de composé obtenu à l'issue de l'étape 1.7, de 0,06 g (0,05 mmol) de palladium tetrakistriphénylphosphine et de 4,7 ml d'une solution NaHCO₃ saturée, dans un mélange de DME/ EtOH/ 2/1 (15 ml). 0,12 g de produit ainsi obtenus sont repris dans le methanol (3 ml) et 0,03 ml HCl concentré 36% sont ajoutés. 0,130 g de produit sont obtenus sous forme de poudre blanche. Rendement : 25 %. Point de fusion : 242°C. MH⁺: 497 (tr: 5,33 min, condition 1).
RMN ¹H (400MHZ, DMSO-d₆), δ (ppm) : 13,7(s, 1H); 11,73(m, 1H); 11,12(q, 1H, 4,5Hz); 8.9(s, 1 H); 8,52(d, 1 H, 8,1 Hz); 8,17(d, 2H, 8,3Hz); 8,02 (large s, 1 H); 7,96(d, 1 H, 8,1 Hz); 7,89(m, 1 H); 7,51 (d, 2H, 8,3Hz); 7,08(d, 1 H, 9,1 Hz); 6,84(large t, 1 H, 6,7Hz); 4,6(m, 2H); 3,69 (m, 2H); 3,01 (t, 2H, 7,2Hz); 2,81 (d, 3H, 4.5Hz); 1,32 (t, 3H, 6,9Hz).

Les composés 1, 2, 3, 4, 5, 6, 7, 8, 9, 10, 11, 12, 13, 14, 15, 16, 17, 18, 20, 23, 25, 28, 30, 31, 45, 46, 47, 51, 52, 56, 62, 63 et 64 ont été synthétisés suivant la voie de synthèse décrite dans l'exemple 1. Les composés 35, 36, 37, 38, 39, 40, 41, 42, 44, 48, 49, 50, 53, 54, et 55 ont été synthétisés suivant la voie de synthèse décrite dans l'exemple 2. Les composés 29, 34 et 43 ont été synthétisés suivant la voie de synthèse décrite dans l'exemple 3.

Les tableaux qui suivent illustrent les structures chimiques et les propriétés physiques de quelques exemples de composés selon l'invention.

Le tableau 1 illustre des composés de formule (la) correspondant à des composés de formule (I) pour lesquels U représente un groupe carbonyle et R6 = - (CH₂)ₙ-L

Le tableau 2 illustre des composés de formule (Ib) correspondant à des composés de formule (I) pour lesquels U représente un groupe carbonyle, Y, V et W représentent un groupe -CH-, Z représente un atome de carbone, R7 représente un atome d'hydrogène et R6 = -(CH₂)ₙ-L.

Le tableau 3 illustre des composés de formule (Ic) correspondant à des composés de formule (I) pour lesquels U représente un groupe -CH₂-, Z, Y, V et W représentent un groupe -CH-, R7 représente un atome d'hydrogène, et R6 = -(CH₂)ₙ-L.

Dans ces tableaux :
- dans la colonne « sel », « - » représente un composé sous forme de base libre, alors que « HCl » représente un composé sous forme de chlorhydrate et le rapport entre parenthèses est le rapport (acide:base),
- Me, Et, n-Pr, i-Pr, n-Bu et i-Bu représentent respectivement des groupes méthyle, éthyle, n-propyle, isopropyle, n-butyle et isobutyle, et
- Ph et Bn représentent respectivement des groupes phényle et benzyle.

**Tableau 1 :**

| | | | | | | | | | | | | | | | |
|---|---|---|---|---|---|---|---|---|---|---|---|---|---|---|---|
| 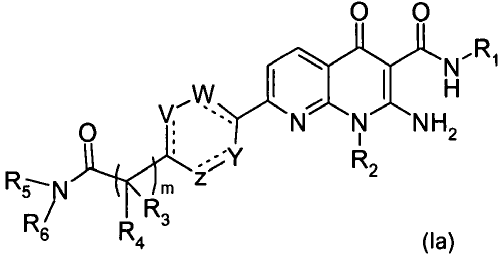 | | | | | | | | | | | | | | | |
| Avec R6 = (CH₂)ₙ-L, | | | | | | | | | | | | | | | |
| (*) condition LC/UV/MS, si elles ne sont pas mentionées, ce sont les conditions 1 qui sont utilisées. | | | | | | | | | | | | | | | |

| **N^{o}** | **m** | **n** | **R1** | **R3** | **R4** | **R5** | **R6** | **R2** | **V** | **W** | **Y** | **Z** | **Sel** | LCMS tr min (*) pureté MH+ | **Point de fusion (°C)** |
|---|---|---|---|---|---|---|---|---|---|---|---|---|---|---|---|
| **1** | 1 | 0 | CH3 | H | H | H | 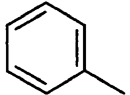 | Et | CH | CH | CH | CH | - | 7.2 (3) | 299 |
| | | | | | | | | | | | | | | 97% | |
| | | | | | | | | | | | | | | 456.36 | |
| **2** | 1 | 1 | CH3 | H | H | CH3 | 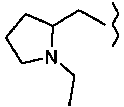 | Et | CH | CH | CH | CH | - | 9.6 | 138 |
| | | | | | | | | | | | | | | 94.5% | |
| | | | | | | | | | | | | | | 505.4 | |
| **3** | 1 | 0 | CH3 | H | H | H | 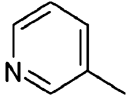 | Et | CH | CH | CH | CH | HCl (1.07) | 6.4(3) | 270 |
| | | | | | | | | | | | | | | 100% | |
| | | | | | | | | | | | | | | 457 | |

| **N°** | **m** | **n** | **R1** | **R3** | **R4** | **R5** | **R6** | **R2** | **V** | **W** | **Y** | **Z** | **Sel** | LCMS tr min(*) pureté MH+ | **Point de fusion (°C)** |
|---|---|---|---|---|---|---|---|---|---|---|---|---|---|---|---|
| **4** | 1 | 0 | CH3 | H | H | H | 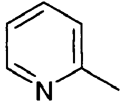 | Et | CH | CH | CH | CH | HCl (1.07) | 6.7 (3) | 258 |
| | | | | | | | | | | | | | | 95.6% | |
| | | | | | | | | | | | | | | 457 | |
| **5** | 1 | 0 | CH3 | H | H | H | 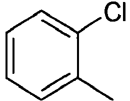 | Et | CH | CH | CH | CH | - | 7.9 (3) | >260 |
| | | | | | | | | | | | | | | 96% | |
| | | | | | | | | | | | | | | 490 | |
| **6** | 1 | 0 | CH3 | H | H | H | 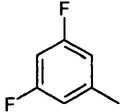 | Et | CH | CH | CH | CH | - | 8.1 | >260 |
| | | | | | | | | | | | | | | 100% | |
| | | | | | | | | | | | | | | 492 | |
| **7** | 1 | 1 | CH3 | H | H | H | 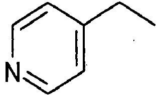 | Et | CH | CH | CH | CH | HCl (1.07) | 4.9 (3) | 230 |
| | | | | | | | | | | | | | | 97% | |
| | | | | | | | | | | | | | | 471.1 | |
| **8** | 1 | 1 | CH3 | H | H | H | 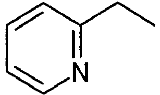 | Et | CH | CH | CH | CH | HCl (1.07) | 5.1 (3) | 268 |
| | | | | | | | | | | | | | | 96% | |
| | | | | | | | | | | | | | | 471.1 | |
| **9** | 1 | 2 | CH3 | H | H | H | 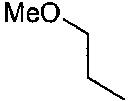 | Et | CH | CH | CH | CH | - | 6.2 | 262 |
| | | | | | | | | | | | | | | 98.4% | |
| | | | | | | | | | | | | | | 438.5 | |

| **N°** | **m** | **n** | **R1** | **R3** | **R4** | **R5** | **R6** | **R2** | **V** | **W** | **Y** | **Z** | **Sel** | LCMS tr min (*) pureté MH+ | **Point de fusion (°C)** |
|---|---|---|---|---|---|---|---|---|---|---|---|---|---|---|---|
| **10** | 1 | 0 | CH3 | H | H | H | 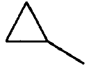 | Et | CH | CH | CH | CH | - | 6.3 | >260 |
| | | | | | | | | | | | | | | 96.5% | |
| | | | | | | | | | | | | | | 420.3 | |
| **11** | 1 | 0 | CH3 | H | H | H | 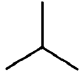 | Et | CH | CH | CH | CH | - | 6.6 | 315 |
| | | | | | | | | | | | | | | 99.2% | |
| | | | | | | | | | | | | | | 422.5 | |
| **12** | 1 | 0 | CH3 | H | H | H | 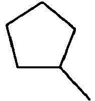 | Et | CH | CH | CH | CH | - | 14.3(2) | 300 |
| | | | | | | | | | | | | | | 98.7% | |
| | | | | | | | | | | | | | | 448.3 | |
| **13** | 1 | 0 | CH3 | H | H | H | 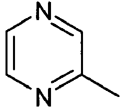 | Et | CH | CH | CH | CH | - | 6.6 | 290 |
| | | | | | | | | | | | | | | 97% | |
| | | | | | | | | | | | | | | 458.3 | |
| **14** | 1 | 1 | CH3 | H | H | H | 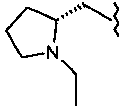 | Et | CH | CH | CH | CH | - | 4.8 | 270 |
| | | | | | | | | | | | | | | 97.4% | |
| | | | | | | | | | | | | | | 491.3 | |
| **15** | 1 | 1 | CH3 | H | H | H | 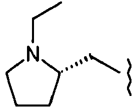 | Et | CH | CH | CH | CH | - | 4.8 | 262 |
| | | | | | | | | | | | | | | 97.9% | |
| | | | | | | | | | | | | | | 491.3 | |
| **16** | 1 | 0 | CH3 | H | H | H | 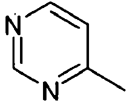 | Et | CH | CH | CH | CH | - | 6.59 | >280 |
| | | | | | | | | | | | | | | 6.5% | |
| | | | | | | | | | | | | | | 458.3 | |
| **17** | 1 | 0 | CH3 | H | H | H | 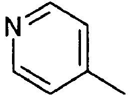 | Et | CH | CH | CH | CH | HCl (1.07) | 5.07 | 250 |
| | | | | | | | | | | | | | | 96% | |
| | | | | | | | | | | | | | | 457.3 | |
| **18** | 1 | 2 | CH3 | H | H | H | 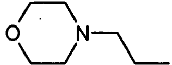 | Et | CH | CH | CH | CH | HCl (1.07) | 5.34 | 220-222 |
| | | | | | | | | | | | | | | 100% | |
| | | | | | | | | | | | | | | 493,2 | |
| **19** | 1 | 0 | CH3 | H | H | H | 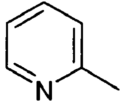 | Et | CH | CH | N | CH | HCl (1.15) | 6.3 | 268-288 |
| | | | | | | | | | | | | | | 97.7% | |
| | | | | | | | | | | | | | | 458.3 | |
| **20** | 1 | 0 | CH3 | H | CH3 | H | 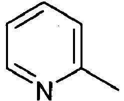 | Et | CH | CH | CH | CH | HCl (1.07) | 7.7 | 188 |
| | | | | | | | | | | | | | | 97.7% | |
| | | | | | | | | | | | | | | 471.1 | |
| **21** | 1 | 0 | CH3 | H | H | H | 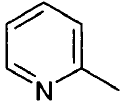 | Et | CH | CH | CH | N | - | 6.5 | 266 |
| | | | | | | | | | | | | | | 96.3% | |
| | | | | | | | | | | | | | | 458.1 | |
| **22** | 1 | 1 | CH3 | H | H | H | 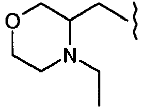 | Et | CH | CH | CH | CH | HCl (1.15) | 5.4 | 188-190 |
| | | | | | | | | | | | | | | 95% | |
| | | | | | | | | | | | | | | 507.1 | |
| **23** | 1 | 1 | CH3 | H | H | H | 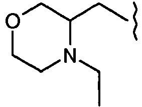 | Et | CH | CH | CH | N | HCl (1.07) | 4.6 | 199 |
| | | | | | | | | | | | | | | 97.8% | |
| | | | | | | | | | | | | | | 508.4 | |
| **24** | 1 | 1 | CH3 | H | H | H | 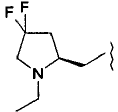 | Et | CH | CH | CH | CH | HCl (1.07) | 6.04 | 266 |
| | | | | | | | | | | | | | | 100% | |
| | | | | | | | | | | | | | | 527.3 | |
| **25** | 1 | 1 | CH3 | H | CH3 | H | 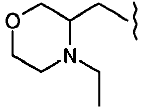 | Et | CH | CH | CH | CH | HCl (1.07) | 5.7 | 190 |
| | | | | | | | | | | | | | | 96.5% | |
| | | | | | | | | | | | | | | 521.3 | |
| **26** | 1 | 1 | CH3 | H | H | H | 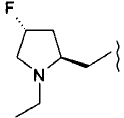 | Et | CH | CH | CH | CH | HCl (1.07) | 5.2 | 170 |
| | | | | | | | | | | | | | | 96.7% | |
| | | | | | | | | | | | | | | 509.3 | |
| **27** | 1 | 1 | CH3 | H | H | H | 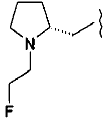 | Et | CH | CH | CH | CH | HC (1.07) | 4.64 | 180 |
| | | | | | | | | | | | | | | 509.3 | |
| **28** | 1 | 1 | CH3 | H | H | H | 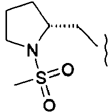 | Et | CH | CH | CH | CH | - | 6.7 | >260 |
| | | | | | | | | | | | | | | 97.2% | |
| | | | | | | | | | | | | | | 541.2 | |
| **29** | 1 | 1 | CH3 | H | H | H | 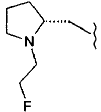 | Et | CH | CH | N | CH | HCl (1.07) | 4.97 | 194 |
| | | | | | | | | | | | | | | 92% | |
| | | | | | | | | | | | | | | 510.2 | |
| **30** | 1 | 1 | CH3 | H | H | H | 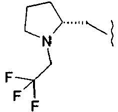 | Et | CH | CH | CH | CH | - | 7.1 | >260 |
| | | | | | | | | | | | | | | 99.1% | |
| | | | | | | | | | | | | | | 545.2 | |
| **31** | 1 | 1 | CH3 | H | H | H | 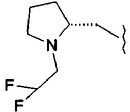 | Et | CH | CH | CH | CH | HCl (1.07) | 9.8 (2) | 214-228 |
| | | | | | | | | | | | | | | 96.4% | |
| | | | | | | | | | | | | | | 527.3 | |
| **32** | 1 | 1 | CH3 | H | H | H | 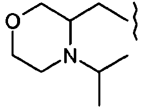 | Et | CH | CH | CH | CH | HCl (1.07) | 9.1 (2) | 192 |
| | | | | | | | | | | | | | | 93.2% | |
| | | | | | | | | | | | | | | 521.3 | |
| **33** | 1 | 1 | CH3 | H | H | H | 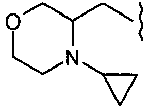 | Et | CH | CH | CH | CH | HCl (1.07) | 5.5 | 250 |
| | | | | | | | | | | | | | | 97.8% | |
| | | | | | | | | | | | | | | 519.2 | |
| **34** | 1 | 1 | CH3 | H | H | H | 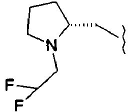 | Et | CH | CH | N | CH | HCl (1.07) | 5.2 | 260 |
| | | | | | | | | | | | | | | 92.1% | |
| | | | | | | | | | | | | | | 528.2 | |
| **35** | 1 | 0 | CH3 | H | H | H | 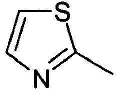 | Et | CH | CH | CH | CH | - | 7.2 | 303 |
| | | | | | | | | | | | | | | 94.4% | |
| | | | | | | | | | | | | | | 463.2 | |
| **36** | 1 | 1 | CH3 | H | H | H | 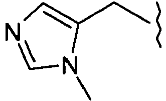 | Et | CH | CH | CH | CH | HCl (1.07) | 5.2 | 270 |
| | | | | | | | | | | | | | | 96.3% | |
| | | | | | | | | | | | | | | 474.2 | |
| **37** | 1 | 2 | CH3 | H | H | H | 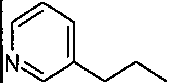 | Et | CH | CH | CH | CH | HCl (1.07) | 5.4 | 222 |
| | | | | | | | | | | | | | | 98.7% | |
| | | | | | | | | | | | | | | 485.2 | |
| **38** | 1 | 3 | CH3 | H | H | H | 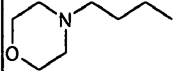 | Et | CH | CH | CH | CH | HCl (1.07) | 5.2 | 196 |
| | | | | | | | | | | | | | | 98.2% | |
| | | | | | | | | | | | | | | 507.2 | |
| **39** | 1 | 2 | CH3 | H | H | H | 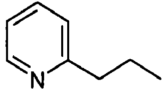 | Et | CH | CH | CH | CH | HCl (1.07) | 5.4 | 248 |
| | | | | | | | | | | | | | | 96.5% | |
| | | | | | | | | | | | | | | 485.2 | |
| **40** | 1 | 2 | CH3 | H | H | H | 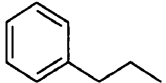 | Et | CH | CH | CH | CH | - | 7.7 | 270 |
| | | | | | | | | | | | | | | 99.3% | |
| | | | | | | | | | | | | | | 484.2 | |
| **41** | 1 | 3 | CH3 | H | H | H | 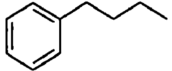 | Et | CH | CH | CH | CH | - | 8.3 (3) | 278 |
| | | | | | | | | | | | | | | 97.1% | |
| | | | | | | | | | | | | | | 498.3 | |
| **42** | 1 | 2 | CH3 | H | H | H | 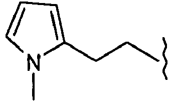 | Et | CH | CH | CH | CH | - | 7.3 | 252 |
| | | | | | | | | | | | | | | 96.4% | |
| | | | | | | | | | | | | | | 487.2 | |
| **43** | 1 | 1 | CH3 | H | H | H | 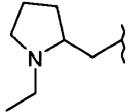 | Et | CH | S | N | - | HCl (1.07) | 5.1 | 228 |
| | | | | | | | | | | | | | | 96.2% | |
| | | | | | | | | | | | | | | 498.2 | |
| **44** | 1 | 0 | CH3 | H | H | H | 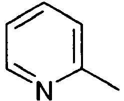 | 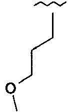 | CH | CH | CH | CH | HCl (1.07) | 6.45 | 230 |
| | | | | | | | | | | | | | | 99.8% | |
| | | | | | | | | | | | | | | 501.3 | |
| **45** | 1 | 0 | CH3 | H | H | H | 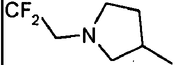 | Et | CH | CH | CH | CH | - | 5.4 | 267 |
| | | | | | | | | | | | | | | 98% | |
| | | | | | | | | | | | | | | 513.2 | |
| **46** | 2 | 1 | CH3 | H | H | H | 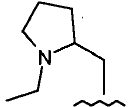 | Et | CH | CH | CH | CH | - | 9.8(2) | 218 |
| | | | | | | | | | | | | | | 97.6% | |
| | | | | | | | | | | | | | | 505.4 | |
| **47** | 3 | 1 | CH3 | H | H | H | 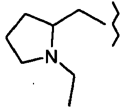 | Et | CH | CH | CH | CH | - | 5.7 | 230 |
| | | | | | | | | | | | | | | 97.6% | |
| | | | | | | | | | | | | | | 519.4 | |
| **48** | 1 | 1 | -(CH₂)₂-CH₃ | H | H | H | 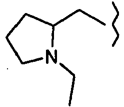 | Et | CH | CH | | CH | - | 5.9 | 264 |
| | | | | | | | | | | | CH | | | 96.8% | |
| | | | | | | | | | | | | | | 519.4 | |
| **49** | 1 | 1 | CH3 | H | H | H | 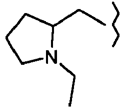 | CH₂CF₃ | CH | CH | CH | CH | - | 5.6 | 300 |
| | | | | | | | | | | | | | | 97.1% | |
| | | | | | | | | | | | | | | 545.2 | |
| **50** | 1 | 1 | CH3 | H | H | H | 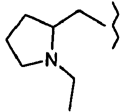 | 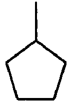 | CH | CH | CH | CH | - | 5.7 | 222 |
| | | | | | | | | | | | | | | 98.7% | |
| | | | | | | | | | | | | | | 531.4 | |
| **51** | 4 | 1 | CH3 | H | H | H | 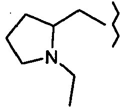 | Et | CH | CH | CH | CH | - | 5.9 | 180 |
| | | | | | | | | | | | | | | 94.2% | |
| | | | | | | | | | | | | | | 533.4 | |
| **52** | 1 | 1 | CH3 | H | H | H | 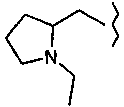 | Et | - | S | CH | CH | - | 5.3 | 250 |
| | | | | | | | | | | | | | | 98% | |
| | | | | | | | | | | | | | | 497 | |
| **53** | 1 | 1 | CH3 | H | H | H | 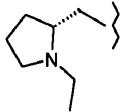 | IsoBu | CH | CH | CH | CH | - | 5.66 | 233 |
| | | | | | | | | | | | | | | 98.9% | |
| | | | | | | | | | | | | | | 519.3 | |
| **54** | 1 | 1 | CH3 | 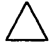 | | H | 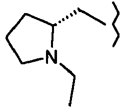 | Et | CH | CH | CH | CH | - | 5.8 | 189 |
| | | | | | | | | | | | | | | 99.3% | |
| | | | | | | | | | | | | | | 517.2 | |
| **55** | 1 | 1 | CH3 | H | H | H | 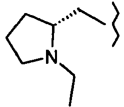 | Et | - | N | CH | S | - | 5.06 | 214 |
| | | | | | | | | | | | | | | 99.6% | |
| | | | | | | | | | | | | | | 498.2 | |
| **56** | 1 | 1 | CH3 | H | H | H | 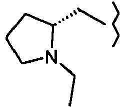 | Et | CH | CF | CH | CH | - | 5.47 | 253 |
| | | | | | | | | | | | | | | 98% | |
| | | | | | | | | | | | | | | 509.2 | |
| **57** | 1 | 1 | CH3 | H | H | H | 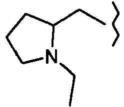 | Et | CCI | CH | CH | CH | HCl (1.07) | 5.58 | 228 |
| | | | | | | | | | | | | | | 96.6% | |
| | | | | | | | | | | | | | | 525.2 | |
| **58** | 1 | 1 | CH3 | H | H | H | 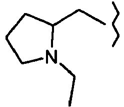 | Et | CF | CH | CH | CH | - | 5.45 | 275 |
| | | | | | | | | | | | | | | 93.2% | |
| | | | | | | | | | | | | | | 509.2 | |
| **59** | 1 | 1 | CH3 | H | H | H | 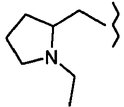 | Et | CCH3 | CH | CH | CH | - | 5.51 | 277 |
| | | | | | | | | | | | | | | 96% | |
| | | | | | | | | | | | | | | 505.2 | |
| **60** | 1 | 1 | CH3 | H | H | H | 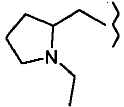 | 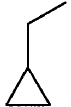 | CH | CH | CH | CH | - | 5.59 | 260 |
| | | | | | | | | | | | | | | 98.3% | |
| | | | | | | | | | | | | | | 517.2 | |
| **61** | 1 | 1 | CH3 | H | H | H | 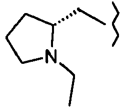 | Et | CH | CCH3 | CH | CH | - | 5.52 | 178 |
| | | | | | | | | | | | | | | 92% | |
| | | | | | | | | | | | | | | 505.2 | |

**Tableau 2 :**

| | | | | | | | | | | | |
|---|---|---|---|---|---|---|---|---|---|---|---|
| 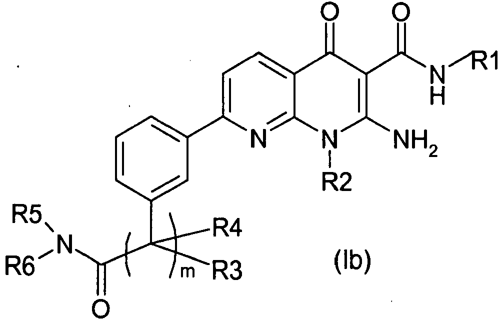 | | | | | | | | | | | |
| Avec Y, V, W représentent un groupe -CH-, Z représente un atome de carbone, R7 représente un atome d'hydrogène et R6 = -(CH₂)ₙ-L (*) condition LC/UV/MS, si elles ne sont pas mentionées, ce sont les conditions 1 qui sont utilisées. | | | | | | | | | | | |

| **N°** | **m** | **n** | **R1** | **R3** | **R4** | **R5** | **R6** | **R2** | **Sel** | LCMS tr min (*) pureté MH+ | **Point de fusion (°C)** |
|---|---|---|---|---|---|---|---|---|---|---|---|
| **62** | 1 | 1 | CH3 | H | H | H | 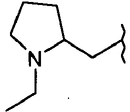 | Et | HCl (1.07) | 5.4 | 213 |
| | | | | | | | | | | 97.7% | |
| | | | | | | | | | | 491.4 | |
| **63** | 1 | 0 | CH3 | H | H | H | 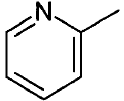 | Et | HCl (1.07) | 6.3 | 176 |
| | | | | | | | | | | 99.6% | |
| | | | | | | | | | | 457.3 | |

**Tableau 3 :**

| | | | | | | | | | | | |
|---|---|---|---|---|---|---|---|---|---|---|---|
| 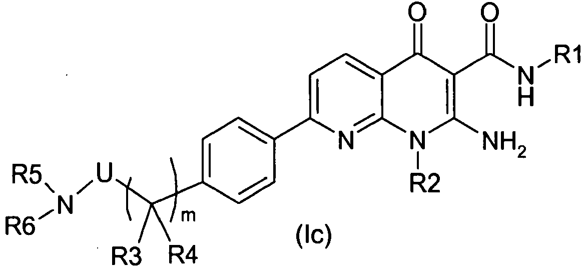 | | | | | | | | | | | |
| Avec U = -CH₂-, V, W représentent un groupe -CH-, Z représente un atome de carbone, R7 représente un atome d'hydrogène et R6 = -(CH₂)ₙ-L | | | | | | | | | | | |
| (*) condition LC/UV/MS, si elles ne sont pas mentionées, ce sont les conditions 1 qui sont utilisées. | | | | | | | | | | | |

| **N°** | **m** | **n** | **R1** | **R3** | **R4** | **R5** | **R6** | **R2** | **Sel** | LCMS tr min (*) pureté MH+ | **point de fusion (°C)** |
|---|---|---|---|---|---|---|---|---|---|---|---|
| **64** | 1 | 0 | CH3 | H | H | H | 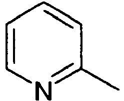 | Et | HCl (1.07) | 5.57 | 242 |
| | | | | | | | | | | 97.5% | |
| | | | | | | | | | | 443.2 | |

Les composés selon l'invention ont fait l'objet d'essais pharmacologiques permettant de déterminer leur effet inhibiteur de protéine à activité tyrosine kinases.
A titre d'exemple, leurs effets inhibiteurs de l'activité tyrosine kinase de PDGF-R et/ou Flt-3, ont été mesurés in vitro dans des modèles cellulaires.

L'activité inhibitrice vis à vis des recepteurs PDGF ou Flt-3 est donnée par la concentration qui inhibe 50% de l'activité de prolifération respectivement des cellules Baf3 tel/PDGF ou MV4-11.

### Mesure de l'inhibition de l'activité tyrosine kinase du récepteur au PDGF beta (PDGF-Rβ) (Baf-3 tel/PDGFRβ) :

Ce test consiste à évaluer les effets des composés sur l'activité tyrosine kinase du récepteur PDGF beta.

L'effet d'inhibition des composés selon l'invention envers l'activité tyrosine kinase du récepteur PDGF-R a été évalué sur la lignée cellulaire murine hématopoïétique BaF/3 transfectée avec un plasmide codant pour la protéine de fusion Tel/PDGF-R beta. Cette protéine de fusion est retrouvée dans les leucémies chroniques myéloïdes myélomonocytaires (CMML). Elle comprend la partie N-Terminale du facteur de transcription Tel et la partie transmembranaire et intracellulaire du récepteur PDGF-R beta. Cette protéine de fusion est présente sous forme dimérisée (présence d'un domaine d'oligomérisation dans la partie N-Terminale de Tel) et de ce fait conduit à l'activité constitutive du domaine kinase de PDGF-R beta. Cette lignée BaF3 Tel/PDGF a été décrite dans la littérature à plusieurs reprises et notamment de façon détaillée dans l'article de M CARROLL et coll, PNAS,1996, 93, 14845-14850, M CARROLL et coll.,Blood 2002, 99, 14845-14850.

Les cellules BaF3 Tel/PDGF sont lavées par du tampon phosphate et ensemencées dans des plaques de 96 puits, à la densité de 5.10⁴ cellules/ml (100 ml par puits), dans du RPMI 1640 contenant 10% de SVF, en présence ou en absence des composés à tester. Après 72 h d'incubation, les cellules viables sont quantifiées par mesure de l'ATP cellulaire grâce à l'utilisation du kit CellTiter-Glo^{®} (Promega, Cat G7571). Les cellules sont traitées selon les instructions données par le fournisseur du kit et la luminescence est mesurée à l'aide d'un Luminoskan (Ascent, Labsystem) avec les paramètres suivants : mesure : unique ; temps d'intégration : 1000 ms, lag time : 5 s.

Il apparaît ainsi que les composés selon l'invention ont une activité inhibitrice sur l'activité tyrosine kinase de PDGF-R beta. Cette activité est donnée par la concentration qui inhibe 50% de la prolifération des cellules Baf3 tel/PDGF (CI₅₀). Les CI₅₀ des composés selon l'invention sont inférieures à 1,0 µM.

Par exemple, les composés n° 6, 14, 19, 22, 24, 29, 33, 35, 41, 47 et 48 ont montré une CI₅₀ de respectivement 11, 11, 27, 0.07, 2, 35, 4.7, 3.7, 5.5, 48, 162 nM dans le test de mesure de l'activité tyrosine kinase du récepteur PDGF.

### Mesure de l'inhibition de l'activité tyrosine kinase du récepteur PDGF alpha :

L'effet d'inhibition des composés selon l'invention envers l'activité tyrosine kinase du récepteur PDGF alpha a été évalué sur la lignée cellulaire EOL-1, lignée établie à partir d'une leucémie de type Leucémie chronique à eosinophiles (CEL) actif de façon constitutive. La lignée EOL-1 a été décrite comme exprimant la protéine de fusion FIP1 L1-PDGF-Ralpha et est sensible à des inhibiteurs de kinases dans des tests de prolifération (Cools J et coll, Blood 2004, 103, 2802-2005). L'activité inhibitrice est corrélée à l'inhibition de la croissance des cellules.

Les cellules EOL-1 sont lavées par du tampon PBS et ensemencées dans des plaques de 96 puits, à la densité de 5.10⁴ cellules/ml (100 µl par puits), dans du RPMI 1640 contenant 10% de SVF, en présence ou en absence des composés à tester. Après 72 h d'incubation, les cellules viables sont quantifiées par mesure de l'ATP cellulaire grâce à l'utilisation du kit CellTiter-Glo^{®} (Promega, Cat G7571). Les cellules sont traitées selon les instructions données par le fournisseur du kit et la luminescence est mesurée à l'aide d'un Luminoskan (Ascent, Labsystem) avec les paramètres suivants : mesure : unique ; temps d'intégration : 1000 ms, lag time : 5 s.
Il apparaît ainsi que les composés selon l'invention ont une activité inhibitrice sur l'activité tyrosine kinase de PDGF-R alpha. Cette activité est donnée par la concentration qui inhibe 50% de la prolifération des cellules EOL-1. Les CI₅₀ des composés selon l'invention sont inférieures à 1,0 µM.

Par exemple, les composés n°5, 6, 22, 19, 24, 33, 35, 43, 50, 51 ont montré une CI₅₀ de respectivement, 1.6, 0.57, <0.01, 0.4, <0.01, <0.01, 0.8, 29.7, 3.2, 92.7 nM dans le test de mesure de l'activité tyrosine kinase du récepteur PDGF alpha.

Outre leurs propriétés d'inhibition du PDGF-R tyrosine kinase, il apparaît également que des composés conformes à l'invention présentent des propriétés d'inhibition de l'activité tyrosine kinase du récepteur Flt-3, comme décrit ci-après.

### Mesure de l'inhibition de l'activité tyrosine kinase du récepteur Flt-3

L'effet d'inhibition des composés selon l'invention envers l'activité tyrosine kinase du récepteur Flt-3 a été évalué sur la lignée cellulaire MV4-11, lignée établie à partir d'une leucémie de type AML et porteuse d'un mutant Flt3ITD, actif de façon constitutive. L'activité inhibitrice est corrélée à l'inhibition de la croissance des cellules, selon les protocoles décrits par SPIEKERMANN, K. et coll, Blood, 2003, 101, (4) 1494-1504 et O'FARRELL, A.-M. et coll., Blood, 2003, 101, (9) 3597-3605.

Les cellules MV4-11 sont lavées par du tampon PBS et ensemencées dans des plaques de 96 puits, à la densité de 1.10⁵ cellules/ml (100 µl par puits), dans du RPMI 1640 contenant 10% de SVF, en présence ou en absence des composés à tester. Après 72 h d'incubation, les cellules viables sont quantifiées par mesure de l'ATP cellulaire grâce à l'utilisation du kit CellTiter-Glo^{®} (Promega, Cat G7571). Les cellules sont traitées selon les instructions données par le fournisseur du kit et la luminescence est mesurée à l'aide d'un Luminoskan (Ascent, Labsystem) avec les paramètres suivants : mesure : unique ; temps d'intégration : 1000 ms, lag time : 5 s.

L'activité inhibitrice vis à vis du recepteur Flt-3 est donnée par la concentration qui inhibe 50% de la prolifération des cellules MV4-11. Il apparaît ainsi que des composés selon l'invention ont une activité inhibitrice sur l'activité tyrosine kinase du récepteur Flt-3 avec des CI₅₀ inférieures à 1,0 µM.
Par exemple les composés n°3, 4, 5, 6, 14, 16, 19, 22, 24, 33, 35 ont montré une CI₅₀ de respectivement 0.33, 0.21, 0.19, 0.1, 0.34, 0.182, 0.1, 0.13, 0.19, 0.056, 0.083 µM, dans le test de mesure de l'activité tyrosine kinase du recepteur Flt-3.

Une activité ex vivo des composés de l'invention a été mesurée. Ces composés sont alors administrés par voie orale à des souris femelles Balb/c à une dose de 10, 30 ou 100 mg/ kg en suspension ou en solution dans un véhicule tel que methylcellulose/ tween et l'inhibition de l'activité tyrosine kinase est mesurée in vitro dans un test cellulaire, à partir des plasmas prélevés à 15 minutes, 1 heure et 4 heure.

### Mesure de l'inhibition de l'activité tyrosine kinase du récepteur PDGF alpha ex vivo :

### 1) protocole d'administration des produits aux souris

Les produits sont préparés au mortier avec 0.5% de tween 80 et de la méthylcellulose 0.6% qsp volume final. Selon les cas des préparations de véhicules différents ont pu être utilisés : eau, mélange labrasol solutol eau glucosée 5%. Les produits mis en suspension ou solubilisés sont administrés par gavage (10ml/kg) à des souris femelles Balb/c âgées de 8 à 15 sem.

Les animaux sont ensuite prélevés au temps déterminé par le protocole (15min, 1h, 4h, 16h).

Les animaux sont anesthésiés par injection (10ml/kg) intra-péritonéale d'un mélange (0.1g/kg) kétamine- (20mg/kg) xylazine. Une laparotomie est effectuée pour mettre à nue l'ensemble aorte abdominale descende et veine cave inférieure. Le prélèvement sanguin est réalisé sur seringue et aiguille sèches au niveau de la veine ou de l'artére. Le sang est immédiatement transféré dans des tubes héparine lithium (BD microtainer). Une centrifugation de 3 min à 12000 tr/min permet de récupérer du plasma sur lequel le test biochimique peut être effectué après conservation par congélation à - 20°C.

### 2) préparation des cellules HEK Tel/PDGF-R et mesure de la phosphorylation de PDGF-R

Afin de détecter l'activité ex-vivo des produits à partir des plasmas de souris, les cellules HEK sont au préalable transfectées transitoirement avec un vecteur d'expression codant pour la protéine de fusion Tel/PDGF-R (protéine de fusion décrite précédemment). Après 24h de transfection, les celllules sont privées en sérum sur la nuit puis incubées pendant 30 min avec les plasmas des animaux traités avec les produits de l'invention. Les cellules sont ensuite lysées dans du tampon RIPA, puis la détection de la phosphorylation de PDGF-R beta est réalisée par technique ELISA, en utilisant un kit commerçial (R&D systems, DYC1767). Les résultats sont exprimés en pourcentage d'inhibition de l'autophosphorylation.
Dans ce test de mesure de l'activité *ex vivo,* l'effet des composés de l'invention est quantifié par le pourcentage d'inhibition de la phosphorylation du domaine kinase du récepteur PDGF-R beta dans des cellules HEK, induite par l'activité inhibitrice du produit présent dans le plasma des animaux traités, tel que décrit dans le tableau suivant :

| Administration 30mg/kg po | % d'inhibition de l'autophosphorylation du PDGFR tyrosine kinase dans les cellules HEK. | | |
|---|---|---|---|
| Composé n=° | 15 min | 1h | 4h |
| 18 | 55 | 62 | 17 |
| 24 | 85 | 57 | 74 |
| 26 | 58 | 67 | 20 |
| 31 | 85 | 68 | 42 |
| 33 | 80 | 82 | 75 |
| 34 | 73 | 64 | 58 |

Ainsi, selon un des objets de la présente invention, les composés de formule (I) présentent une activité très intéressante d'inhibition de la phosphorylation du domaine kinase du récepteur PDGF-R beta dans des cellules HEK, induite par l'activité inhibitrice du produit présent dans le plasma des animaux traités.

Les composés selon l'invention sont donc des inhibiteurs de protéines kinases, notamment des récepteurs tyrosine kinase PDGF alpha et beta et, pour certains d'entre eux, également du récepteur Flt-3 tyrosine kinase.

Les composés selon l'invention peuvent donc être utilisés pour la préparation de médicaments, en particulier de médicaments inhibiteurs de protéine kinases.

Il s'agit de médicaments inhibiteurs de protéine kinase, notamment de médicaments inhibiteurs du recepteur PDGF-R tyrosine kinase et éventuellement du récepteur Flt-3 tyrosine kinases.

Ainsi, selon un autre de ses aspects, l'invention a pour objet des médicaments qui comprennent un composé de formule (I), ou un sel d'addition de ce dernier à un acide pharmaceutiquement acceptable, ou encore un solvat du composé de formule (I).

Ces médicaments trouvent leur emploi en thérapeutique, notamment dans le traitement des maladies liées à l'activité des protéines kinases et notamment des maladies prolifératives telles que les cancers à tumeurs liquides, les leucémies chroniques ou aiguës, les lymphomes lymphocytaires, la maladie de Hodgkin, et les syndromes myeloproliferatifs, et les syndromes myelodysplasiques.

Et telles que les cancers à tumeurs solides par exemple les cancers du poumon (NSCLC), des os, du pancréas, de la peau, syndrome de Kaposi, les mélanomes intraoculaires, les cancers du sein, de l'utérus, du col de l'utérus, des ovaires, de l'endomètre, du vagin, de la vulve, de l'urètre, du pénis, de la prostate, les carcinomes des trompes de Fallope, les cancers tel que les GIST et de la région anale, du rectum, de l'intestin grêle, du colon, de l'estomac, de l'oesophage, des glandes endocrines, thyroïdes, parathyroïdes ou surrénales, les sarcomes des tissus mous, sarcomes d'Ewing, des ostésarcomes, dermatofibrosarcome et autres fibrosarcomes, les cancers de la vessie ou du rein, les néoplasmes du système nerveux central, les tumeurs de la colonne vertébrale et desmoïdes, les gliomes du tronc cérébral et glioblastomes, les adénomes pituitaires et leurs métastases.

Un autre aspect de l'invention comprend une association entre au moins un composé selon l'invention et au moins un agent de chimiothérapie.

En effet, les composés de la présente invention peuvent être utilisés seuls ou en mélange avec au moins un agent de chimiothérapie pouvant être choisi parmi des agents cytotoxiques et/ou des antiangiogènes. Par exemple, les agents antiangiogènes peuvent être un composé inhibiteur de l'activité kinase de VEGF-R ou un composé antagoniste d'un facteur de croissance.

Il est également possible de combiner les composés selon l'invention avec un traitement par radiations.

Les combinaisons des composés de l'invention avec les agents de chimiothérapie cités ci-dessus et/ou les radiations sont un autre objet de la présente invention.

Les agents de chimiothérapie cités ci-dessus et/ou les radiations peuvent être administrés simultanément, séparément ou séquentiellement. Le traitement sera adapté par le praticien en fonction du malade à traiter.

Ces médicaments trouvent également leur emploi en thérapeutique, dans des maladies prolifératives non-malignes, comme par exemple la resténose, l'athérosclérose, la thrombose, l'insuffisance cardiaque, l'hypertrophie cardiaque, hypertension artérielle pulmonaire, la fibrose, la néphropathie diabétique, la glomérulonéphrite, la pyélonéphrite chronique, les hemangiomes, les maladies auto-immunes telles que le psoriasis, les sclérodermatites, l'immunosuppression (rejet de greffes par exemple).

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable de ce dernier, ou encore un un solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, intratrachéale, intranasale, transdermique ou rectale, le principe actif de formule (I) ci-dessus, ou son sel ou solvat éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention, selon un autre de ses aspects, concerne également une méthode de traitement des pathologies ci-dessus indiquées qui comprend l'administration, à un patient, d'une dose efficace d'un composé selon l'invention ou un de ses sels pharmaceutiquement acceptables ou solvats.

## Revendications

1. Composé répondant à la formule (I) 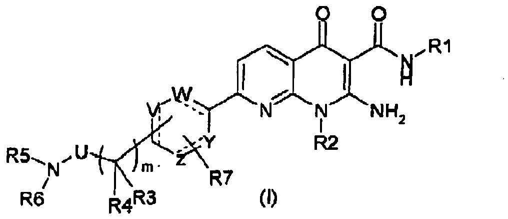 **R1** représente un groupe (C1-C4) alkyle,
**R2** représente -(CH₂)ₙ-B où n'=0,1,2,3,4 et **B** est un groupe (C3-C5)cycloalkyle ou un groupe (C1-C4) alkyle éventuellement substitué par un ou plusieurs atomes de fluor, un groupe (C1-C4) alcoxy,
U représente un groupe carbonyle ou un groupe -CH₂-,
**Y, Z, V** et **W** représentent indépendamment les uns des autres un groupe - CH- ou un atome de carbone éventuellement substitué par un groupe R7 ou un hétéroatome, ou pas d'atome, étant entendu que le cycle doit être aromatique et comprendre entre 5 et 6 chainons,
**R3, R4** représentent indépendamment l'un de l'autre un atome d'hydrogène ou un groupe (C1-C4) alkyle linéaire, ou R3 et R4 forment ensemble avec le carbone auxquels ils sont liés un groupe (C3-C5) cycloalkyle,
**m** est un nombre entier égal à 1, 2, 3, 4,
**R5** représente un atome d'hydrogène ou un groupe (C1-C4) alkyle,
**R6** représente -(CH₂)ₙ-L dans lequel n= 0, 1, 2, 3 et L est un groupe sélectionné indépendamment parmi les groupes suivants :
o un groupe (C1-C5) alkyle éventuellement substitué par un groupe (C.1-C4) alcoxy,
o un groupe (C3-C5) cycloalkyle,
o un aryle comprenant 6 atomes de carbone et éventuellement substitué par un ou plusieurs atomes d'halogènes,
o un hétéroaryle comprenant entre 5 et 6 chainons dont au moins un hétéroatome choisi parmi un atome d'azote ou de soufre, éventuellement substitué par un groupe (C1-C4) alkyle,
o un hétérocycle saturé où ledit hétérocycle comprend entre 4 et 7 chainons dont au moins un hétéroatome choisi parmi un atome d'azote, un atome d'oxygène et est éventuellement substitué en des positions quelconques, y compris sur l'atome d'azote par un ou plusieurs substituants parmi un atome de fluor, un groupe fluoroalkyle en (C1-C4), un groupe (C1-C4)alkyle linéaire ou ramifié, un groupe (C3-C5) cycloalkyle ou un groupe (C1-C4)alkyl-sulfonamide, la configuration absolue d'un carbone substitué sur ledit hétérocycle pouvant être R ou S, ou racémique,
**R7** représente un atome d'hydrogène ou un groupe (C1-C4) alkyle ou un atome d'halogène,
à l'état de base ou de sel d'addition à un acide, à l'état de solvat, ainsi que ses énantiomères et diastéréoisomères, y compris ses mélanges.

2. Composé de formule (I) selon la revendication 1, **caractérisé en ce que**
**R1** représente un groupe (C1-C4) alkyle,
et/ou
**R2** représente -(CH₂)ₙ-**B** où n'= 0, 1 et **B** est un groupe (C3-C5) cycloalkyle ou un groupe (C1-C4) alkyle éventuellement substitué par un ou plusieurs atomes de fluor, un groupe (C1-C4) alcoxy,
et/ou
**U** représente un groupe carbonyle ou un groupe -CH₂-,
et/ou
**Y, Z, V** et **W** représentent indépendamment les uns des autres un groupe - CH- ou un atome de carbone éventuellement substitué par un groupe R7 ou un hétéroatome, ou pas d'atome, étant entendu que le cycle doit être aromatique et comprendre entre 5 et 6 chainons,
et/ou
**R3, R4** représentent indépendamment l'un de l'autre un atome d'hydrogène ou un groupe (C1-C4) alkyle linéaire ou R3 et R4 forment ensemble avec le carbone auxquels Ils sont liés un groupe (C3-C5) cycloalkyle,
et/ou
**m** est un nombre entier égal à 1, 2, 3, 4,
et/ou
**R5** représente un atome d'hydrogène ou un groupe (C1-C4) alkyle,
et/ou
**R8** représente -(CH₂)ₙ-L dans lequel n= 0, 1, 2, 3 et L est un groupe sélectionné indépendamment parmi les groupes suivants,
o un groupe (C1-C5) alkyle éventuellement substitué par un groupe (C1-C4) alcoxy ,
o un groupe (C3-C5) cycloalkyle,
o aryle comprenant 6 atomes de carbone et éventuellement substitué par un ou plusieurs atomes d'halogènes,
o hétéroaryle comprenant entre 5 et 6 chainons dont au moins un hétéroatome choisi parmi un atome d'azote ou de soufre, éventuellement substitué par un groupe (C1-C4) alkyle,
o hétérocycle saturé où ledit hétérocycle comprend entre 5 et 7 chainons dont au moins un hétéroatome choisi parmi un atome d'azote, un atome d'oxygène et est éventuellement substitué en des positions quelconques, y compris sur l'atome d'azote par un ou plusieurs substituants parmi un atome de fluor, un groupe fluoroalkyle an (C1-C4), un groupe (C1-C4) alkyle linéaire ou ramifié, un groupe (C3-C5) cycloalkyle ou un groupe (C1-C4) alkyl-sulfonamide, la configuration absolue d'un carbone substitué sur ledit hétérocycle pouvant être R ou S, ou racémique,
et/ou
**R7** représente un atome d'hydrogène ou un groupe (C1-C4) alkyle ou un atome d'halogène,
à l'état de base ou de sel d'addition à un acide, à l'état de solvat, ainsi que ses énantiomères et diastéréoisomères, y compris ses mélanges.

3. Composé de formule (I) selon la revendication 1, **caractérisé en ce que** U représente un groupe carbonyle.

4. Composé de formule (I) selon la revendication 1, **caractérisé en ce que** le cycle comprenant **Y, Z, V** et **W** est choisi parmi les groupes phényle, pyridine, thiazole, thiophène.

5. Composé de formule (I) selon la revendication 1, **caractérisé en ce que R3** et/ou **R4** et/ou **R5** représentent un atome d'hydrogène.

6. Composé de formule (I) selon la revendication 1, **caractérisé en ce que** la chaîne -[C(R3R4)]ₘ-U-N(R5)(R6) est en position para ou meta par rapport au cycle auquelle elle est liée.

7. Composé de formule (I) selon la revendication 1, **caractérisé en ce qu'**il est choisi parmi un des composés suivants :
2-amino-1-ethyl-N-methyl-4-oxo-7-{4-[2-oxo-2-(pheny(amino)ethyljphenyl}-1,4-dihydro-1,8-naphthyridine-3-carboxamide,
2-amlno-1-ethyl-7-[4-(2-{[(1-ethylpyrrolidin-2-yl)methyl](methyl)amino}-2-oxoethyl)phenyl]-N-methyl-4-oxo-1,4-dihydro-1,8-naphthyridine-3-carboxamide,
2-amino-1-ethyl-N-methyl-4-oxo-7-{4-[2-oxo-2-(pyridin-3-ylamlno)ethyl]phenyl}-1,4-dihydro-1,8-naphthyridine-3-carboxamide,
2-amino-1-ethyl-N-methyl-4-oxo-7-{4-[2-oxo-2-(pyridin-2-ylamino)ethyl]phenyl}-1,4-dihydro-1,8-naphthyridine-3-carboxamide,
2-amino-7-(4-{2-[(2-chlorophenyl)amino]-2-oxoethyl}phenyl)-1-ethyl-N-methyl-4-oxo-1,4-dihydro-1,8-naphthyridine-3-carboxamide,
2-amino-7-(4-{2-[(3,5-difluorophenyl)amino]-2-oxoethyl}phenyl)-1-ethyl-N-methyl-4-oxo-1,4-dihydro-1,8-naphthyridine-3-carboxamide,
2-amino-1-ethyl-N-methyl-4-oxo-7-(4-{2-oxo-2-[(pyridin-4-ylmethyl)amino]ethyl}phenyl)-1,4-dihydro-1,8-naphthyridine-3-carboxamide,
2-amino-1-ethyl-N-methyl-4-oxo-7-(4-{2-oxo-2-[(pyridin-2-ylmethyl)amino]ethyl}phenyl)-1,4-dihydro-1,8-naphthyridine-3-carboxamide,
2-amino-1-ethyl-7-(4-{2-[(2-methoxyethyl)amino]-2-oxoethyl}phenyl)-N-methyl-4-oxo-1,4-dihydro-1,8-naphthyridine-3-carboxamide,
2-amino-7-{4-[2-(cyclopropylamino)-2-oxoethyl]phenyl}-1-ethyl-N-methyl-4-oxo-1,4-dihydro-1,8-naphthyridine-3-carboxamide,
2-amino-1-ethyl-N-methyl-7-(4-{2-[(1-methylethyl)amino]-2-oxoethyl}phenyl)-4-oxo-1,4-dihydro-1,8-naphthyridine-3-carboxamide,
2-amino-7-{4-[2-(cyclopentylamino)-2-oxoethyl]phenyl}-1-ethyl-N-methyl-4-oxo-1,4-dihydro-1,8-naphthyridine-3-carboxamide,
2-amino-1-ethyl-N-methyl-4-oxo-7-{4-[2-oxo-2-(pyrazin-2-ylamino)ethyl]phenyl}-1,4-dihydro-1,8-naphthyridine-3-carboxamide,
2-amino-1-ethyl-7-{4-[2-({[(2R)-1-ethylpyrrolidin-2-yl]methyl}amino)-2-oxoethyl]phenyl}-N-methyl-4-oxo-1,4-dihydro-1,8-naphthyridine-3-carboxamide,
2-amino-1-ethyl-7-[4-[2-({[(2S)-1-ethylpyrrolidin-2-yl]methyl}amino)-2-oxoethyl]phenyl}-N-methyl-4-oxo-1,4-dihydro-1,8-naphthyridine-3-carboxamide,
2-amino-1-ethyl-N-methyl-4-oxo-7-{4-[2-oxo-2-(pyrimidin-4-ylamino)ethyl]phenyl}-1,4-dihydro-1,8-naphthyridine-3-carboxamide,
2-amino-1-ethyl-N-methyl-4-oxo-7-{4-[2-oxo-2-(pyridin-4-ylamino)ethyl]phenyl}-1,4-dihydro-1,8-naphthyridine-3-carboxamide,
2-amino-1-ethyl-N-methyl-7-(4-{2-[(2-morpholin-4-ylethyl)amino]-2-oxoethyl}phenyl)-4-oxo-1,4-dihydro-1,8-naphthyridine-3-carboxamide,
2-amino-1-ethyl-N-methyl-4-oxo-7-{5-[2-oxo-2-(pyridin-2-ylamino)ethyl]pyridin-2-yl}-1,4-dihydro-1,8-naphthyridine-3-carboxamide,
2-amino-1-ethyl-N-methyl-7-{4-[1-methyl-2-oxo-2-(pyridin-2-ylamino)ethyl]phenyl}-4-oxo-1,4-dihydro-1,8-naphthyridine-3-carboxamide,
2-amino-1-ethyl-N-methyl-4-oxo-7-{6-[2-oxo-2-(pyridin-2-ylamino)ethyl]pyridin-3-yl}-1,4-dihydro-1,8-naphthyridine-3-carboxamide,
2-amino-1-ethyl-7-[4-(2-{[(4-ethylmorpholin-3-yl)methyl]amino}-2-oxoethyl)phenyl]-N-methyl-4-oxo-1,4-dihydro-1,8-naphthyridine-3-carboxamide,
2-amino-1-ethyl-7-[6-(2-{[(4-ethylmorpholin-3-yl)methyl]amino}-2-oxoethyl)pyridin-3-yl]-N-methyl-4-oxo-1,4-dihydro-1,8-naphthyridine-3-carboxamide,
2-amino-1-ethyl-7-{4-[2-({[(2S)-1-ethyl-4,4-difluoropyrrolidin-2-yl]methyl}amino)-2-oxoethyl]phenyl}-N-methyl-4-oxo-1,4-dihydro-1,8-naphthyridine-3-carboxamide,
2-amino-1-ethyl-7-[4-(2-{[(4-ethylmorpholin-3-yl)methyl]amino}-1-methyl-2-oxoethyl)phenyl]-N-methyl-4-oxo-1,4-dihydro-1,8-naphthyridine-3-carboxamide,
2-amino-1-ethyl-7-{4-[2-({[(2S,4R)-1-ethyl-4-fluoropyrrolidin-2-yl]methyl}amino)-2-oxoethyl]phenyl}-N-methy-4-oxo-1,4-dihydro-1,8-naphthyridine-3-carboxamide,
2-amino-1-ethyl-7-{4-[2-({[(2R)-1-(2-fluoroethyl)pyrrolidin-2-yl]methyl}amino)-2-oxoethyl]phenyl}-N-methyl-4-oxo-1,4-dihydro-1,8-naphthyridine-3-carboxamide,
2-amino-1-ethyl-N-methyl-7-{4-[2-({[(2R)-1-(methylsulfonyl)pyrrolidin-2-yl]methyl}amino)-2-oxoethyl]phenyl}-4-oxo-1,4-dihydro-1,8-naphthyridine-3-carboxamide,
2-amino-1-ethyl-7-{5-[2-({[(2R)-1-(2-fluoroethyl)pyrrolidin-2-yl]methyl}amino)-2-oxoethyl]pyridin-2-yl}-N-methyl-4-oxo-1,4-dihydro-1,8-naphthyridine-3-carboxamide,
2-amino-1-ethyl-N-methyl-4-oxo-7-{4-[2-oxo-2-({[(2R)-1-(2,2,2-trifluoroethyl)pyrrolidin-2-yl]methyl}amino)ethyl]phenyl}-1,4-dihydro-1,8-naphthyridine-3-carboxamide,
2-amino-7-{4-[2-({[(2R)-1-(2,2-difluoroethyl)pyrrolidin-2-yl]methyl}amino)-2-oxoethyl]phenyl}-1-ethyl-N-methyl-4-oxo-1,4-dihydro-1,8-naphthyridine-3-ca rboxam ide,
2-amino-1-ethyl-N-methyl-7-{4-[2-({[4-(1-methylethyl)morpholin-3-yl]methyl}amino)-2-oxoethyl]phenyl}-4-oxo-1,4-dihydro-1,8-naphthyridine-3-carboxamide,
2-amino-7-[4-(2-{[(4-cyclopropylmorpholin-3-yl)methyl]amino}-2-oxoethyl)phenyl]-1-ethyl-N-methyl-4-oxo-1,4-dihydro-1,8-naphthyridine-3-carboxamide,
2-amino-7-{5-[2-({[(2R)-1-(2,2-dfluoroethyl)pyrrolidin-2-yl]methyl}amino)-2-oxoethyypyridin-2-yl}-1-ethyl-N-methyl-4-oxo-1,4-dihydro-1,8-naphthyridine-3-carboxamide,
2-amino-1-ethyl-N-methyl-4-oxo-7-{4-[2-oxo-2-(1,3-thiazol-2-ylamino)ethyl]phenyl}-1,4-dihydro-1,8-naphthyridine-3-carboxamide,
2-amino-1-ethyl-N-methyl-7-[4-(2-{[(1-methyl-1H-imidazol-5-yl)methyl]amino}-2-oxoethyl)phenyl]-4-oxo-1,4-dihydro-1,8-naphthyridine-3-carboxamide,
2-amino-1-ethyl-N-methyl-4-oxo-7-(4-{2-oxo-2-[(2-pyridin-3-ylethyl)amino]ethyl}phenyl)-1,4-dihydro-1,8-naphthyridine-3-carboxamide,
2-amino-1-ethyl-N-methyl-7-(4-{2-[(3-morpholin-4-ylpropyl)amino]-2-oxoethyl}phenyl)-4-oxo-1,4-dihydro-1,8-naphthyridine-3-carboxamide,
2-amino-1-ethyl-N-methyl-4-oxo-7-(4-{2-oxo-2-[(2-pyridin-2-ylethyl)amino]ethyl}phenyl)1,4-dihydro-1,8-naphthyridine-3-carboxamide,
2-amino-1-ethyl-N-methyl-4-oxo-7-(4-{2-oxo-2-[(2-phenylethyl)amino]ethyl}phenyl)-1,4-dihydro-1,8-naphthyridine-3-carboxamide,
2-amino-1-ethyl-N-methyl-4-oxo-7-(4-{2-oxo-2-[(3-phenylpropyl)amino]ethyl}phenyl)-1,4-dihydro-1,8-naphthyridine-3-carboxamide,
2-amino-1-ethyl-N-methyl-7-[4(2-{[2-(1-methyl-1H-pyrrol-2-yl)ethyl]amino}-2-oxoethyl)phenyl]-4-oxo-1,4-dihydro-1,8-naphthyridine-3-carboxamide,
2-amino-1-ethyl-7-[4-(2-{[(1-ethylpyrrolidin-2-yl)methyl]amino}-2-oxoethyl)-1,3-thiazol-2-yl]-N-methyl-4-oxo-1,4-dihydro-1,8-naphthyridine-3-carboxamide,
2-amino-1-(3-methoxypropyl)-N-methyl-4-oxo-7-{4-[2-oxo-2-(pyridin-2-ylamino)ethyl]phenyl}-1,4-dihydro-1,8-naphthyridine-3-carboxamide,
2-amino-7-[4-(2-{[1-(2,2-difiuoroethyl)pyrrolidin-3-yl]amina}-2-oxoethyl)phenyl]-1-ethyl-N-methyl-4-oxo-1,4-dihydro-1,8-naphthyridine-3-carboxamide,
2-amino-1-ethyl-7-[4-(3-{[(1-ethylpyrrolidin-2-yl)meth yl]amino}-3-oxopropyl)phenyl]-N-methyl-4-oxo-1,4-dlhydro-1,8-naphthyrldine-3-carboxamide,
2-amino-1-ethyl-7-[4-(4-{[(1-ethylpyrrolidin-2-yl)methyl]amino}-4-oxobutyl)phenyl]-N-methyl-4-oxo-1,4-dihydro-1,8-naphthyridine-3-carboxamide,
2-amino-1-ethyl-7-[4-(2-{[(1-ethypyrrolidin-2-yl)methyl]amino}-2-oxoethyl)phenyl]-4-oxo-N-propyl-1,4-dihydro-1,8-naphthyridine-3-carboxamide,
2-amino-7-[4-(2-{[(1-ethylpyrrolidin-2-yl)methyl]amino}-2-oxoethyl)phenyl]-N-methyl-4-oxo-1-(2,2,2-trifluoroethyl)-1,4-dihydro-1,8-naphthyridine-3-carboxamide,
2-amino-1-cyclopentyl-7-[4-(2-{[(1-ethylpyrrolidin-2-yl)methyl]amino}-2-oxoethyl)phenyl]-*N*-methyl-4-oxo-1,4-dihydro-1,8-naphthyridine-3-carboxamide,
2-amino-1-ethyl-7-[4-(5-{[(1-ethylpyrrolidin-2-yl)methyl]amino}-5-oxopentyl)phenyl]-*N*-methyl-4-oxo-1,4-dihydro-1,8-naphthyridine-3-carboxamide,
1-ethyl-7-[5-(2-{[(1-ethylpyrrolidin-2-yl)methyl]amino}-2-oxoethyl)thiophen-2-yl]-*N*,2-dimethyl-4-oxo-1,4-dihydro-1,8-naphthyridine-3-carboxamide,
2-amino-7-{4-[2-({[(2R)-1-ethylpyrrolidin-2-yl]methyl}amino)-2-oxoethyl]phenyl}-N-methyl-1-(2-methylpropyl)-4-oxo-1,4-dihydro-1,8-naphthyridine-3-carboxamide,
2-amino-1-ethyl-7-{4-[1-({[(2R)-1-ethylpyrrolidin-2-yl]methyl}carbamoyl)cyclopropyl]phenyl}-N-methyl-4-oxo-1,4-dihydro-1,8-naphthyridine-3-carboxamide,
2-amino-1-ethyl-7-{2-[2-({[(2R)-1-ethylpyrrolidin-2-yl]methyl}amino)-2-oxoethyl]-1,3-thiazol-4-yl}-N-methyl-4-oxo-1,4-dihydro-1,8-naphthyridine-3-carboxamide,
2-amino-1-ethyl-7-{4-[2-({[(2R)-1-ethylpyrrolidin-2-yl]methyl}amino)-2-oxoethyl]-2-fluorophenyl}-N-methyl-4-oxo-1,4-dihydro-1,8-naphthyridine-3-carboxamide,
2-amino-7-[3-chloro-4-(2-{[{1-ethylpyrrolidin-2-yl)methyl]amino}-2-oxoethyl)phenyl]-1-ethyl-N-methyl-4-oxo-1,4-dihydro-1,8-naphthyridine-3-carboxamide,
2-amino-7-[3-fluoro-4-(2-{[(1-ethylpyrrolidin-2-yl)methyl]amino}-2-oxoethyl)phenyl]-1-ethyl-N-methyl-4-oxo-1,4-dihydro-1,8-naphthyridine-3-carboxamide,
2-amino-7-[3-methyl-4-(2-{[(1-ethylpyrrolidin-2-yl)methyl]amino}-2-oxoethyl)phenyl]-1-ethyl-N-methyl-4-oxo-1,4-dihydro-1,8-naphthyridine-3-carboxamide,
2-amino-1-(cyclopropylmethyl)-7-[4-(2-{[(1-ethylpyrrolidin-2-yl)methyl]amino}-2-oxoethyl)phenyl]-N-methyl-4-oxo-1,4-dihydro-1,8-naphthyridine-3-carboxamide,
2-amino-1-ethyl-7-[4-(2-{[(1-ethylpyrrolidin-2-yl)methyl]amino}-2-oxoethyl)-2-methylphenyl]-*N*-methyl-4-oxo-1,4-dihydro-1,8-naphthyridine-3-carboxamide,
2-amino-1-ethyl-7-[3-(2-{[(1-ethylpyrrolidin-2-yl)methyl]amino}-2-oxoethyl)phenyl]-N-methyl-4-oxo-1,4-dihydro-1,8-naphthyridine-3-carboxamide,
2-amino-1-ethyl-N-methyl-4-oxo-7-{3-[2-oxo-2-(pyridin-2-ylamino)ethyl]phenyl}1,4-dihydro-1,8-naphthyridine-3-carboxamide,
2-amino-1-ethyl-*N*-methyl-4-oxo-7-{4-[2-(pyridin-2-ylamino)ethyl]phenyl}-1,4-dihydro-1,8-naphthyridine-3-carboxamide,
à l'état de base ou de sel d'addition à un acide, à l'état de solvat, ainsi que ses énentiomères et diastéréoisomères, y compris ses mélanges.

8. Procédé de préparation d'un composé de formule (I) selon l'une quelconque des revendications 1 à 7, **caractérisé en ce que** l'on fait réagir un composé de formule (XI) : 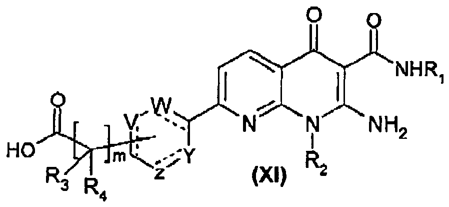 avec un composé de formule HNR5R6, en présence d'un agent de couplage et d'une base,
R1, R2, R3, R4, R5, R6, V, W, Y, Z, m étant tels que définis dans la revendication 1.

9. Procédé de préparation d'un composé de formule (I) selon l'une quelconque des revendications 1 à 7, **caractérisé en ce que** l'on fait réagir un composé de formule (VII) : 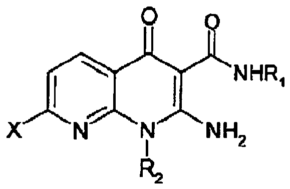 avec un composé de formule (IXa) 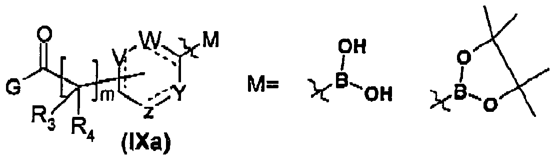 où X représente un atome d'halogène,
G représente un groupe (C1-C4)alcoxy ou un groupe -NR5R6, et
R1, R2, R3, R4, R5, R6, V, W, Y, Z, m étant tels que définis dans la revendication 1.

10. Procédé de préparation d'un composé de formule (i) selon l'une quelconque des revendications 1 à 7, **caractérisé en ce que** l'on fait réagir un composé de formule (VII) : 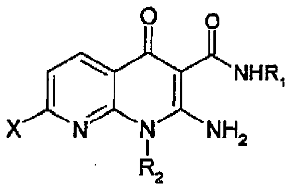 avec un composé de formule (IXb) : 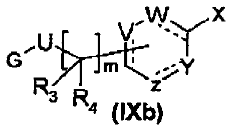 où G représente un groupe (C1-C4)alcoxy ou un groupe -NR5R6, X représente un atome d'halogène et R1, R2, R3, R4, R5, R6, V, W, Y, Z, m étant tels que définis dans la revendication 1.

11. Médicament, **caractérisé en ce qu'**il comprend un composé de formule (I) selon l'une quelconque des revendications 1 à 7, ou un sel d'addition de ce composé à un acide pharmaceutiquement acceptable, ou encore un solvat du composé de formule (I).

12. Composition pharmaceutique, **caractérisée en ce qu'**elle comprend un composé de formule (I) selon l'une quelconque des revendications 1 à 7, ou un sel pharmaceutiquement acceptable, un solvat de ce composé, ainsi qu'au moins un excipient pharmaceutiquement acceptable.

13. Utilisation d'un composé de formule (I) selon l'une quelconque des revendications 1 à 7 pour la préparation d'un médicament destiné au traitement des maladies prolifératives.

14. Utilisation selon la revendication 13 pour laquelle les maladies prolifératives sont les cancers à tumeurs liquides, les leucémies chroniques ou algués, les lymphomes lymphocytaires, la maladie de Hadgkin, et les syndromes myeloproliferatifs, et les syndromes myelodysplasiques.

15. Utilisation d'un composé de formule (I) selon l'une quelconque des revendications 1 à 7 pour la préparation d'un médicament destiné au traitement des maladies prolifératives.

16. Utilisation selon la revendication 15 pour laquelle les maladies prolifératives sont les cancers à tumeurs solides par exemple cancers du poumon (NSCLC), des os, du pancréas, de la peau, syndrome de Kaposi, les mélanomes intraoculaires, les cancers du sein, de l'utérus, du col de l'utérus, des ovaires, de l'endomètre, du vagin, de la vulve, de l'urètre, du pénis, de la prostate, les carcinomes des trompes de Fallope, les cancers tel que les GIST et de la région anale, du rectum, de l'intestin grêle, du colon, de l'estomac, de l'oesophage, des glandes endocrines, thyroïde, parathyroïdes ou surrénales, les sarcomes des tissus mous, sarcomes d'Ewing, des ostésarcomes, dermatofibrosarcome et autres fibrosarcomes, les cancers de la vessie ou du rein, les néoplasmes du système nerveux central, les tumeurs de la colonne vertébrale et desmoïdes, les gliomes du tronc cérébral et glioblastomes, les adénomes pituitaires et leurs métastases

17. Utilisation d'un composé de formule (I) selon l'une quelconque des revendications 1 à 7 pour la préparation d'un médicament destiné au traitement de maladies prolifératives non-malignes.

18. Utilisation selon la revendication 17 pour laquelle les maladies prolifératives non-malignes sont la resténose, l'athérosclérose, la thrombose, l'insuffisance cardiaque, l'hypertrophie cardiaque, hypertension artérielle pulmonaire, la fibrose, la néphropathie diabétique, la glomérulonéphrite, la pyélonéphrite chronique, les hemanglomes, les maladies auto-immunes telles que le psoriasis, les sclérodermatites, l'Immunosuppression.

## Patentansprüche

1. Verbindung der Formel (I) 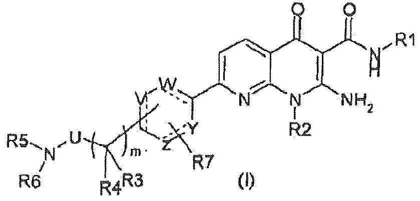 **R1** für eine (C1-C4)-Alkylgruppe steht,
**R2** für - (CH₂)ₙ'-**B** steht, wobei n' = 0, 1, 2, 3, 4 und B für eine (C3-C5)-Cycloalkylgruppe oder eine (C1-C4)-Alkylgruppe, die gegebenenfalls durch ein oder mehrere Fluoratome oder eine (C1-C4)-Alkoxygruppe substituiert ist, steht;
**U** für eine Carbonylgruppe oder eine -CH₂-Gruppe steht,
**Y, Z, V und W** unabhängig voneinander für eine -CH-Gruppe oder ein Kohlenstoffatom, das gegebenenfalls durch eine Gruppe R7 substituiert ist, oder ein Heteroatom oder kein Atom stehen, mit der Maßgabe, dass der Ring aromatisch und 5-oder 6-gliedrig sein muss,
**R3, R4** unabhängig voneinander für ein Wasserstoffatom oder eine lineare (C1-C4)-Alkylgruppe stehen oder R3 und R4 zusammen mit dem Kohlenstoff, an den sie gebunden sind, eine (C3-C5)-Cycloalkylgruppe bilden,
**m** für eine ganze Zahl mit einem Wert von 1, 2, 3, 4 steht,
**R5** für ein Wasserstoffatom oder eine (C1-C4)-Alkylgruppe steht,
**R6** für - (CH₂)ₙ-**L** steht, wobei n = 0, 1, 2, 3 und **L** für eine Gruppe steht, die unabhängig aus den folgenden Gruppen ausgewählt ist:
○ einer (C1-C5)-Alkylgruppe, die gegebenenfalls durch eine (C1-C4)-Alkoxygruppe substituiert ist,
○ einer (C3-C5)-Cycloalkylgruppe,
○ einem Aryl mit 6 Kohlenstoffatomen, das gegebenenfalls durch ein oder mehrere Halogenatome substituiert ist,
○ einem 5- oder 6-gliedrigen Heteroaryl mit mindestens einem aus Stickstoff- oder Schwefelatom ausgewählten Heteroatom, das gegebenenfalls durch eine (C1-C4)-Alkylgruppe substituiert ist,
○ einem gesättigten Heterocyclus, wobei der Heterocyclus 4- bis 7-gliedrig ist, mindestens ein aus einem Stickstoffatom und einem Sauerstoffatom ausgewähltes Heteroatom enthält und gegebenenfalls in beliebigen Positionen, das Stickstoffatom eingeschlossen, durch einen oder mehrere Substituenten, die aus einem Fluoratom, einer (C1-C4)-Fluoralkylgruppe, einer linearen oder verzweigten (C1-C4)-Alkylgruppe, einer (C3-C5)-Cycloalkylgruppe und einer (C1-C4)-Alkyl-sulfonamidgruppe ausgewählt sind, substituiert ist, wobei die absolute Konfiguration eines substituierten Kohlenstoffs an dem Heterocyclus R oder S oder racemisch sein kann,
**R7** für ein Wasserstoffatom oder eine (C1-C4)-Alkylgruppe oder ein Halogenatom steht,
in Basen- oder Säureadditionssalzform oder Solvatform sowie Enantiomere oder Diastereoisomere davon einschließlich Gemischen davon.

2. Verbindung der Formel (I) nach Anspruch 1, **dadurch gekennzeichnet, dass**
**R1** für eine (C1-C4)-Alkylgruppe steht,
und/oder
**R2** für - (CH₂)ₙ,-**B** steht, wobei n' = 0, 1 und B für eine (C3-C5)-Cycloalkylgruppe oder eine (C1-C4)-Alkylgruppe, die gegebenenfalls durch ein oder mehrere Fluoratome oder eine (C1-C4)-Alkoxygruppe substituiert ist, steht;
und/oder
U für eine Carbonylgruppe oder eine -CH₂-Gruppe steht,
und/oder
**Y, Z, V und W** unabhängig voneinander für eine -CH-Gruppe oder ein Kohlenstoffatom, das gegebenenfalls durch eine Gruppe R7 substituiert ist, oder ein Heteroatom oder kein Atom stehen, mit der Maßgabe, dass der Ring aromatisch und 5-oder 6-gliedrig sein muss,
und/oder
**R3, R4** unabhängig voneinander für ein Wasserstoffatom oder eine lineare (C1-C4)-Alkylgruppe stehen oder R3 und R4 zusammen mit dem Kohlenstoff, an den sie gebunden sind, eine (C3-C5)-Cycloalkylgruppe bilden,
und/oder
**m** für eine ganze Zahl mit einem Wert von 1, 2, 3, 4 steht,
und/oder
**R5** für ein Wasserstoffatom oder eine (C1-C4)-Alkylgruppe steht,
und/oder
**R6** für -(CH₂)ₙ-**L** steht, wobei n = 0, 1, 2, 3 und **L** für eine Gruppe steht, die unabhängig aus den folgenden Gruppen ausgewählt ist:
○ einer (C1-C5)-Alkylgruppe, die gegebenenfalls durch eine (C1-C4)-Alkoxygruppe substituiert ist,
○ einer (C3-C5)-Cycloalkylgruppe,
○ einem Aryl mit 6 Kohlenstoffatomen, das gegebenenfalls durch einen oder mehrere Halogenatome substituiert ist,
○ einem 5- oder 6-gliedrigen Heteroaryl mit mindestens einem aus Stickstoff- oder Schwefelatom ausgewählten Heteroatom, das gegebenenfalls durch eine (C1-C4)-Alkylgruppe substituiert ist,
○ einem gesättigten Heterocyclus, wobei der Heterocyclus 5- bis 7-gliedrig ist, mindestens ein aus einem Stickstoffatom und einem Sauerstoffatom ausgewähltes Heteroatom enthält und gegebenenfalls in beliebigen Positionen, das Stickstoffatom eingeschlossen, durch einen oder mehrere Substituenten, die aus einem Fluoratom, einer (C1-C4)-Fluoralkylgruppe, einer linearen oder verzweigten (C1-C4)-Alkylgruppe, einer (C3-C5)-Cycloalkylgruppe und einer (C1-C4)-Alkylsulfonamidgruppe ausgewählt sind, substituiert ist, wobei die absolute Konfiguration eines substituierten Kohlenstoffs an dem Heterocyclus R oder S oder racemisch sein kann,
und/oder
**R7** für ein Wasserstoffatom oder eine (C1-C4)-Alkylgruppe oder ein Halogenatom steht,
in Basen- oder Säureadditionssalzform oder Solvatform sowie Enantiomere oder Diastereoisomere davon einschließlich Gemischen davon.

3. Verbindung der Formel (I) nach Anspruch 1, **dadurch gekennzeichnet, dass U** für eine Carbonylgruppe steht.

4. Verbindung der Formel (I) nach Anspruch 1, dadurch gekenntzeichnet, dass der **Y, Z, V und W** enthaltende Ring aus Phenyl-, Pyridin-, Thiazol- und Thiophen-gruppen ausgewählt ist.

5. Verbindung der Formel (I) nach Anspruch 1, **dadurch gekennzeichnet, dass R3** und/oder **R4** und/oder **R5** für ein Wasserstoffatom stehen.

6. Verbindung der Formel (I) nach Anspruch 1, **dadurch gekennzeichnet, dass** die Kette -[C(R3R4)]ₘ-U-N(R5) (R6) in para- oder meta-Position zu dem Ring, an den sie gebunden ist, steht.

7. Verbindung der Formel (I) nach Anspruch 1, **dadurch gekennzeichnet, dass** sie aus den folgenden Verbindungen ausgewählt ist:
2-Amino-1-ethyl-N-methyl-4-oxo-7-{4-[2-oxo-2-(phenylamino)ethyllphenyll-1,4-dihydro-1,8-naphthyridin-3-carboxamid,
2-Amino-1-ethyl-7-[4-(2-{[(1-ethylpyrrolidin-2-yl)methyl](methyl)amino}-2-oxoethyl)phenyl]-N-methyl-4-oxo-1,4-dihydro-1,8-naphthyridin-3-carboxamid,
2-Amino-1-ethyl-N-methyl-4-oxo-7-{4-[2-oxo-2-(pyridin-3-ylamino)ethyl]phenyl}-1,4-dihydro-1,8-naphthyridin-3-carboxamid,
2-Amino-1-ethyl-N-methyl-4-oxo-7-{4-[2-oxo-2-(pyridin-2-ylamino)ethyl]phenyl}-1,4-dihydro-1,8-naphthyridin-3-carboxamid,
2-Amino-7-(4-{2-[(2-chlorphenyl)amino]-2-oxoethyl}phenyl)-1-ethyl-N-methyl-4-oxo-1,4-dihydro-1,8-naphthyridin-3-carboxamid,
2-Amino-7-(4-{2-[(3,5-difluorphenyl)amino]-2-oxoethyl}phenyl)-1-ethyl-N-methyl-4-oxo-1,4-dihydro-1,8-naphthyridin-3-carboxamid,
2-Amino-1-ethyl-N-methyl-4-oxo-7-(4-{2-oxo-2-[(pyridin-4-ylmethyl)amino]ethyl}phenyl)-1,4-dihydro-1,8-naphthyridin-3-carboxamid,
2-Amino-1-ethyl-N-methyl-4-oxo-7-(4-{2-oxo-2-[(pyridin-2-ylmethyl)amino]ethyl}phenyl)-1,4-dihydro-1,8-naphthyridin-3-carboxamid,
2-Amino1-ethyl-7-(4-{2-[(2-methoxyethyl)amino]-2-oxoethyl}phenyl)-N-methyl-4-oxo-1,4-dihydro-1,8-naphthyridin-3-carboxamid,
2-Amino-7-{4-[2-(cyclopropylamino)-2-oxoethyl]-phenyl}-1-ethyl-N-methyl-4-oxo-1,4-dihydro-1,8-naphthyridin-3-carboxamid,
2-Amino-1-ethyl-N-methyl-7-(4-{2-[(1-methylethyl)amino]-2-oxoethyl}phenyl)-4-oxo-1,4-dihydro-1,8-naphthyridin-3-carboxamid,
2-Amino-7-{4-[2-(cyclopentylamino)-2-oxoethyl]-phenyl}-1-ethyl-N-methyl-4-oxo-1,4-dihydro-1,8-naphthyridin-3-carboxamid,
2-Amino-1-ethyl-N-methyl-4-oxo-7-{4-[2-oxo-2-(pyrazin-2-ylamino)ethyl]phenyl}-1,4-dihydro-1,8-naphthyridin-3-carboxamid,
2-Amino-1-ethyl-7-{4-[2-({[(2R)-1-ethylpyrrolidin-2-yl]methyl}amino)-2-oxoethyl]phenyl}-N-methyl-4-oxo-1,4-dihydro-1,8-naphthyridin-3-carboxamid,
2-Amino-1-ethyl-7-{4-[2-({[(2S)-1-ethylpyrrolidin-2-yl]methyl}amino)-2-oxoethyl]phenyl}-N-methyl-4-oxo-1,4-dihydro-1,8-naphthyridin-3-carboxamid,
2-Amino-1-ethyl-N-methyl-4-oxo-7-{4-[2-oxo-2-(pyrimidin-4-ylamino)ethyl]phenyl}-1,4-dihydro-1,8-naphthyridin-3-carboxamid,
2-Amino-1-ethyl-N-methyl-4-oxo-7-{4-[2-oxo-2-(pyridin-4-ylamino)ethyl]phenyl}-1,4-dihydro-1,8-naphthyridin-3-carboxamid,
2-Amino-1-ethyl-N-methyl-7-(4-{2-[(2-morpholin-4-ylethyl)amino]-2-oxoethyl}phenyl)-4-oxo-1,4-dihydro-1,8-naphthyridin-3-carboxamid,
2-Amino-1-ethyl-N-methyl-4-oxo-7-{5-[2-oxo-2-(pyridin-2-ylamino)ethyl]pyridin-2-yl}-1,4-dihydro-1,8-naphthyridin-3-carboxamid,
2-Amino-1-ethyl-N-methyl-7-{4-[1-methyl-2-oxo-2-(épyridin-2-ylamino)ethyl]phenyl}-4-oxo-1,4-dihydro-1,8-naphthyridin-3-carboxamid,
2-Amino-1-ethyl-N-methyl-4-oxo-7-{6-[2-oxo-2-(pyridin-2-ylamino)ethyl]pyridin-3-yl}-1,4-dihydro-1,8-naphthyridin-3-carboxamid,
2-Amino-1-ethyl-7-[4-(2-{[(4-ethylmorpholin-3-yl)methyl]amino}-2-oxoethyl)phenyl]-N-methyl-4-oxo-1,4-dihydro-1,8-naphthyridin-3-carboxamid,
2-Amino-1-ethyl-7-[6-(2-{[(4-ethylmorpholin-3-yl)methyl]amino}-2-oxoethyl)pyridin-3-yl]-N-methyl-4-oxo-1,4-dihydro-1,8-naphthyridin-3-carboxamid,
2-Amino-1-ethyl-7-{4-[2-({[(2S)-1-ethyl-4,4-difluorpyrrolidin-2-yl]methyl}amino)-2-oxoethyl]-phenyl}-N-methyl-4-oxo-1,4-dihydro-1,8-naphthyridin-3-carboxamid,
2-Amino-1-ethyl-7-[4-(2-{[(4-ethylmorpholin-3-yl)methyl]amino}-1-methyl-2-oxoethyl)phenyl]-N-methyl-4-oxo-1,4-dihydro-1,8-naphthyridin-3-carboxamid,
2-Amino-1-ethyl-7-{4-[2-({[(2S,4R)-1-ethyl-4-fluorpyrrolidin-2-yl]methyl}amino)-2-oxoethyl]-phenyl}-N-methyl-4-oxo-1,4-dihydro-1,8-naphthyridin-3-carboxamid,
2-Amino-1-ethyl-7-{4-[2-({[(2R)-1-(2-fluorethyl)-pyrrolidin-2-yl]methyl}amino)-2-oxoethyl]phenyl}-N-methyl-4-oxo-1,4-dihydro-1,8-naphthyridin-3-carboxamid,
2-Amino-1-ethyl-N-methyl-7-{4-[2-({[(2R)-1-(methylsulfonyl)pyrrolidin-2-yl]methyl}amino)-2-oxoethyl]phenyl}-4-oxo-1,4-dihydro-1,8-naphthyridin-3-carboxamid,
2-Amino-1-ethyl-7-{5-[2-({[(2R)-1-(2-fluorethyl)-pyrrolidin-2-yl]methyl}amino)-2-oxoethyl]pyridin-2-yl}-N-methyl-4-oxo-1,4-dihydro-1,8-naphthyridin-3-carboxamid,
2-Amino-1-ethyl-N-methyl-4-oxo-7-{4-[2-oxo-2-({[(2R)-1-(2,2,2-trifluorethyl)pyrrolidin-2-yl]-methyl}amino)ethyl]phenyl}-1,4-dihydro-1,8-naphthyridin-3-carboxamid,
2-Amino-7-{4-[2-({[(2R)-1-(2,2-difluorethyl)-pyrrolidin-2-yl]methyl}amino)-2-oxoethyl]phenyl}-1-ethyl-N-methyl-4-oxo-1,4-dihydro-1,8-naphthyridin-3-carboxamid,
2-Amino-1-ethyl-N-methyl-7-{4-[2-({[4-(1-methylethyl)morpholin-3-yl]methyl}amino)-2-oxoethyl]phenyl}-4-oxo-1,4-dihydro-1,8-naphthyridin-3-carboxaznid,
2-Amino-7-[4-(2-{[(4-cyclopropylmorpholin-3-yl)methyl]amino}-2-oxoethyl)phenyl]-1-ethyl-N-methyl-4-oxo-1,4-dihydro-1,8-naphthyridin-3-carboxamid,
2-Amino-7-{5-[2-({[(2R)-1-(2,2-difluorethyl)-pyrrolidin-2-yl]methyl}amino)-2-oxoethyl]pyridin-2-yl}-1-ethyl-N-methyl-4-oxo-1,4-dihydro-1,8-naphthyridin-3-carboxamid,
2-Amino-1-ethyl-N-methyl-4-oxo-7-{4-[2-oxo-2-(1,3-thiazol-2-ylamino)ethyl]phenyl}-1,4-dihydro-1,8-naphthyridin-3-carboxamid,
2-Amino-1-ethyl-N-methyl-7-[4-(2-{[(1-methyl-1H-imidazol-5-yl)methyl]amino}-2-oxoethyl)phenyl]-4-oxo-1,4-dihydro-1,8-naphthyridin-3-carboxamid,
2-Amino-1-ethyl-N-methyl-4-oxo-7-(4-{2-oxo-2-[(2-pyridin-3-ylethyl)amino]ethyl}phenyl)-1,4-dihydro-1,8-naphthyridin-3-carboxamid,
2-Amino-1-ethyl-N-methyl-7-(4-{2-[(3-morpholin-4-ylpropyl)amino]-2-oxoethyl}phenyl)-4-oxo-1,4-dihydro-1,8-naphthyridin-3-carboxamid,
2-Amino-1-ethyl-N-methyl-4-oxo-7-(4-{2-oxo-2-[(2-pyridin-2-ylethyl)amino]ethyl}phenyl)-1,4-dihydro-1,8-naphthyridin-3-carboxamid,
2-Amino-1-ethyl-N-methyl-4-oxo-7-(4-{2-oxo-2-[(2-phenylethyl)amino]ethyl}phenyl)-1,4-dihydro-1,8-naphthyridin-3-carboxamid,
2-Amino-1-ethyl-N-methyl-4-oxo-7-(4-{2-oxo-2-[(3-phenylpropyl)amino]ethyl}phenyl)-1,4-dihydro-1,8-naphthyridin-3-carboxamid,
2-Amino-1-ethyl-N-methyl-7-[4-(2-{[2-(1-methyl-1H-pyrrol-2-yl)ethyl]amino}-2-oxoethyl)phenyl]-4-oxo-1,4-dihydro-1,8-naphthyridin-3-carboxamid,
2-Amino-1-ethyl-7-[4-(2-{[(1-ethylpyrrolidin-2-yl)methyl]amino}-2-oxoethyl)-1,3-thiazol-2-yl]-N-methyl-4-oxo-1,4-dihydro-1,8-naphthyridin-3-carboxamid,
2-Amino-1-(3-methoxypropyl)-N-methyl-4-oxo-7-{4-[2-oxo-2-(pyridin-2-ylamino)ethyl]phenyl}-1,4-dihydro-1,8-naphthyridin-3-carboxamid, 2-Amino-7-[4-(2-{[1-(2,2-difluorethyl)pyrrolidin-3-yl]amino}-2-oxoethyl)phenyl]-1-ethyl-N-methyl-4-oxo-1,4-dihydro-1,8-naphthyridin-3-carboxamid,
2-Amino-1-ethyl-7-[4-(3-{[(1-ethylpyrrolidin-2-yl)methyl]amino}-3-oxopropyl)phenyl]-N-methyl-4-oxo-1,4-dihydro-1,8-naphthyridin-3-carboxamid,
2-Amino-1-ethyl-7-[4-(4-{[(1-ethylpyrrolidin-2-yl)methyl]amino}-4-oxobutyl)phenyl]-N-methyl-4-oxo-1,4-dihydro-1,8-naphthyridin-3-carboxamid,
2-Amino-1-ethyl-7-[4-(2-{[(1-ethylpyrrolidin-2-yl)methyl]amino}-2-oxoethyl)phenyl]-4-oxo-N-propyl-1,4-dihydro-1,8-naphthyridin-3-carboxamid,
2-Amino-7-[4-(2-{[(1-ethylpyrrolidin-2-yl)methyl]amino}-2-oxoethyl)phenyl]-*N*-methyl-4-oxo-1-(2,2,2-trifluorethyl)-1,4-dihydro-1,8-naphthyridin-3-carboxamid,
2-Amino-1-cyclopentyl-7-[4-(2-{[(1-ethylpyrrolidin-2-yl)methyl]amino}-2-oxoethyl)-phenyl]-*N*-methyl-4-oxo-1,4-dihydro-1,8-naphthyridin-3-carboxamid,
2-Amino-1-ethyl-7-[4-(5-{[(1-ethylpyrrolidin-2-yl)methyl]amino}-5-oxopentyl)phenyl]-*N*-methyl-4-oxo-1,4-dihydro-1,8-naphthyridin-3-carboxamid,
1-Ethyl-7-[5-(2-{[(1-ethylpyrrolidin-2-yl)methyl]amino}-2-oxoethyl)thiophen-2-yl]-*N*,2-dimethyl-4-oxo-1,4-dihydro-1,8-naphthyridin-3-carboxamid,
2-Amino-7-{4-[2-({[(2R)-2-ethylpyrrolidin-2-yl]-methyl}amino)-2-oxoethyl]phenyl}-N-methyl-1-(2-methylpropyl)-4-oxo-1,4-dihydro-1,8-naphthyridin-3-carboxamid,
2-Amino-1-ethyl-7-{4-[1-({[(2R)-1-ethylpyrrolidin-2-yl]methyl}carbamoyl)cyclopropyl]phenyl}-N-methyl-4-oxo-1,4-dihydro-1,8-naphthyridin-3-carboxamid,
2-Amino-1-ethyl-7-{2-[2-({[(2R)-1-ethylpyrrolidin-2-yl]methyl}amino)-2-oxoethyl]-1,3-thiazol-4-yl}-N-methyl-4-oxo-1,4-dihydro-1,8-naphthyridin-3-carboxamid,
2-Amino-1-ethyl-7-{4-[2-({[(2R)-1-ethylpyrrolidin-2-yl]methyl}amino)-2-oxoethyl]-2-fluorphenyl}-N-methyl-4-oxo-1,4-dihydro-1,8-naphthyridin-3-carboxamid,
2-Amino-7-[3-chlor-4-(2-{[(1-ethylpyrrolidin-2-yl)methyl]amino}-2-oxoethyl)phenyl]-1-ethyl-N-methyl-4-oxo-1,4-dihydro-1,8-naphthyridin-3-carboxamid,
2-Amino-7-[3-fluor-4-(2-{[(1-ethylpyrrolidin-2-yl)methyl]amino}-2-oxoethyl)phenyl]-1-ethyl-N-methyl-4-oxo-2,4-dihydro-2,8-naphthyridin-3-carboxamid,
2-Amino-7-[3-methyl-4-(2-{[(1-ethylpyrrolidin-2-yl)methyl]amino}-2-oxoethyl)phenyl]-1-ethyl-N-methyl-4-oxo-1,4-dihydro-1,8-naphthyridin-3-carboxamid,
2-Amino-1-(cyclopropylmethyl)-7-[4-(2-{[(1-ethylpyrrolidin-2-yl)methyl]amino}-2-oxoethyl)-phenyl]-N-methyl-4-oxo-1,4-dihydro-1,8-naphthyridin-3-carboxamid,
2-Amino-1-ethyl-7-[4-(2-{[(1-ethylpyrrolidin-2-yl)methyl]amino}-2-oxoethyl)-2-methylphenyl]-*N-*methyl-4-oxo-1,4-dihydro-1,8-naphthyridin-3-carboxamid,
2-Amino-1-ethyl-7-[3-(2-{[(1-ethylpyrrolidin-2-yl)methyl]amino}-2-oxoethyl)phenyl]-N-methyl-4-oxo-1,4-dihydro-1,8-naphthyridin-3-carboxamid,
2-Amino-1-ethyl-N-methyl-4-oxo-7-{3-[2-oxo-2-(pyridin-2-ylamino)ethyl]phenyl}-1,4-dihydro-1,8-naphthyridin-3-carboxamid,
2-Amino-1-ethyl-*N*-methyl-4-oxo-7-{4-[2-(pyridin-2-ylamino)ethyl]phenyl}-1,4-dihydro-1,8-naphthyridin-3-carboxamid,
in Basen- oder Säureadditionssalzform oder Solvat-form sowie Enantiomere oder Diastereoisomere davon einschließlich Gemischen davon.

8. Verfahren zur Herstellung einer Verbindung der Formel (I) nach einem der Ansprüche 1 bis 7, **dadurch gekennzeichnet, dass** man eine Verbindung der Formel (XI): 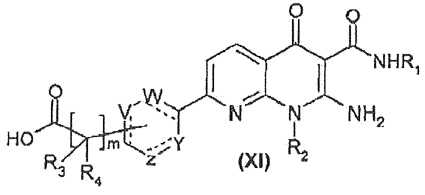 in Gegenwart eines Kupplungsmittels und einer Base mit einer Verbindung der Formel HNR5R6 umsetzt, wobei R1, R2, R3, R4, R5, R6, V, W, Y, Z und m die in Anspruch 1 angegebene Bedeutung besitzen.

9. Verfahren zur Herstellung einer Verbindung der Formel (I) nach einem der Ansprüche 1 bis 7, **dadurch gekennzeichnet, dass** man eine Verbindung der Formel (VII): 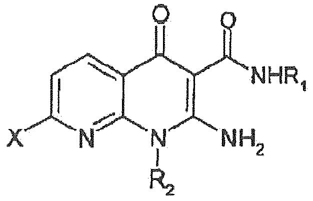 mit einer Verbindung der Formel (IXa) 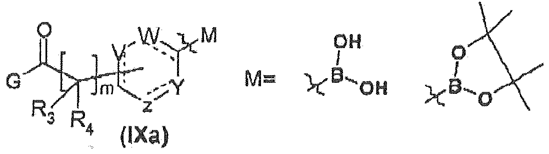 umsetzt,
wobei X für ein Halogenatom steht,
G für eine (C1-C4)-Alkoxygruppe oder eine Gruppe -NR5R6 steht und
R1, R2, R3, R4, R5, R6, V, W, Y, Z und m die in Anspruch 1 angegebene Bedeutung besitzen.

10. Verfahren zur Herstellung einer Verbindung der Formel (I) nach einem der Ansprüche 1 bis 7, **dadurch gekennzeichnet, dass** man eine Verbindung der Formel (VII): 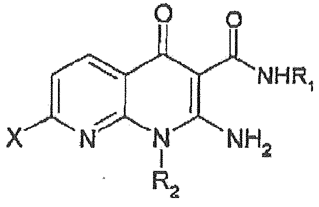 mit einer Verbindung der Formel (IXb) 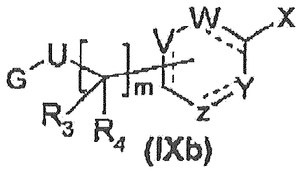 umsetzt,
wobei G für eine (C1-C4)-Alkoxygruppe oder eine Gruppe -NR5R6 steht, X für ein Halogenatom steht und R1, R2, R3, R4, R5, R6, V, W, Y, Z und m die in Anspruch 1 angegebene Bedeutung besitzen.

11. Medikament, **dadurch gekennzeichnet, dass** es eine Verbindung der Formel (I) nach einem der Ansprüche 1 bis 7 oder ein Additionssalz dieser Verbindung mit einer pharmazeutisch unbedenklichen Säure oder auch Solvat der Verbindung der Formel (I) umfasst.

12. Pharmazeutische Zusammensetzung, **dadurch gekennzeichnet, dass** sie eine Verbindung der Formel (I) nach einem der Ansprüche 1 bis 7 oder ein pharmazeutisch unbedenkliches Salz oder ein Solvat dieser Verbindung sowie mindestens einen pharmazeutisch unbedenklichen Hilfsstoff umfasst.

13. Verwendung einer Verbindung der Formel (I) nach einem der Ansprüche 1 bis 7 zur Herstellung eines Medikaments zur Behandlung von proliferativen Erkrankungen.

14. Verwendung nach Anspruch 13, für die es sich bei den proliferativen Erkrankungen um Krebserkrankungen mit flüssigen Tumoren, chronische oder akute Leukämien, lymphozytische Lymphome, Hodgkin-Krankheit und myeloproliferative Syndrome und myelodysplastische Syndrome handelt.

15. Verwendung einer Verbindung der Formel (I) nach einem der Ansprüche 1 bis 7 zur Herstellung eines Medikaments zur Behandlung von proliferativen Erkrankungen.

16. Verwendung nach Anspruch 15, für die es sich bei den proliferativen Erkrankungen um Krebserkrankungen mit soliden Tumoren, beispielsweise Krebserkrankungen der Lunge (NSCLC), der Knochen, der Bauchspeicheldrüse, der Haut, Kaposi-Sarkom, intraokuläre Melanome, Krebserkrankungen der Brust, der Gebärmutter, des Gebärmutterhalses, der Eierstöcke, des Endometriums, der Vagina, der Vulva, der Harnröhre, des Penis, der Prostata, Eileiterkarzinome, Krebserkrankungen wie GIST und der Analregion, des Rektums, des Dünndarms, des Columns, des Magens, der Speiseröhre, der endokrinen Drüsen, der Schilddrüse, der Nebenschilddrüse oder der Nebenniere, Weichteilkarzinome, Ewing-Sarkome, Osteosarkome, Dermatofibrosarkom und andere Fibrosarkome, Krebserkrankungen der Blase oder der Nieren, Neoplasmen des Zentralnervensystems, Tumore der Wirbelsäule und Desmoide, Hirnstammgliome und Glioblastome, Hypophysenadenom und Metastasen davon handelt.

17. Verwendung einer Verbindung der Formel (I) nach einem der Ansprüche 1 bis 7 zur Herstellung eines Medikaments zur Behandlung von nichtmalignen proliferativen Erkrankungen.

18. Verwendung nach Anspruch 17, für die es sich bei den nichtmalignen proliferativen Erkrankungen um Restenose, Atherosklerose, Thrombose, Herz-insuffizienz, Herzhypertrophie, Pulmonalarterien-hypertonie, Fibrose, diabetische Nephropathie, Glomerulonephritis, chronische Pyelonephritis, Hämangiome, Autoimmunkrankheiten wie Psoriasis, Sklerodermatitis und Immunsuppression handelt.

## Claims

1. Compound corresponding to formula (I): 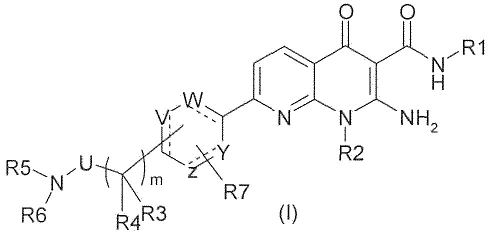 **R1** represents a (C1-C4)alkyl group,
**R2** represents (CH₂)_{n'}-**B** in which n' = 0, 1, 2, 3, 4 and B is a (C3-C5)cycloalkyl group or a (C1-C4)alkyl group optionally substituted with one or more fluorine atoms or a (C1-C4)alkoxy group,
**U** represents a carbonyl group or a -CH₂- group,
**Y, Z, V and W** represent, independently of each other, a -CH- group or a carbon atom optionally substituted with a group R7 or a heteroatom, or no atom, it being understood that the ring must be aromatic and be 5- or 6-membered,
**R3, R4** represent, independently of each other, a hydrogen atom or a linear (C1-C4)alkyl group, or R3 and R4 form, together with the carbon to which they are attached, a (C3-C5)cycloalkyl group,
**m is** an integer equal to 1, 2, 3, 4,
**R5** represents a hydrogen atom or a (C1-C4)alkyl group,
**R6** represents -(CH₂)ₙ-L in which n = 0, 1, 2, 3 and **L** is a group independently selected from the following groups:
○ a (C1-C5)alkyl group optionally substituted with a (C1-C4)alkoxy group,
○ a (C3-C5)cycloalkyl group,
○ an aryl comprising 6 carbon atoms and optionally substituted with one or more halogen atoms,
○ a 5- or 6-membered heteroaryl including at least one heteroatom chosen from a nitrogen or sulfur atom, optionally substituted with a (C1-C4)alkyl group,
○ a saturated heterocycle, in which the said heterocycle is 4- to 7-membered including at least one heteroatom chosen from a nitrogen atom and an oxygen atom, and is optionally substituted in any position, including on the nitrogen atom, with one or more substituents chosen from a fluorine atom, a (C1-C4) fluoroalkyl group, a linear or branched (C1-C4)alkyl group, a (C3-C5)cycloalkyl group and a (C1-C4)alkylsulfonamide group, the absolute configuration of a carbon substituted on the said heterocycle possibly being R or S, or racemic,
**R7** represents a hydrogen atom or a (C1-C4)alkyl group or a halogen atom,
in the form of the base or of an acid-addition salt, or in the form of a solvate, and also the enantiomers and diastereoisomers thereof, including mixtures thereof.

2. Compound of formula (I) according to Claim 1, **characterized in that**
**R1** represents a (C1-C4)alkyl group,
and/or
**R2** represents -(CH₂)_{n'}-**B** in which n' = 0, 1 and B is a (C3-C5)cycloalkyl group or a (C1-C4)alkyl group optionally substituted with one or more fluorine atoms or a (C1-C4)alkoxy group,
and/or
**U** represents a carbonyl group or a -CH₂- group,
and/or
**Y, Z, V and W** represent, independently of each other, a -CH- group or a carbon atom optionally substituted with a group R7 or a heteroatom, or no atom, it being understood that the ring must be aromatic and be 5- or 6-membered,
and/or
**R3, R4** represent, independently of each other, a hydrogen atom or a linear (C1-C4) alkyl group or R3 and R4 form, together with the carbon to which they are attached, a (C3-C5)cycloalkyl group,
and/or
**m** is an integer equal to 1, 2, 3, 4,
and/or
**R5** represents a hydrogen atom or a (C1-C4)alkyl group,
and/or
**R6** represents - (CH₂)ₙ-**L** in which n = 0, 1, 2, 3 and **L** is a group independently selected from the following groups:
○ a (C1-C5)alkyl group optionally substituted with a (C1-C4)alkoxy group,
○ a (C3-C5)cycloalkyl group,
○ an aryl comprising 6 carbon atoms and optionally substituted with one or more halogen atoms,
○ a 5- or 6-membered heteroaryl including at least one heteroatom chosen from a nitrogen or sulfur atom, optionally substituted with a (C1-C4)alkyl group,
○ a saturated heterocycle, in which the said heterocycle is 5- to 7-membered including at least one heteroatom chosen from a nitrogen atom and an oxygen atom, and is optionally substituted in any position, including on the nitrogen atom, with one or more substituents chosen from a fluorine atom, a (C1-C4) fluoroalkyl group, a linear or branched (C1-C4)alkyl group, a (C3-C5)cycloalkyl group and a (C1-C4)alkylsulfonamide group, the absolute configuration of a carbon substituted on the said heterocycle possibly being R or S, or racemic,
and/or
**R7** represents a hydrogen atom or a (C1-C4)alkyl group or a halogen atom,
in the form of the base or of an acid-addition salt, or in the form of a solvate, and also the enantiomers and diastereoisomers thereof, including mixtures thereof.

3. Compound of formula (I) according to Claim 1, **characterized in that U** represents a carbonyl group.

4. Compound of formula (I) according to Claim 1, **characterized in that** the ring comprising **Y, Z, V and W** is chosen from phenyl, pyridine, thiazole and thiophene groups.

5. Compound of formula (I) according to Claim 1, **characterized in that R3** and/or **R4** and/or **R5** represent a hydrogen atom.

6. Compound of formula (I) according to Claim 1, **characterized in that** the chain -[C(R3R4)]ₘ-U-N(R5)(R6) is in the para or meta position relative to the ring to which it is attached.

7. Compound of formula (I) according to Claim 1, **characterized in that** it is chosen from one of the following compounds:
2-amino-1-ethyl-N-methyl-4-oxo-7-{4-[2-oxo-2-(phenylamino)ethyl]phenyl}-1,4-dihydro-1,8-naphthyridine-3-carboxamide,
2-amino-1-ethyl-7-[4-(2-{[(1-ethylpyrrolidin-2-yl)methyl](methyl)amino}-2-oxoethyl)phenyl]-N-methyl-4-oxo-1,4-dihydro-1,8-naphthyridine-3-carboxamide,
2-amino-1-ethyl-N-methyl-4-oxo-7-{4-[2-oxo-2-(pyrid-3-ylamino)ethyl]phenyl}-1,4-dihydro-1,8-naphthyridine-3-carboxamide,
2-amino-1-ethyl-N-methyl-4-oxo-7-{4-[2-oxo-2-(pyrid-2-ylamino)ethyl]phenyl}-1,4-dihydro-1,8-naphthyridine-3-carboxamide,
2-amino-7-(4-{2-[(2-chlorophenyl)amino]-2-oxoethyl}phenyl)-1-ethyl-N-methyl-4-oxo-1,4-dihydro-1,8-naphthyridine-3-carboxamide,
2-amino-7-(4-{2-[(3,5-difluorophenyl)amino]-2-oxoethyl}phenyl)-1-ethyl-N-methyl-4-oxo-1,4-dihydro-1,8-naphthyridine-3-carboxamide,
2-amino-1-ethyl-N-methyl-4-oxo-7-(4-{2-oxo-2-[(pyrid-4-ylmethyl)amino]ethyl}phenyl)-1,4-dihydro-1,8-naphthyridine-3-carboxamide,
2-amino-1-ethyl-N-methyl-4-oxo-7-(4-{2-oxo-2-[(pyrid-2-ylmethyl)amino]ethyl}phenyl)-1,4-dihydro-1,8-naphthyridine-3-carboxamide,
2-amino-1-ethyl-7-(4-{2-[(2-methoxyethyl)amino]-2-oxoethyl}phenyl)-N-methyl-4-oxo-1,4-dihydro-1,8-naphthyridine-3-carboxamide,
2-amino-7-{4-[2(cyclopropylamino)-2-oxoethyl]-phenyl}-1-ethyl-N-methyl-4-oxo-1,4-dihydro-1,8-naphthyridine-3-carboxamide,
2-amino-1-ethyl-N-methyl-7-(4-{2-[(1-methylethyl)amino]-2-oxoethyl}phenyl)-4-oxo-1,4-dihydro-1,8-naphthyridine-3-carboxamide,
2-amino-7-{4-[2-(cyclopentylamino)-2-oxoethyl]-phenyl}-1-ethyl-N-methyl-4-oxo-1,4-dihydro-1,8-naphthyridine-3-carboxamide,
2-amino-1-ethyl-N-methyl-4-oxo-7-{4-[2-oxo-2-(pyrazin-2-ylamino)ethyl]phenyl}-1,4-dihydro-1,8-naphthyridine-3-carboxamide,
2-amino-1-ethyl-7-{4-[2-({[(2R)-1-ethylpyrrolidin 2-yl]methyl}amino)-2-oxoethyl]phenyl}-N-methyl-4-oxo-1,4-dihydro-1,8-naphthyridine-3-carboxamide,
2-amino-1-ethyl-7-{4-[2-({[(2S)-1-ethylpyrrolidin-2-yl]methyl}amino)-2-oxoethyl]phenyl}-N-methyl-4-oxo-1,4-dihydro-1,8-naphthyridine-3-carboxamide,
2-amino-1-ethyl-N-methyl-4-oxo-7-{4-[2-oxo-2-(pyrimidin-4-ylamino)ethyl]phenyl}-1,4-dihydro-1,8-naphthyridine-3-carboxamide,
2-amino-1-ethyl-N-methyl-4-oxo-7-{4-[2-oxo-2-(pyrid-4-ylamino)ethyl]phenyl}-1,4-dihydro-1,8-naphthyridine-3-carboxamide,
2-amino-1-ethyl-N-methyl-7-(4-{2-[(2-morpholin-4-ylethyl)amino]-2-oxoethyl}phenyl)-4-oxo-1,4-dihydro-1,8-naphthyridine-3-carboxamide,
2-amino-1-ethyl-N-methyl-4-oxo-7-{5-[2-oxo-2-(pyrid-2-ylamino)ethyl]pyrid-2-yl}-1,4-dihydro-1,8-naphthyridine-3-carboxamide,
2-amino-1-ethyl-N-methyl-7-{4-[1-methyl-2-oxo-2-(pyrid-2-ylamino)ethyl]phenyl}-4-oxo-1,4-dihydro-1,8-naphthyridine-3-carboxamide,
2-amino-1-ethyl-N-methyl-4-oxo-7-{6-[2-oxo-2-(pyrid-2-ylamino)ethyl]pyrid-3-yl}-1,4-dihydro-1,8-naphthyridine-3-carboxamide,
2-amino-1-ethyl-7-[4-(2-{[(4-ethylmorpholin-3-yl)methyl]amino}-2-oxoethyl)phenyl]-N-methyl-4-oxo-1,4-dihydro-1,8-naphthyridine-3-carboxamide,
2-amino-1-ethyl-7-[6-(2-{[(4-ethylmorpholin-3-yl)methyl]amino}-2-oxoethyl)pyrid-3-yl]-N-methyl-4-oxo-1,4-dihydro-1,8-naphthyridine-3-carboxamide,
2-amino-1-ethyl-7-{4-[2-({[(2S)-1-ethyl-4,4-difluoropyrrolidin-2-yl]methyl}amino)-2-oxoethyl]-phenyl}-N-methyl-4-oxo-1,4-dihydro-1,8-naphthyridine-3-carboxamide,
2-amino-1-ethyl-7-[4-(2-{[(4-ethylmorpholin-3-yl)methyl]amino}-1-methyl-2-oxoethyl)phenyl]-N-methyl-4-oxo-1,4-dihydro-1,8-naphthyridine-3-carboxamide,
2-amino-1-ethyl-7-{4-[2-({[(2S,4R)-1-ethyl-4-fluoropyrrolidin-2-yl]methyl}amino)-2-oxoethyl]phenyl}-N-methyl-4-oxo-1,4-dihydro-1,8-naphthyridine-3-carboxamide,
2-amino-1-ethyl-7-{4-[2-({[(2R)-1-(2-fluoroethyl)-pyrrolidin-2-yl]methyl}amino)-2-oxoethyl]phenyl}-N-methyl-4-oxo-1,4-dihydro-1,8-naphthyridine-3-carboxamide,
2-amino-1-ethyl-N-methyl-7-{4-[2-({[(2R)-1-(methylsulfonyl)pyrrolidin-2-yl]methyl}amino)-2-oxoethyl]phenyl}-4-oxo-1,4-dihydro-1,8-naphthyridine-3-carboxamide,
2-amino-1-ethyl-7-{5-[2-({[(2R)-1-(2-fluoroethyl)-pyrrolidin-2-yl]methyl}amino)-2-oxoethyl]pyrid-2-yl}-N-methyl-4-oxo-1,4-dihydro-1,8-naphthyridine-3-carboxamide,
2-amino-1-ethyl-N-methyl-4-oxo-7-{4-[2-oxo-2-({[(2R)-1-(2,2,2-trifluoroethyl)pyrrolidin-2-yl]-methyl}amino)ethyl]phenyl}-1,4-dihydro-1,8-naphthyridine-3-carboxamide,
2-amino-7-{4-[2-({[(2R)-1-(2,2-difluoroethyl)-pyrrolidin-2-yl]methyl}amino)-2-oxoethyl]phenyl}-1-ethyl-N-methyl-4-oxo-1,4-dihydro-1,8-naphthyridine-3-carboxamide,
2-amino-1-ethyl-N-methyl-7-{4-[2-({[4-(1-methylethyl)morpholin-3-yl]methyl}amino)-2-oxoethyl]-phenyl)-4-oxo-1,4-dihydro-1,8-naphthyridine-3-carboxamide,
2-amino-7-[4-(2-{[(4-cyclopropylmorpholin-3-yl)methyl]amino}-2-oxoethyl)phenyl]-1-ethyl-N-methyl-4-oxo-1,4-dihydro-1,8-naphthyridine-3-carboxamide,
2-amino-7-{5-[2-({[(2R)-1-(2,2-difluoroethyl)-pyrrolidin-2-yl]methyl}amino)-2 oxoethyl]pyrid-2-yl}-1-ethyl-N-methyl-4-oxo-1,4-dihydro-1,8-naphthyridine-3-carboxamide,
2-amino-1-ethyl-N-methyl-4-oxo-7-{4-[2-oxo-2-(1,3-thiazol-2-ylamino)ethyl]phenyl}-1,4-dihydro-1,8-naphthyridine-3-carboxamide,
2-amino-1-ethyl-N-methyl-7-[4-(2-{[(1-methyl-1H-imidazol-5-yl)methyl]amino}-2-oxoethyl)phenyl]-4-oxo-1,4-dihydro-1,8-naphthyridine-3-carboxamide,
2-amino-1-ethyl-N-methyl-4-oxo-7-(4-{2-oxo-2-[(2-pyrid-3-ylethyl)amino]ethyl}phenyl)-1,4-dihydro-1,8-naphthyridine-3-carboxamide,
2-amino-1-ethyl-N-methyl-7-(4-{2-[(3-morpholin-4-ylpropyl)amino]-2-oxoethyl}phenyl)-4-oxo-1,4-dihydro-1,8-naphthyridine-3-carboxamide,
2-amino-1-ethyl-N-methyl-4-oxo-7-(4-{2-oxo-2-[(2-pyrid-2-ylethyl)amino]ethyl}phenyl)-1,4-dihydro-1,8-naphthyridine-3-carboxamide,
2-amino-1-ethyl-N-methyl-4-oxo-7-(4-{2-oxo-2-[(2-phenylethyl)amino]ethyl}phenyl)-1,4-dihydro-1,8-naphthyridine-3-carboxamide,
2-amino-1-ethyl-N-methyl-4-oxo-7-(4-{2-oxo-2-[(3-phenylpropyl)amino]ethyl}phenyl)-1,4-dihydro-1,8-naphthyridine-3-carboxamide,
2-amino-1-ethyl-N-methyl-7-[4-(2-{[2-(1-methyl-1H-pyrrol-2-yl)ethyl]amino}-2-oxoethyl)phenyl]-4-oxo-1,4-dihydro-1,8-naphthyridine-3-carboxamide,
2-amino-1-ethyl-7-[4-(2-{[(1-ethylpyrrolidin-2-yl)methyl]amino}-2-oxoethyl)-1,3-thiazol-2-yl]-N-methyl-4-oxo-1,4-dihydro-1,8-naphthyridine-3-carboxamide,
2-amino-1-(3-methoxypropyl)-N-methyl-4-oxo-7-{4-[2-oxo-2-(pyrid-2-ylamino)ethyl]phenyl}-1,4-dihydro-1,8-naphthyridine-3-carboxamide,
2-amino-7-[4-(2-{[1-(2,2-difluoroethyl)pyrrolidin-3-yl]amino}-2-oxoethyl)phenyl]-1-ethyl-N-methyl-4-oxo-1,4-dihydro-1,8-naphthyridine-3-carboxamide,
2-amino-1-ethyl-7-[4-(3-{[(1-ethylpyrrolidin-2-yl)methyl]amino}-3-oxopropyl)phenyl]-N-methyl-4-oxo-1,4-dihydro-1,8-naphthyridine-3-carboxamide,
2-amino-1-ethyl-7-[4-(4-{[(1-ethylpyrrolidin-2-yl)methyl]amino}-4-oxobutyl)phenyl]-N-methyl-4-oxo-1,4-dihydro-1,8-naphthyridine-3-carboxamide,
2-amino-1-ethyl-7-[4-(2-{[(1-ethylpyrrolidin-2-yl)methyl]amino}-2-oxoethyl)phenyl]-4-oxo-N-propyl-1,4-dihydro-1,8-naphthyridine-3-carboxamide,
2-amino-7-[4-(2-{[(1-ethylpyrrolidin-2-yl)methyl]amino}-2-oxoethyl)phenyl]-N-methyl-4-oxo-1-(2,2,2-trifluoroethyl)-1,4-dihydro-1,8-naphthyridine-3-carboxamide,
2-amino-1-cyclopentyl-7-[4-(2-{[(1-ethylpyrrolidin-2-yl)methyl]amino}-2-oxoethyl)phenyl]-*N*-methyl-4-oxo-1,4-dihydro-1,8-naphthyridine-3-carboxamide,
2-amino-1-ethyl-7-[4-(5-{[(1-ethylpyrrolidin-2-yl)methyl]amino}-5-oxopentyl)phenyl]-*N*-methyl-4-oxo-1,4-dihydro-1,8-naphthyridine-3-carboxamide,
1-ethyl-7-[5-(2-{[(1-ethylpyrrolidin-2-yl)methyl]amino}-2-oxoethyl)thiophen-2-yl]-*N*,2-dimethyl-4-oxo-1,4-dihydro-1,8-naphthyridine-3-carboxamide,
2-amino-7-{4-[2-({[(2R)-1-ethylpyrrolidin-2-yl]-methyl}amino)-2-oxoethyl]phenyl}-N-methyl-1-(2-methylpropyl)-4-oxo-1,4-dihydro-1,8-naphthyridine-3-carboxamide,
2-amino-1-ethyl-7-{4-[1-({[(2R)-1-ethylpyrrolidin-2-yl]methyl}carbamoyl)cyclopropyl]phenyl}-N-methyl-4-oxo-1,4-dihydro-1,8-naphthyridine-3-carboxamide,
2-amino-1-ethyl-7-{2-[2-({[(2R)-1-ethylpyrrolidin-2-yl]methyl}amino)-2-oxoethyl]-1,3-thiazol-4-yl}-N-methyl-4-oxo-1,4-dihydro-1,8-naphthyridine-3-carboxamide,
2-amino-1-ethyl-7-{4-[2-({[(2R)-1-ethylpyrrolidin-2-yl]methyl}amino)-2-oxoethyl]-2-fluorophenyl}-N-methyl-4-oxo-1,4-dihydro-1,8-naphthyridine-3-carboxamide,
2-amino-7-[3-chloro-4-(2-{[(1-ethylpyrrolidin-2-yl)methyl]amino}-2-oxoethyl)phenyl]-1-ethyl-N-methyl-4-oxo-1,4-dihydro-1,8-naphthyridine-3-carboxamide,
2-amino-7-[3-fluoro-4-(2-{[(1-ethylpyrrolidin-2-yl)methyl]amino}-2-oxoethyl)phenyl]-1-ethyl-N-methyl-4-oxo-1,4-dihydro-1,8-naphthyridine-3-carboxamide,
2-amino-7-[3-methyl-4-(2-{[(1-ethylpyrrolidin-2-yl)methyl]amino}-2-oxoethyl)phenyl]-1-ethyl-N-methyl-4-oxo-1,4-dihydro-1,8-naphthyridine-3-carboxamide,
2-amino-1-(cyclopropylmethyl)-7-[4-(2-{[(1-ethylpyrrolidin-2-yl)methyl]amino}-2-oxoethyl)phenyl]-N-methyl-4-oxo-1,4-dihydro-1,8-naphthyridine-3-carboxamide,
2-amino-1-ethyl-7-[4-(2-{[(1-ethylpyrrolidin-2-yl)methyl]amino}-2-oxoethyl)-2-methylphenyl]-*N*-methyl-4-oxo-1,4-dihydro-1,8-naphthyridine-3-carboxamide,
2-amino-1-ethyl-7-[3-(2-{[(1-ethylpyrrolidin-2-yl)methyl]amino}-2-oxoethyl)phenyl]-N-methyl-4-oxo-1,4-dihydro-1,8-naphthyridine-3-carboxamide,
2-amino-1-ethyl-N-methyl-4-oxo-7-{3-[2-oxo-2-(pyrid-2-ylamino)ethyl]phenyl}-1,4-dihydro-1,8-naphthyridine-3-carboxamide,
2-amino-1-ethyl-*N*-methyl-4-oxo-7-{4-[2-(pyrid-2-ylamino)ethyl]phenyl}-1,4-dihydro-1,8-naphthyridine-3-carboxamide,
in the form of the base or of an acid-addition salt, or in the form of a solvate, and also the enantiomers and diastereoisomers thereof, including mixtures thereof.

8. Process for preparing a compound of formula (I) according to any one of Claims 1 to 7, **characterized in that** a compound of formula (XI): 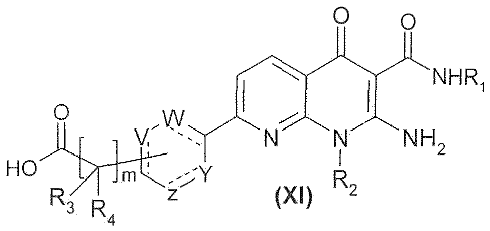 is reacted with a compound of formula HNR5R6, in the presence of a coupling agent and a base, R1, R2, R3, R4, R5, R6, V, W, Y, Z and m being as defined in Claim 1.

9. Process for preparing a compound of formula (I) according to any one of Claims 1 to 7, **characterized in that** a compound of formula (VII): 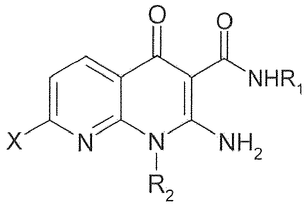 is reacted with a compound of formula (IXa) 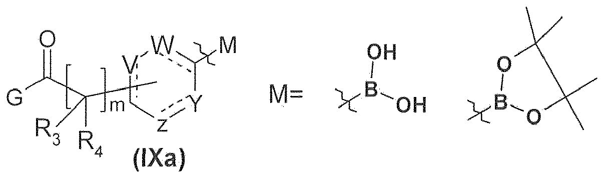 in which X represents a halogen atom,
G represents a (C1-C4)alkoxy group or a group -NR5R6, and
R1, R2, R3, R4, R5, R6, V, W, Y, Z and m are as defined in Claim 1.

10. Process for preparing a compound of formula (I) according to any one of Claims 1 to 7, **characterized in that** a compound of formula (VII): 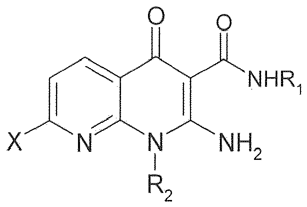 is reacted with a compound of formula (IXb): 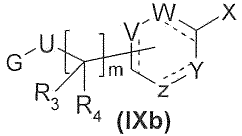 in which G represents a (C1-C4)alkoxy group or a group -NR5R6, X represents a halogen atom and R1, R2, R3, R4, R5, R6, V, W, Y, Z and m are as defined in Claim 1.

11. Medicament, **characterized in that** it comprises a compound of formula (I) according to any one of Claims 1 to 7, or an addition salt of this compound with a pharmaceutically acceptable acid, or alternatively a solvate of the compound of formula (I).

12. Pharmaceutical composition, **characterized in that** it comprises a compound of formula (I) according to any one of Claims 1 to 7, or a pharmaceutically acceptable salt or a solvate of this compound, and also at least one pharmaceutically acceptable excipient.

13. Use of a compound of formula (I) according to any one of Claims 1 to 7 for the preparation of a medicament for treating proliferative diseases.

14. Use according to Claim 13 for which the proliferative diseases are liquid-tumour cancers, chronic or acute leukaemias, lymphocytic lymphomas, Hodgkin's disease, and myeloproliferative syndromes, and myelodysplastic syndromes.

15. Use of a compound of formula (I) according to any one of Claims 1 to 7 for the preparation of a medicament for treating proliferative diseases.

16. Use according to Claim 15 for which the proliferative diseases are solid-tumour cancers, for example lung cancer (NSCLC), bone cancer, pancreatic cancer or skin cancer, Kaposi's sarcoma, intraocular melanomas, breast cancer, uterine cancer, cervical cancer, ovarian cancer, cancer of the endometrium, of the vagina, of the vulva, of the urethra, of the penis or of the prostate, fallopian tube carcinomas, cancers such as GIST and of the anal region, of the rectum, of the small intestine, bowel cancer, stomach cancer, cancer of the oesophagus, of the endocrine, thyroid, parathyroid or adrenal glands, soft-tissue sarcomas, Ewing's sarcomas, osteosarcomas, dermatofibrosarcoma and other fibrosarcomas, cancer of the bladder or of the kidney, neoplasms of the central nervous system, spinal column tumours and desmoid tumours, brain stem gliomas and glioblastomas, pituitary adenomas and metastases thereof.

17. Use of a compound of formula (I) according to any one of Claims 1 to 7 for the preparation of a medicament for treating non-malignant proliferative diseases.

18. Use according to Claim 17 for which the non-malignant proliferative diseases are restenosis, atherosclerosis, thrombosis, cardiac insufficiency, cardiac hypertrophy, pulmonary arterial hypertension, fibrosis, diabetic nephropathy, glomerulonephritis, chronic pyelonephritis, haemangiomas, autoimmune diseases such as psoriasis, sclerodermatitis and immunosuppression.
